# EUROPEAN PATENT SPECIFICATION

(11) **EP 3 917 957 B1**
(45) Date of publication and mention of the grant of the patent: **26.11.2025**
(21) Application number: 20708772.7
(22) Date of filing: 26.01.2020
(51) Int. Cl.: C07K 16/24, A61P 37/06, A61P 1/16

(54) **PSMP ANTAGONISTS FOR USE IN TREATMENT OF FIBROTIC DISEASE OF THE LUNG, KIDNEY OR LIVER**
PSMP-ANTAGONISTEN ZUR VERWENDUNG BEI DER BEHANDLUNG VON FIBROTISCHEN ERKRANKUNGEN DER LUNGE, NIERE ODER LEBER
ANTAGONISTES DE PSMP DESTINÉS À ÊTRE UTILISÉS DANS LE TRAITEMENT D'UNE MALADIE FIBROTIQUE DU POUMON, DU REIN OU DU FOIE

(30) Priority: 28.01.2019 US 201962797440 P; 07.10.2019 US 201962911511 P; 11.10.2019 US 201962913937 P
(43) Date of publication of application: 08.12.2021
(73) Proprietor: Maple Biotech LLC, Bellaire TX 77401 (US)
(72) Inventor: WANG, Ying, Beijing 100875 (CN); SHE, Shaoping, Beijing 100191 (CN); PEI, Xiaolei, Tianjin 300020 (CN); LI, Qingqing, Beijing 100191 (CN); LIU, Zhongtian, Beijing 100191 (CN); SONG, Zhanming, Beijing 100191 (CN); DI, Chunhui, Morrisville, North Carolina 27560 (US); LIU, Christopher, Bellaire, Texas 77401 (US)
(74) Representative: Script Intellectual Property LLP
(86) International application number: PCT/US2020/015131
(87) International publication number: WO 2020/159836

(56) References cited:
- CN-A- 106 749 661
- SHE SHAOPING ET AL: "PSMP/MSMP promotes hepatic fibrosis through CCR2 and represents a novel therapeutic target", JOURNAL OF HEPATOLOGY, ELSEVIER, AMSTERDAM, NL, vol. 72, no. 3, 6 December 2019 (2019-12-06), pages 506 - 518, XP086049256, ISSN: 0168-8278, [retrieved on 20191206], DOI: 10.1016/J.JHEP.2019.09.033
- RANGARAJAN SUNAD ET AL: "Metformin reverses established lung fibrosis in a bleomycin model", NATURE MEDICINE, NATURE PUB. CO, NEW YORK, vol. 24, no. 8, 2 July 2018 (2018-07-02), pages 1121 - 1127, XP036928730, ISSN: 1078-8956, [retrieved on 20180702], DOI: 10.1038/S41591-018-0087-6

## Description

### REFERENCE TO SEQUENCE LISTING

The instant application contains a Sequence Listing which has been submitted in ASCII format via EFS-Web. Said ASCII Copy, created on December 26, 2019, is named "MAB_0110PC_20191226_Seq_Listing_ST25" and is 11,153 bytes in size.

### BACKGROUND OF THE INVENTION

### Liver Fibrosis and Nonalcoholic Fatty Liver Disease (NAFLD)

Fibrosis is the excessive accumulation of extracellular matrix that often occurs as a wound healing response to repeated or chronic tissue injury and may lead to the disruption of organ architecture and loss of function. Fibrosis affects nearly every tissue in the body. The mechanism of fibrosis resolution encompasses degradation of the fibrotic extracellular matrix as well as elimination of fibrogenic myofibroblasts.

Liver fibrosis, a wound-healing response to chronic liver injury, is characterized by excessive deposition of extracellular matrix (ECM) in the liver, triggered by a variety of causes, including hepatitis virus infection, alcohol abuse, nonalcoholic steatohepatitis (NASH) or primary biliary cholangitis, which leads to loss of liver function and disruption of liver structure. Recent evidence for the resolution of organ fibrosis in humans is observed in the liver. Patients with liver fibrosis associated with hepatitis B virus (HBV) or HCV infection treated with antiviral therapies have shown fibrosis regression and histological improvements even in cases of cirrhosis.

Nonalcoholic fatty liver disease (NAFLD) has emerged as a leading cause of chronic liver disease. NAFLD is a complex spectrum of liver diseases ranging from benign hepatic steatosis to its more aggressive necroinflammatory manifestation, NASH. NASH is distinct from other liver diseases, because it is tightly associated with comorbidities of the metabolic syndrome, such as insulin resistance and type 2 diabetes, and cardiovascular complications linked to hypertension and dyslipidemia. These comorbidities already exist in mere NAFLD, which is defined by the absence of fibrosis and major liver inflammation. These non-hepatic companion diseases represent the major comorbidities and causes of mortality in NAFLD and the early stages of fibrotic NASH, up to stage 2 fibrosis. Liver-related morbidity and mortality only increase significantly beyond stage 1 fibrosis, especially with the emergence of cirrhosis. Thus, prevention and therapy must address two largely independent targets: the metabolic complications which are treatable with a range of medications, and moderate to advanced fibrosis (stage 2-4) for which no approved drugs exist.

Common experimental rodent models for liver fibrosis include administration of a hepatotoxin (*e.g*., carbon tetrachloride [CCl₄]) to induce acute hepatocellular injury or bile duct ligation (BDL) to induce cholestasis, resulting in pericentral or periportal liver fibrosis, respectively. The NASH models are essentially distinguished by their ability to mimic the etiology and/or natural history (obesogenic dietary models) or histopathology (nutrient-deficient dietary models or chemically induced models). Methionine and choline deficient (MCD) diet is one of the very commonly used diets which produces the most severe phenotype of NASH in the shortest time. The MCD diet induces significant fibrosis compared to other dietary animal models. Also, genetic models (monogenetic or polygenetic) are widely used in NASH research.

The initiation of fibrosis crucially depends on an inflammatory phase in which liver resident macrophages and Kupffer cells are activated and release transforming growth factor (TGF-β) as well as other proinflammatory cytokines that activate hepatic stellate cells (HSCs). HSCs are responsible for producing most of the ECM and play a central role in liver fibrogenesis. HSCs are quiescent and located in the space between hepatocytes and sinusoidal endothelium (space of Disse) as retinoid storage cells. Upon liver injury, HSCs, the major collagen-synthesizing cells in the liver, become activated and transdifferentiate into myofibroblast-like cells that proliferate faster and display enhanced chemotaxis, survival and collagen production. HSC activation is driven by multiple mediators, such as chemokines, reactive oxygen species, growth factor, matrix stiffness, matricellular proteins and damageassociated molecular patters.

### Alcoholic Liver Disease (ALD)

Alcoholic liver disease (ALD) affects millions of patients worldwide each year. The progression of ALD is well-characterized and is actually a spectrum of liver diseases, ranging from steatosis, to inflammation and necrosis (steatohepatitis), to fibrosis and cirrhosis, and eventually hepatocellular carcinoma (HCC) in some cases. Fatty liver (steatosis) is the first stage of response in the liver to binge drinking or chronic ethanol consumption. Accumulation of lipid products such as triglycerides in hepatocytes leads to lipid superoxidation and oxidative stress, resulting in apoptosis, hepatic inflammation and the activation of HSCs.

Alcohol-induced fatty liver involves increasing hepatic sterol regulatory elementbinding protein (SREBP)-1 and decreasing hepatic peroxisome proliferator-activated receptor (PPAR)α activity. SREBP1 is mainly expressed in the liver and an important transcriptional regulator of the biosynthesis of cholesterol, fatty acid, and triglyceride. PPARα serves as the master regulator of hepatic lipid metabolism.

Currently, the most widely used model for alcoholic liver disease is ad libitum feeding with the Lieber-DeCarli liquid diet containing ethanol for 4-6 weeks. This model can induce hepatic steatosis and slight fibrosis. In addition, a chronic-plus-binge alcohol feeding model, also named the National Institute on Alcohol Abuse and Alcoholism (NIAAA) model, has been reported. See, e.g., Bertola et al., Nat. Protoc. 8:627-37, 2013. The NIAAA model mimics acute-on-chronic alcoholic liver injury in patients and is similar to the drinking pattern in many alcoholic hepatitis patients who have a background of chronic drinking for many years (chronic) and a history of recent excessive alcohol consumption (binge). The protocol for the chronic-plus-binge alcohol feeding model is ad libitum chronic oral feeding *(e.g.,* 10 days) with the Lieber-DeCarli ethanol liquid diet plus a single binge ethanol feeding (*e.g*., gavage of a single dose of ethanol, 5g/kg body weight, 31.5% ethanol). Such chronic-plus-binge ethanol feeding synergistically induced significant steatosis, liver injury, and inflammation in mice. See, *e.g.,* Bertola *et al., supra.*

### Primary Sclerosing Cholangitis (PSC) and Primary Biliary Cholangitis (PBC)

Liver cholestasis is characterized by impairment in bile flow. Among cholestatic diseases, primary sclerosing cholangitis and primary biliary cholangitis represent relevant causes of chronic liver disease, associated to significant morbidity and mortality.

Primary sclerosing cholangitis (PSC) is a rare, chronic, cholestatic liver disease of uncertain etiology characterized biochemically by cholestasis and histologically and cholangiographically by fibro-obliterative inflammation of the bile ducts. In a clinically significant proportion of patients, PSC progresses to cirrhosis, end-stage liver disease, and/or hepatobiliary cancer, though the disease course can be highly variable.

Primary biliary cholangitis (PBC), formerly known as primary biliary cirrhosis, is a chronic disease in which the small bile ducts in the liver become injured and inflamed and are eventually destroyed. When there are no bile ducts, bile builds up and causes liver damage.

Chronic feeding of 3,5-diethoxycarbonyl-1,4-dihydrocollidine, named DDC, has been proposed as an *in vivo* model for cholestatic disease, such as primary sclerosing cholangitis (PSC) and primary biliary cholangitis/primary biliary cirrhosis (PBC), due to the formation of intraductal porphyrin plugs. In these diseases as in DDC, the primary damage is directed toward cholangiocytes. Chronic feeding of DDC in mice reproduces the main histopathological hallmarks of human cholestatic disease such as (1) remodeling of biliary compartments giving rise to ductular reaction, (2) periductular fibrosis, and (3) inflammatory infiltrate.

### Lung Fibrosis

Lung fibrosis is associated with diverse etiologies, including scleroderma (systemic sclerosis), sarcoidosis, infections, and exposure to toxicants or radiation. Idiopathic pulmonary fibrosis (IPF) is the most common form of idiopathic interstitial pneumonia and is usually fatal, with a median survival of 2 to 3 years. In 2014, the FDA granted fast-track approval for the profibrotic signaling inhibitors pirfenidone and nintedanib for treating IPF on the basis of their slowing of lung function decline as measured by forced vital capacity and reduced all-cause mortality. However, the efficacy of these drugs to promote fibrosis resolution in IPF has not been demonstrated. The most common experimental mouse model for lung fibrosis is intratracheal administration of the chemotherapeutic drug bleomycin, which induces inflammation followed by fibrosis. Various studies have found that myofibroblasts in the fibrotic lung are largely derived from pericytes, with contribution from mesothelial cells through mesothelial-to-mesenchymal transition (MMT).

### Graft-versus-host Disease (GVHD)

GVHD (graft-versus-host disease) is a common complication occurring after the human allogeneic hematopoietic stem cell transplantation (allo-HSCT), which is believed to be the main obstacle of the recovery from HSCT. Monocyte and macrophage have been reported by many research teams to be attracted and accumulated at the intestinal mucous, liver, and skin during GVHD and aggravating the disease. *See, e.g.,* Zhang et al., J. Leukoc. Biol. 99:279-87, 2016. Various mouse models of GVHD have been established to help understand the procession of the disease and find an effective method for reducing GVHD prevalence. *See generally, e.g.,* Schroeder et al., Dis. Model Mech. 4:318-333, 2011.

In the clinic, GVHD is defined into two subtypes, acute GVHD and chronic GVHD, based on the time of symptoms occurring. Acute GVHD is the main cause of death after HSCT, the symptoms of which include skin rash, gastrointestinal tract disorders and liver disorders usually happening within 100 days after HSCT. Symptoms of chronic GVHD may be restricted to a single organ or site in the body, or they may be widespread, occurring after 100 days to several years. Symptoms may affect any of the following: eyes, mouth, nails, skin, scalp and body hair, gastrointestinal tract, lungs, liver, muscles and joints, genitals and sex organs. GVHD is classified into Grade I and Grade II or higher GVHD according to the severity of acute GVHD. Grade I means cutaneous GVHD over ≤50 percent body surface area without liver or gastrointestinal tract involvement, the treatment of which is the use of topical treatments *(e.g.,* topical steroids) and the optimization of prophylactic measures (*e.g.,* cyclosporine levels). Patients with more severe disease are considered to have Grade II or higher GVHD, which is typically treated using systemic glucocorticoids (*e.g*., methylprednisolone). Oral beclomethasone is suggested for use in patients with gastrointestinal involvement but should be suspended if there is a gastrointestinal infection. *See* Zeiser et al., N. Engl. J. Med. 377:2167-2179, 2017; Zeiser et al., N. Engl. J. Med. 377:2565-2579, 2017.

Therapeutic directions in acute GVHD, according to early data from clinical trials, include the use of costimulatory pathway blockade, targeted anti-interleukin-6 monoclonal antibodies, histone deacetylase inhibitors, kinase inhibitors, proteasome inhibitors, the anti-inflammatory protease inhibitor alpha-1-antitrypsin, CTLA-4 antagonism, CCR5 blockade, and adoptive Treg transfer. These and other recent strategies that are being developed must be tested in prospective Phase 3 trials before they can become standard therapy for acute GVHD. *See* Zeiser et al., N. Engl. J. Med. 377:2167-2179, 2017.

For several decades, little progress was made in the treatment of chronic GVHD, with no drugs approved by the Food and Drug Administration (FDA) for treating glucocorticoid-dependent patients or those with glucocorticoid-refractory disease. Recent advances have been achieved by reducing the alloreactive T-cell and antibody-producing B-cell burden (*e.g*., through treatment with cyclophosphamide after transplantation, naive T-cell depletion, and B-cell depletion with the use of rituximab). New therapeutic approaches are based on a better understanding of the pathogenesis of chronic GVHD, especially the prominent role of B-cell signaling and prolonged immune activation of certain T-cell subsets, Treg-cell deficiency, and tissue fibrosis. Prospects include targeting fibrosis and inciting mechanisms (*e.g*., with pirfenidone and interleukin-17 or RORγt inhibitors), targeting plasma cells (*e.g*., with immunoproteasome inhibitors and anti-interleukin-6 receptor), and inhibiting chemokine induced T-cell and B-cell recruitment to target organs affected by chronic GVHD, as identified in biomarker studies (*e.g*., with CXCL9 inhibitors). Although these developments are promising, glucocorticoids still constitute standard front-line therapy, despite the substantial side effects of long-term use. *See* Zeiser et al., N. Engl. J. Med. 377:2565-2579, 2017.

### Acute kidney injury (AKI) and chronic kidney disease (CKD)

Renal disease is increasingly recognized as a global health problem. *See, e.g.,* Felix et al., Nat. Rev. Nephrology. 15:263-27, 2019. Acute kidney injury (AKI) and chronic kidney disease (CKD) are linked to high morbidity and mortality. AKI is regarded as a rapid and reversible decline in renal function and is associated with accelerated CKD. Compared to patients with no history of AKI or CKD, AKI patients are more likely to develop new CKD or end-stage renal disease (ESRD). Conversely, CKD also plays an important role in AKI. Patients with CKD may suffer higher risk of transient decreases in renal function consistent with AKI. *See, e.g.,* Yin-Wu Bao et al., Zoological Research. 39:72-86, 2018. AKI and CKD are closely linked and are therefore regarded as an integrated clinical syndrome. *See, e.g.,* Chawla et al., N. Engl. J. Med. 371:58-66, 2014.

Mechanisms of disease generation and progression in AKI and CKD remain incompletely understood. *See, e.g.,* John et al., J. Clin. Invest. 124:2294-2298, 2014. Consequently, few strategies are available to slow the progression of renal disease, with most treatment options centered around lowering blood pressure and reducing proteinuria. Clearly, additional therapeutic avenues are needed. *See generally, e.g.,* Siew et al., Kidney International. 87:46-61, 2015.

Rhabdomyolysis is a serious syndrome caused by skeletal muscle injury and the subsequent release of breakdown products from damaged muscle cells into systemic circulation. The muscle damage most often results from strenuous exercise, muscle hypoxia, medications, or drug abuse and can lead to life-threatening complications, such as AKI. *See, e.g.,* Koshu et al., Nat. Med. 24:232-238, 2018. Experimental AKI induced by glycerol injection is a well-established model of rhabdomyolysis. It is characterized by intense cortical acute tubular necrosis and inflammatory cell infiltration. *See generally, e.g.,* Yanqiu et al., Stem Cell Research & Therapy 5:80, 2014.

Renal fibrosis is a key pathological phenomenon of CKD contributing to the progressive loss of renal function. Renal fibrosis involves glomerular sclerosis and/or interstitial fibrosis. Aberrant and excessive depositions of extracellular matrix (ECM) proteins in both glomeruli and interstitial regions are typical hallmarks of renal fibrosis and amplify the severity of kidney injury.

Obstructive uropathy is the main cause of end-stage renal disease in children, being one of the main reasons for pediatric kidney transplants. Unilateral ureteral obstruction (UUO) is the most common rodent model used to study AKI and CKD, where the primary feature of UUO is interstitial inflammation, tubular cell injury/death and fibrosis as a result of obstructed urine flow. *See, e.g.,* Elena Martinez-Klimova et al., Biomolecules 9:141, 2019.

Systemic lupus erythematosus (SLE) is a systemic autoimmune disease that occurs when the body's immune system attacks the body's own tissues and organs. Inflammation caused by SLE can affect many different body systems. Kidney failure is one of the leading causes of death among people with SLE. Lupus nephritis is a common and severe manifestation of systemic lupus erythematosus (SLE), and an important cause of both acute kidney injury and end-stage renal disease. *See, e.g.,* Davidson et al., Nat. Rev. Rheumatol, 6:13-20, 2010. It is initiated by the glomerular deposition of immune complexes which triggers a cascade of inflammatory events including activation of Fc receptors and complement, recruitment of inflammatory cells, and eventual fibrosis. *See, e.g.,* Anne Davidson, Nat. Rev. Rheumatol. 12:143-153, 2015. The current approaches to the management of lupus nephritis rely on high-dose corticosteroids plus a broad-spectrum immunosuppressive agent. *See, e.g.,* Feng et al., Nat. Rev. Nephrology. 13:483-495, 2017; Samir et al., J. Am. Soc. Nephrol. 27:2929-2939, 2016. A well-known murine model of lupus nephritis utilizes the inoculation of female B6D2F1 mice with T lymphocytes from male DBA/2 mice to induce an immunostimulatory GVH reaction and a lupus-like disease in which mice show the symptoms of SLE patients. *See, e.g.,* Via et al., J Immunol. 139:1840-1849, 1987.

IgA nephropathy-identified 50 years ago by Jacques Berger and once thought to be a rare variant of mesangial proliferative glomerular diseases-is the most frequent glomerular disease worldwide and an important cause of chronic kidney disease and end-stage kidney failure. The pathogenetic mechanisms of IgA nephropathy are only partly understood. Data from clinical and basic research suggest multiple hypotheses, according to which galactose deficient IgA₁ molecules are recognized by specific autoantibodies, resulting in the formation of IgA₁-IgG immune complexes that are deposited in the glomerular mesangium, where they induce renal injury. *See, e.g.,* Jennifer et al., Clin. J. Am. Soc. Nephrol. 12:677-686, 2017. It is recognized as an immune complex disease.

### PC3-secreted Microprotein

PSMP, namely PC3-secreted microprotein, was initially found in PC3 cells and benign and malignant prostate tissues *(see* Valtonen-André et al., Biol. Chem. 388:289-95, 2007). PSMP is also called prostate-associated microseminoprotein (MSMP) *(see* Frankenberg et al., BMC Evol. Biol. 11:373-85, 2011). MSMP tissue expression is reported to be restricted to testis only as a member of the beta-microseminoprotein family (*see id.*)*,* apart from its expression in PC3 cells and benign and malignant prostate tissues. A study using omics strategies reveals PSMP is a novel chemotactic cytokine acting as a CCR2 ligand to recruit peripheral blood monocytes and lymphocytes *(see* Pei et al., J. Immunol. 192:1878-86, 2014). The affinity between PSMP and CCR2 was found to be comparable to that between CCL2 and CCR2 (*see id*.).

Another study demonstrated that PSMP is expressed in human colitis tissues and significantly up-regulated DSS-induced mouse colitis *(see* Pei et al., Sci. Rep. 7:5107, 2017). PSMP plays a vital role in promoting DSS colitis by chemo-attracts Ly6Chi monocytes in a CCR2 dependent manner (*see id.*)*.* Anti-PSMP neutralizing antibody mollified the colitis by reducing macrophage infiltration and inhibiting the expression of IL-6, TNFα and CCL2 *(see id*.).

A recent study found that expression of the MSMP gene was substantially upregulated in anti-VEGF therapy resistant compared with control tumors. *See* Mitamura et al., Oncogene 37:722-731, 2018. MSMP secretion from cancer cells was induced by hypoxia. Recruitment of the transcriptional repressor CCCTC-binding factor (CTCF) to the MSMP enhancer region was decreased by histone acetylation under hypoxic conditions in cancer cells. MSMP siRNA, delivered *in vivo* using the DOPC nanoliposomes, restored tumor sensitivity to anti-VEGF therapy. In ovarian cancer patients treated with bevacizumab, serum MSMP concentration increased significantly only in non-responders. *See id.*

CN106749661 discloses monoclonal antibody 3D5 and its use in treating colitis in mice.

Shaoping et al. (2020) Journal of Hepatology 72, 3, p506-518 discloses PSMP/MSMP promoting hepatic fibrosis through CCR2 as a therapeutic target.

Sunad et al. (2018) Nature 24, 8, p1121-1127 discloses that metformin reverses established lung fibrosis in a bleomycin model.

### SUMMARY

The references herein to methods of treatment are to be interpreted as references to compounds, compositions and medicaments for use in methods of treatment of the human or animal body by therapy.

The aspects and embodiments of the present invention are set forth in the claims.

In one aspect, the present disclosure provides a method for treating liver fibrosis. The method generally includes administering to a subject having liver fibrosis an effective amount of a PC3-secreted microprotein (PSMP) antagonist. In a related aspect, the present disclosure provides a PSMP antagonist for use in the treatment of liver fibrosis. In another related aspect, the present disclosure provides use of a PSMP antagonist in the manufacture of a medicament for the treatment of liver fibrosis. In some embodiments of a method, PSMP antagonist, or use as above, the liver fibrosis has progressed to liver cirrhosis. In other, non-mutually exclusive embodiments, the liver fibrosis is hepatitis B virus (HBV)-induced, hepatitis C virus (HCV)-induced, or alcohol-induced liver fibrosis, or the liver fibrosis is associated with nonalcoholic fatty liver disease. In some variations in which the liver fibrosis has progressed to liver cirrhosis, the liver cirrhosis is primary biliary cirrhosis. In some variations in which the liver fibrosis is associated with nonalcoholic fatty liver disease, the nonalcoholic fatty liver disease is nonalcoholic steatohepatitis (NASH). In some embodiments, the liver fibrosis is associated with a disease or disorder selected from alcoholic liver disease (ALD), alcoholic hepatitis, alcoholic cirrhosis, chronic hepatitis B, chronic hepatitis C, chronic hepatitis D, nonalcoholic fatty liver disease (NAFLD), nonalcoholic steatohepatitis (NASH), hemochromatosis, cystic fibrosis, Wilson's disease, biliary atresia, alpha-1 antitrypsin deficiency, galactosemia, glycogen storage disease, a genetic digestive disorder, Alagille syndrome, autoimmune hepatitis, primary sclerosing cholangitis (PSC), primary biliary cholangitis (PBC, previously known as primary biliary cirrhosis), an infection, drug-induced liver injury, and Budd-Chiari syndrome.

In another aspect, the present disclosure provides a method of treating lung fibrosis. The method generally includes administering to a subject having lung fibrosis an effective amount of a PC3-secreted microprotein (PSMP) antagonist. In a related aspect, the present disclosure provides a PSMP antagonist for use in the treatment of lung fibrosis. In another related aspect, the present disclosure provides use of a PSMP antagonist in the manufacture of a medicament for the treatment of lung fibrosis. In some embodiments, the lung fibrosis is associated with a disease or disorder selected from the group consisting of dermatomyositis, polymyositis, mixed connective tissue disease, systemic lupus erythematosus, rheumatoid arthritis, sarcoidosis, scleroderma, pneumonia, chronic radiation pneumonitis, a pneumoconiosis, an infection, and drug-induced lung injury.

In another aspect, the present disclosure provides a method of treating nonalcoholic fatty liver disease. The method generally includes administering to a subject having nonalcoholic fatty liver disease an effective amount of a PC3-secreted microprotein (PSMP) antagonist. In a related aspect, the present disclosure provides a PSMP antagonist for use in the treatment of nonalcoholic fatty liver disease. In another related aspect, the present disclosure provides use of a PSMP antagonist in the manufacture of a medicament for the treatment of nonalcoholic fatty liver disease. In some embodiments of a method, PSMP antagonist, or use as above, the nonalcoholic fatty liver disease is nonalcoholic steatohepatitis (NASH).

In another aspect, the present disclosure provides a method of treating alcoholic liver disease (ALD). The method generally includes administering to a subject having alcoholic liver disease an effective amount of a PC3-secreted microprotein (PSMP) antagonist. In a related aspect, the present disclosure provides a PSMP antagonistfor use in the treatment of alcoholic liver disease. In another related aspect, the present disclosure provides use of a PSMP antagonist in the manufacture of a medicament for the treatment of alcoholic liver disease.

In another aspect, the present disclosure provides a method of treating primary sclerosing cholangitis (PSC). The method generally includes administering to a subject having primary sclerosing cholangitis an effective amount of a PC3-secreted microprotein (PSMP) antagonist. In a related aspect, the present disclosure provides a PSMP antagonist for use in the treatment of primary sclerosing cholangitis. In another related aspect, the present disclosure provides use of a PSMP antagonist in the manufacture of a medicament for the treatment of primary sclerosing cholangitis.

In another aspect, the present disclosure provides a method of treating primary biliary cholangitis (PBC). The method generally includes administering to a subject having primary biliary cholangitis an effective amount of a PC3-secreted microprotein (PSMP) antagonist. In a related aspect, the present disclosure provides a PSMP antagonist for use in the treatment of primary biliary cholangitis. In another related aspect, the present disclosure provides use of a PSMP antagonist in the manufacture of a medicament for the treatment of primary biliary cholangitis.

In another aspect, the present disclosure provides a disclosure of treating graft-versus-host disease (GVHD). The method generally includes administering to a subject having GVHD an effective amount of a PC3-secreted microprotein (PSMP) antagonist. In a related aspect, the present disclosure provides a PSMP antagonist for use in the treatment of GVHD. In another related aspect, the present disclosure provides use of a PSMP antagonist in the manufacture of a medicament for the treatment of GVHD. In some embodiments, the GVHD to be treated is acute GVHD (aGVHD). In other embodiments, the GVHD to be treated is chronic GVHD (cGVHD).

In another aspect, the present disclosure provides a method of treating systemic lupus erythematosus (SLE). The method generally includes administering to a subject having SLE an effective amount of a PC3-secreted microprotein (PSMP) antagonist. In a related aspect, the present disclosure provides a PSMP antagonist for use in the treatment of SLE. In another related aspect, the present disclosure provides use of a PSMP antagonist in the manufacture of a medicament for the treatment of SLE.

In another aspect, the present disclosure provides a method of treating lupus nephritis. The method generally includes administering to a subject having lupus nephritis an effective amount of a PC3-secreted microprotein (PSMP) antagonist. In a related aspect, the present disclosure provides a PSMP antagonist for use in the treatment of lupus nephritis. In another related aspect, the present disclosure provides use of a PSMP antagonist in the manufacture of a medicament for the treatment of lupus nephritis.

In another aspect, the present disclosure provides a method for treating kidney fibrosis. The method generally includes administering to a subject having kidney fibrosis an effective amount of a PC3-secreted microprotein (PSMP) antagonist. In a related aspect, the present disclosure provides a PSMP antagonist for use in the treatment of kidney fibrosis. In another related aspect, the present disclosure provides use of a PSMP antagonist in the manufacture of a medicament for the treatment of kidney fibrosis. In some embodiments of a method, PSMP antagonist, or use as above, the kidney fibrosis is associated with a disease or disorder selected from IgA nephropathy, membranoproliferative glomerulonephritis, membranous glomerulonephritis, crescentic glomerulonephritis, diabetic nephropathy, hypertension nephropathy, lupus nephritis, hepatic nephropathy, polycystic kidney disease, Alport syndrome, Fabry disease, primary hyperoxaluria, cystinosis, coenzyme Q10-related gene mutations causing focal segmental glomerulosclerosis (FSGS), complement 3 glomerulonephritis, acute or subacute immune complex glomerulonephritis, renal vasculitis, systemic lupus erythematosus (SLE), recent-onset renal artery stenosis (fibromuscular or vasculitic), diabetic kidney disease, chronic urate nephropathy, toxic nephropathies, bacterial pyelonephritis, viral nephropathies, multiple myeloma, and obstructive nephropathy.

In yet another aspect, the present disclosure provides a method for treating acute kidney injury (AKI) or chronic kidney disease (CKD). The method generally includes administering to a subject having AKI or CKD an effective amount of a PC3-secreted microprotein (PSMP) antagonist. In a related aspect, the present disclosure provides a PSMP antagonist for use in the treatment of AKI or CKD. In another related aspect, the present disclosure provides use of a PSMP antagonist in the manufacture of a medicament for the treatment of AKI or CKD. In some embodiments of a method, PSMP antagonist, or use as above for the treatment of AKI, the AKI is rhabdomyolysis-induced. In some embodiments of a method, PSMP antagonist, or use as above for the treatment of CKD, the CKD is caused by a disease or disorder selected from IgA nephropathy, membranoproliferative glomerulonephritis, membranous glomerulonephritis, crescentic glomerulonephritis, diabetic nephropathy, hypertension nephropathy, lupus nephritis, hepatic nephropathy, polycystic kidney disease, Alport syndrome, Fabry disease, primary hyperoxaluria, cystinosis, coenzyme Q10-related gene mutations causing focal segmental glomerulosclerosis (FSGS), complement 3 glomerulonephritis, acute or subacute immune complex glomerulonephritis, renal vasculitis, systemic lupus erythematosus (SLE), recent-onset renal artery stenosis (fibromuscular or vasculitic), diabetic kidney disease, chronic urate nephropathy, toxic nephropathies, bacterial pyelonephritis, viral nephropathies, multiple myeloma, and obstructive nephropathy.

According to the invention, the PSMP antagonist is an antibody that specifically binds to PSMP. Particularly suitable soluble protein antagonists include neutralizing anti-PSMP antibodies. In more specific variations, an antibody is a humanized antibody, a chimeric antibody, or a human antibody. In other, non-mutually exclusive embodiments, an antibody is a single chain antibody and/or a bispecific antibody. Typically, an antibody for use in accordance with the present invention is a monoclonal antibody. In some variations, an antibody includes an immunoglobulin constant such as, for example, an immunoglobulin heavy chain constant region *(e.g.,* an immunoglobulin Fc region).

In some embodiments, a soluble protein PSMP antagonist (*e.g*., a neutralizing anti-PSMP antibody) competes for binding to PSMP with an antibody comprising (i) a heavy chain variable domain (VH) having the amino acid sequence shown in SEQ ID NO:4 and (ii) a light chain variable domain (VL) having the amino acid sequence shown in SEQ ID NO:5. In some such embodiments where the PSMP antagonist is an antibody, the antibody comprises a VH domain comprising complementarity determining regions (CDRs) CDR-H1_{Ab}, CDR-H2_{Ab}, and CDR-H3_{Ab}, where the set of VH CDRs has three or fewer amino acid substitutions relative to a reference set of CDRs CDR-H1_{Ref}, CDR-H2_{Ref}, and CDR-H3_{Ref} in which CDR-H1_{Ref} is a CDR-H1 of SEQ ID NO:4, CDR-H2_{Ref} is a CDR-H2 of SEQ ID NO:4, and CDR-H3_{Ref} is a CDR-H3 of SEQ ID NO:4 *(e.g.,* a set of VH CDRs having zero amino acid substitutions relative to CDR-H1_{Ref}, CDR-H2_{Ref}, and CDR-H3_{Ref}, whereby CDR-H1_{Ab} is a CDR-H1 of SEQ ID NO:4, CDR-H2_{Ab} is a CDR-H2 of SEQ ID NO:4, and CDR-H3_{Ab} is a CDR-H3 of SEQ ID NO:4). Each VH CDR may be defined, for example, according to the Chothia definition, the Kabat definition, the AbM definition, or the contact definition of CDR. In a specific variation, each VH CDR is defined according to the Chothia definition of CDR, whereby CDR-H1_{Ref} has the amino acid sequence shown in residues 31-35 of SEQ ID NO:4, CDR-H2_{Ref} has the amino acid sequence shown in residues 50-69 of SEQ ID NO:4, and CDR-H3_{Ref} has the amino acid sequence shown in residues 99-108 of SEQ ID NO:4; in some such embodiments, the set of VH CDRs has zero amino acid substitutions relative to CDR-H1_{Ref}, CDR-H2_{Ref}, and CDR-H3_{Ref}, whereby CDR-H1_{Ab} has the amino acid sequence shown in residues 31-35 of SEQ ID NO:4, CDR-H2_{Ab} has the amino acid sequence shown in residues 50-69 of SEQ ID NO:4, and CDR-H3_{Ab} has the amino acid sequence shown in residues 99-108 of SEQ ID NO:4. In some variations, the antibody comprises a humanized VH domain derived from a VH domain having the amino acid sequence shown in SEQ ID NO:4. In other embodiments, the antibody comprises a VH domain having the amino acid sequence shown in SEQ ID NO:4 *(e.g.,* the antibody is a chimeric antibody comprising the VH domain of SEQ ID NO:4).

In other, non-mutually exclusive embodiments in which the PSMP antagonists is an antibody that competes for binding to PSMP with an antibody comprising (i) a heavy chain variable domain (VH) having the amino acid sequence shown in SEQ ID NO:4 and (ii) a light chain variable domain (VL) having the amino acid sequence shown in SEQ ID NO:5, the antibody comprises a VL domain comprising complementarity determining regions (CDRs) CDR-L1_{AB}, CDR-L2_{Ab}, and CDR-L3_{Ab}, where the set of VL CDRs has three or fewer amino acid substitutions relative to a reference set of CDRs CDR-L1_{Ref}, CDR-L2_{Ref}, and CDR-L3_{Ref} in which CDR-L1_{Ref} is a CDR-L1 of SEQ ID NO:5, CDR-L2_{Ref} is a CDR-L2 of SEQ ID NO:5, and CDR-L3_{Ref} is a CDR-L3 of SEQ ID NO:5 *(e.g.,* a set of VL CDRs having zero amino acid substitutions relative to CDR-L1_{Ref}, CDR-L2_{Ref}, and CDR-L3_{Ref}, whereby CDR-L1_{Ab} is a CDR-L1 of SEQ ID NO:5, CDR-L2_{Ab} is a CDR-L2 of SEQ ID NO:5, and CDR-L3_{Ab} is a CDR-L3 of SEQ ID NO:5). Each VL CDR may be defined, for example, according to the Chothia definition, the Kabat definition, the AbM definition, or the contact definition of CDR. In a specific variation, each VL CDR is defined according to the Chothia definition of CDR, whereby CDR-L1_{Ref} has the amino acid sequence shown in residues 24-34 of SEQ ID NO:5, CDR-L2_{Ref} has the amino acid sequence shown in residues 50-56 of SEQ ID NO:5; and CDR-L3_{Ref} has the amino acid sequence shown in residues 89-97 of SEQ ID NO:5; in some such embodiments, the set of VL CDRs has zero amino acid substitutions relative to CDR-L1_{Ref}, CDR-L2_{Ref}, and CDR-L3_{Ref}, whereby CDR-L1_{Ab} has the amino acid sequence shown in residues 24-34 of SEQ ID NO:5, CDR-L2_{Ab} has the amino acid sequence shown in residues 50-56 of SEQ ID NO:5, and CDR-L3_{Ab} has the amino acid sequence shown in residues 89-97 of SEQ ID NO:5. In some variations, the antibody comprises a humanized VL domain derived from a VL domain having the amino acid sequence shown in SEQ ID NO:5. In other embodiments, the antibody comprises a VL domain having the amino acid sequence shown in SEQ ID NO:5 *(e.g.,* the antibody is a chimeric antibody comprising the VL domain of SEQ ID NO:5).

In some embodiments of a method, PSMP antagonist, or use as above, the treatment is a combination therapy.

### DEFINITIONS

Unless defined otherwise, all technical and scientific terms used herein have the same meaning as commonly understood by one of ordinary skill in the art pertinent to the methods and compositions described. As used herein, the following terms and phrases have the meanings ascribed to them unless specified otherwise.

The terms "a," "an," and "the" include plural referents, unless the context clearly indicates otherwise.

As used herein, the term "antagonist" denotes a compound that reduces the activity of another compound in a biological setting.

Within the present invention, a "PSMP antagonist" is a compound that reduces the receptor-mediated biological activity (*e.g.*, chemotactic ability) of PSMP on a target cell. Antagonists may exert their action by competing with PSMP for binding sites on a cell-surface receptor, by binding to PSMP and preventing it from binding to a cell-surface receptor, by otherwise interfering with receptor function, by reducing production of PSMP, or by other means.

A "polypeptide" is a polymer of amino acid residues joined by peptide bonds, whether produced naturally or synthetically. Polypeptides of less than about 30 amino acid residues are commonly referred to as "peptides."

A "protein" is a macromolecule comprising one or more polypeptide chains. A protein may also comprise non-peptidic components, such as carbohydrate groups. Carbohydrates and other non-peptidic substituents may be added to a protein by the cell in which the protein is produced and will vary with the type of cell. Proteins are defined herein in terms of their amino acid backbone structures; substituents such as carbohydrate groups are generally not specified but may be present nonetheless.

The term "antibody" is used herein to denote proteins produced by the body in response to the presence of an antigen and that bind to the antigen, as well as antigen-binding fragments and engineered variants thereof. Hence, the terms "antibody" and "antibodies" include polyclonal antibodies, affinity-purified polyclonal antibodies, monoclonal antibodies, and antigen-binding antibody fragments, such as F(ab')₂ and Fab fragments. Genetically engineered intact antibodies and fragments, such as chimeric antibodies, humanized antibodies, single-chain Fv fragments, single-chain antibodies, diabodies, minibodies, linear antibodies, multivalent or multispecific hybrid antibodies, and the like are also included. Thus, the term "antibody" is used expansively to include any protein that comprises an antigen-binding site and is capable of binding to its antigen.

The term "genetically engineered antibodies" means antibodies wherein the amino acid sequence has been varied from that of a native (*i.e.,* naturally occurring) antibody. Because of the relevance of recombinant DNA techniques in the generation of antibodies, one need not be confined to the sequences of amino acids found in natural antibodies; antibodies can be redesigned to obtain desired characteristics. The possible variations are many and range from the changing of just one or a few amino acids to the complete redesign of, for example, the variable or constant region. Changes in the constant region will, in general, be made in order to improve or alter characteristics, such as complement fixation, interaction with cells and other effector functions. Typically, changes in the variable region will be made in order to improve the antigen binding characteristics, improve variable region stability, or reduce the risk of immunogenicity.

An "antigen-binding site" is that portion of an antibody that is sufficient to bind to its antigen. The minimum such region is typically an immunoglobulin variable domain or fragment thereof, or a genetically engineered variant of an immunoglobulin variable domain or fragment thereof. Single-domain binding sites can be generated from camelid antibodies *(see* Muyldermans and Lauwereys, J. Mol. Recog. 12:131-140, 1999; Nguyen et al., EMBO J. 19:921-930, 2000) or from VH domains of other species to produce single-domain antibodies ("dAbs"; *see* Ward et al., Nature 341:544-546, 1989; US Patent No. 6,248,516 to Winter et al.)*.* In certain variations, an antigen-binding site is a polypeptide region having only 2 complementarity determining regions (CDRs) of a naturally or non-naturally (*e.g*., mutagenized) occurring heavy chain variable domain or light chain variable domain, or combination thereof *(see, e.g.,* Pessi et al., Nature 362:367-369, 1993; Qiu et al., Nature Biotechnol. 25:921-929, 2007). More commonly, an antigen-binding site comprises both a heavy chain variable domain and a light chain variable domain that bind to a common epitope. Within the present invention, in addition to "an antigen-binding site," an antibody may further comprise one or more additional components such as, for example, one or more of a second antigen-binding site (which may bind to the same or a different epitope or to the same or a different antigen), a peptide linker, an immunoglobulin constant domain, an immunoglobulin hinge, an amphipathic helix *(see, e.g.,* Pack and Pluckthun, Biochem. 31:1579-1584, 1992), a non-peptide linker, an oligonucleotide *(see, e.g.,* Chaudri et al., FEES Letters 450:23-26, 1999), and the like, and may be a monomeric or multimeric protein. In addition to proteins having the structure of an intact, native antibody (*i.e.,* a tetramer consisting of two immunoglobulin heavy chains and two immunoglobulin light chains), examples of molecules comprising an antigen-binding site are generally known in the art and include, for example, Fv fragments, single-chain Fv fragments (scFv), Fab fragments, diabodies, minibodies, Fab-scFv fusions, bispecific (scFv)₄-IgG, and bispecific (scFv)₂-Fab. (*See, e.g.,* Hu et al., Cancer Res. 56:3055-3061, 1996; Atwell et al., Molecular Immunology 33:1301-1312, 1996; Carter and Merchant, Curr. Opin. Biotechnol. 8:449-454, 1997; Zuo et al., Protein Engineering 13:361-367, 2000; and Lu et al., J. Immunol. Methods 267:213-226, 2002.)

As used herein, the term "immunoglobulin" refers to a protein consisting of one or more polypeptides substantially encoded by immunoglobulin gene(s). One form of immunoglobulin constitutes the basic structural unit of an intact, native antibody. This form is a tetramer and consists of two identical pairs of immunoglobulin chains, each pair having one light and one heavy chain. In each pair, the light and heavy chain variable regions are together responsible for binding to an antigen, and the constant regions are responsible for the antibody effector functions. Immunoglobulins typically function as antibodies in a vertebrate organism. Five classes of immunoglobulin protein (IgG, IgA, IgM, IgD, and IgE) have been identified in higher vertebrates. IgG comprises the major class; it normally exists as the second most abundant protein found in plasma. In humans, IgG consists of four subclasses, designated IgG1, IgG2, IgG3, and IgG4. The heavy chain constant regions of the IgG class are identified with the Greek symbol γ. For example, immunoglobulins of the IgG1 subclass contain a γ1 heavy chain constant region. Each immunoglobulin heavy chain possesses a constant region that consists of constant region protein domains (CH1, hinge, CH2, and CH3; IgG3 also contains a CH4 domain) that are essentially invariant for a given subclass in a species. DNA sequences encoding human and non-human immunoglobulin chains are known in the art. (*See, e.g.,* Ellison et al., DNA 1:11-18, 1981; Ellison et al., Nucleic Acids Res. 10:4071-4079, 1982; Kenten et al., Proc. Natl. Acad. Sci. USA 79:6661-6665, 1982; Seno et al., Nuc. Acids Res. 11:719-726, 1983; Riechmann et al., Nature 332:323-327, 1988; Amster et al., Nuc. Acids Res. 8:2055-2065, 1980; Rusconi and Kohler, Nature 314:330-334, 1985; Boss et al., Nuc. Acids Res. 12:3791-3806, 1984; Bothwell et al., Nature 298:380-382, 1982; van der Loo et al., Immunogenetics 42:333-341, 1995; Karlin et al., J. Mol. Evol. 22:195-208, 1985; Kindsvogel et al., DNA 1:335-343, 1982; Breiner et al., Gene 18:165-174, 1982; Kondo et al., Eur. J. Immunol. 23:245-249, 1993; and GenBank Accession No. J00228.) For a review of immunoglobulin structure and function see Putnam, The Plasma Proteins, Vol V, Academic Press, Inc., 49-140, 1987; and Padlan, Mol. Immunol. 31:169-217, 1994. The term "immunoglobulin" is used herein for its common meaning, denoting an intact antibody, its component chains, or fragments of chains, depending on the context.

Full-length immunoglobulin "light chains" (about 25 Kd or 214 amino acids) are encoded by a variable region gene at the NH₂-terminus (encoding about 110 amino acids) and a by a kappa or lambda constant region gene at the COOH-terminus. Full-length immunoglobulin "heavy chains" (about 50 Kd or 446 amino acids) are encoded by a variable region gene (encoding about 116 amino acids) and a gamma, mu, alpha, delta, or epsilon constant region gene (encoding about 330 amino acids), the latter defining the antibody's isotype as IgG, IgM, IgA, IgD, or IgE, respectively. Within light and heavy chains, the variable and constant regions are joined by a "J" region of about 12 or more amino acids, with the heavy chain also including a "D" region of about 10 more amino acids. (*See generally* Fundamental Immunology (Paul, ed., Raven Press, N.Y., 2nd ed. 1989), Ch. 7).

An immunoglobulin "Fv" fragment contains a heavy chain variable domain (VH) and a light chain variable domain (VL), which are held together by non-covalent interactions. An immunoglobulin Fv fragment thus contains a single antigen-binding site. The dimeric structure of an Fv fragment can be further stabilized by the introduction of a disulfide bond via mutagenesis. (*See* Almog et al., Proteins 31:128-138, 1998.)

As used herein, the term "single-chain antibody" refers to an antibody having an antigen-binding site contained within a single polypeptide chain *(e.g.,* the variable regions from both heavy and light chains within a single polypeptide chain). The term "single-chain Fv" refers to a single-chain antibody that comprises the variable regions from both heavy and light chains but lacks constant regions. In general, a single-chain Fv further comprises a polypeptide linker between the VH and VL domains, which enables it to form the desired structure that allows for antigen binding. Single-chain antibodies are discussed in detail by, for example, Pluckthun in The Pharmacology of Monoclonal Antibodies, vol. 113 (Rosenburg and Moore eds., Springer-Verlag, New York, 1994), pp. 269-315. (*See also* WIPO Publication WO 88/01649; U.S. Patent Nos. 4,946,778 and 5,260,203; Bird et al., Science 242:423-426, 1988.) Single-chain antibodies can also be bi-specific and/or humanized.

A "Fab fragment" contains one light chain and the CH1 and variable regions of one heavy chain. The heavy chain of a Fab fragment cannot form a disulfide bond with another heavy chain molecule. A "Fab' fragment" contains one light chain and one heavy chain that contains more of the constant region, between the CH1 and CH2 domains, such that an interchain disulfide bond can be formed between two heavy chains to form a "F(ab')₂ fragment" containing two light chains and two heavy chains.

The terms "Fc fragment" and "Fc region," as used herein, are synonymous and refer to the portion of an antibody that is responsible for binding to antibody receptors on cells and the C1q component of complement. Fc stands for "fragment crystalline," the fragment of an antibody that will readily form a protein crystal. Distinct protein fragments, which were originally described by proteolytic digestion, can define the overall general structure of an immunoglobulin protein. As originally defined in the literature, the Fc fragment consists of the disulfide-linked heavy chain hinge regions and CH2 and CH3 domains. However, more recently the term has been applied to a single chain consisting of CH3, CH2, and at least a portion of the hinge sufficient to form a disulfide-linked dimer with a second such chain. For a review of immunoglobulin structure and function, *see* Putnam, The Plasma Proteins, Vol. V (Academic Press, Inc., 1987), pp. 49-140; and Padlan, Mol. Immunol. 31:169-217, 1994. As used herein, the term Fc includes variants of naturally occurring sequences.

An immunoglobulin light or heavy chain variable region consists of a framework region interrupted by three hypervariable regions. The term "hypervariable region," also referred to herein as "complementarity determining region" ("CDR"), refers to the amino acid residues of an antibody that are responsible for antigen binding. A CDR may be defined according to any of several known methods of analysis. Examples of such methods include, *e.g.,* the Kabat definition, the Chothia definition, the AbM definition and the contact definition. The Kabat definition is a standard for numbering the residues in an antibody and is typically used to identify CDR regions. *See, e.g.,* Johnson & Wu, Nucleic Acids Res. 28:214-8, 2000. The Chothia definition is similar to the Kabat definition, but the Chothia definition takes into account positions of certain structural loop regions. *See, e.g.,* Chothia et al., J. Mol. Biol. 196:901-17, 1986; Chothia et al., Nature 342:877-83, 1989. The AbM definition uses an integrated suite of computer programs produced by Oxford Molecular Group that model antibody structure. *See, e.g.,* Martin et al., 1989, Proc. Natl. Acad. Sci. USA 86:9268-9272, 1989; "AbM™, A Computer Program for Modeling Variable Regions of Antibodies," Oxford, UK; Oxford Molecular, Ltd. The AbM definition models the tertiary structure of an antibody from primary sequence using a combination of knowledge databases and *ab initio* methods, such as those described by Samudrala et al., "Ab Initio Protein Structure Prediction Using a Combined Hierarchical Approach," in PROTEINS, Structure, Function and Genetics Suppl. 3:194-198, 1999. The contact definition is based on an analysis of the available complex crystal structures. *See, e.g.,* MacCallum et al., J. Mol. Biol. 5:732-45, 1996. CDRs L1, L2, and L3 of a VL domain are also referred to herein, respectively, as CDR-L1, CDR-L2, and CDR-L3; CDRs H1, H2, and H3 of a VH domain are also referred to herein, respectively, as CDR-H1, CDR-H2, and CDR-H3

"Framework region" are those variable domain residues other than the hypervariable region residues (*e.g*., other than the residues of CDRs as defined by the Kabat definition, the Chothia definition, the AbM definition, or the contact definition of CDR). The sequences of the framework regions of different light or heavy chains are relatively conserved within a species. Thus, a "human framework region" is a framework region that is substantially identical (about 85% or more, usually 90-95% or more) to the framework region of a naturally occurring human immunoglobulin. The framework region of an antibody, that is the combined framework regions of the constituent light and heavy chains, serves to position and align the CDRs.

"Chimeric antibodies" are antibodies whose light and heavy chain genes have been constructed, typically by genetic engineering, from immunoglobulin variable and constant region genes belonging to different species. For example, the variable segments of the genes from a mouse monoclonal antibody may be joined to human constant region-encoding segments (e.g., human gamma 1 or gamma 3 heavy chain genes, and human kappa light chain genes). A therapeutic chimeric antibody is thus a hybrid protein, typically composed of the variable or antigen-binding domains from a mouse antibody and the constant domains from a human antibody, although other mammalian species may be used. Specifically, a chimeric antibody is produced by recombinant DNA technology in which all or part of the hinge and constant regions of an immunoglobulin light chain, heavy chain, or both, have been substituted for the corresponding regions from another animal's immunoglobulin light chain or heavy chain. In this way, the antigen-binding portion of the parent monoclonal antibody is grafted onto the backbone of another species' antibody. Chimeric antibodies may be optionally "cloaked" with a human-like surface by replacement of exposed residues, the result of which is a "veneered antibody."

As used herein, the term "human antibody" means an antibody having an amino acid sequence corresponding to that of an antibody produced by a human and/or which has been made using any of the techniques for making human antibodies known to those skilled in the art or disclosed herein. This definition of a human antibody includes antibodies comprising at least one human heavy chain polypeptide or at least one human light chain polypeptide. One such example is an antibody comprising murine light chain and human heavy chain polypeptides. Human antibodies can be produced using various techniques known in the art. In one embodiment, the human antibody is selected from a phage library, where that phage library expresses human antibodies. *See, e.g.,* Vaughan et al., Nature Biotechnology 14:309-314, 1996; Sheets et al., Proc. Natl. Acad. Sci. USA 95:6157-6162, 1998; Hoogenboom and Winter, J. Mol. Biol. 227:381, 1991; Marks et al., J. Mol. Biol. 222:581, 1991. Human antibodies can also be made by immunization of animals into which human immunoglobulin loci have been transgenically introduced in place of the endogenous loci, *e.g.,* mice in which the endogenous immunoglobulin genes have been partially or completely inactivated. This approach is described, *e.g*., in U.S. Patent Nos. 5,545,807; 5,545,806; 5,569,825; 5,625,126; 5,633,425; 5,661,016; and 7,041,870. Alternatively, the human antibody may be prepared by immortalizing human B lymphocytes that produce an antibody directed against a target antigen (such B lymphocytes may be recovered from an individual or may have been immunized *in vitro*). *See, e.g.,* Cole et al., "Monoclonal Antibodies and Cancer Therapy," Alan R. Liss, p. 77, 1985; Boerner et al., J. Immunol. 147:86-95, 1991; and U.S. Patent No. 5,750,373.

The term "humanized immunoglobulin" refers to an immunoglobulin comprising a human framework region and one or more CDRs from a non-human (*e.g*., a mouse or rat) immunoglobulin. The non-human immunoglobulin providing the CDRs is called the "donor" and the human immunoglobulin providing the framework is called the "acceptor." Constant regions need not be present, but if they are, they are substantially identical to human immunoglobulin constant regions, *i.e.,* at least about 85-90%, preferably about 95% or more identical. Hence, all parts of a humanized immunoglobulin, except possibly the CDRs, are substantially identical to corresponding parts of natural human immunoglobulin sequences. In some instances, humanized antibodies may retain non-human residues within the human variable region framework domains to enhance proper binding characteristics (*e.g*., mutations in the frameworks may be required to preserve binding affinity when an antibody is humanized). A "humanized antibody" is an antibody comprising a humanized light chain immunoglobulin and/or a humanized heavy chain immunoglobulin. For example, a humanized antibody would not encompass a typical chimeric antibody as defined above because, *e.g*., the entire variable region of a chimeric antibody is non-human (or, in the case of a veneered antibody, substantially non-human).

A "bispecific" antibody is an antibody having two different antigen binding sites, each having a different specificity. Bispecific antibodies may be produced by a variety of methods including, e.g., by chemical conjugation with cross-linkers, by somatic fusion of two hybridoma lines (quadroma), and by genetic engineering. *See generally, e.g.,* Sedykh et al., Drug Design, Development and Therapy 12:195-208, 2018.

The term "diabodies" refers to small antibody fragments with two antigen-binding sites, which fragments comprise a heavy chain variable domain (VH) connected to a light chain variable domain (VL) in the same polypeptide chain (VH-VL). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA 90:6444-6448, 1993.

The term "minibody" refers herein to a polypeptide that encodes only 2 complementarity determining regions (CDRs) of a naturally or non-naturally (*e.g*., mutagenized) occurring heavy chain variable domain or light chain variable domain, or combination thereof. Examples of minibodies are described by, *e.g.,* Pessi et al., Nature 362:367-369, 1993; and Qiu et al., Nature Biotechnol. 25:921-929, 2007.

The term "linear antibodies" refers to the antibodies described in Zapata et al., Protein Eng. 8:1057-1062, 1995. Briefly, these antibodies comprise a pair of tandem Fd segments (VH-CH1-VH-CH1) which form a pair of antigen binding regions. Linear antibodies can be bispecific or monospecific.

As used herein, the term "monoclonal antibody" refers to an antibody obtained from a population of substantially homogeneous antibodies, *i.e.,* the individual antibodies comprising the population are identical in amino acid sequence except for possible naturallyoccurring mutations that may be present in minor amounts. Monoclonal antibodies are highly specific, being directed against a single antigenic site. Furthermore, in contrast to polyclonal antibody preparations, which typically include different antibodies directed against different determinants (epitopes), each monoclonal antibody is directed against a single determinant on the antigen. The modifier "monoclonal" indicates the character of the antibody as being obtained from a substantially homogeneous population of antibodies and is not to be construed as requiring production of the antibody by any particular method. For example, a monoclonal antibody for use in accordance with the present invention may be made by a hybridoma method such as, *e.g.,* first described by Kohler and Milstein (Nature 256:495, 1975), or may be made by recombinant DNA methods such as, *e.g*., described in U.S. Patent No. 4,816,567. Monoclonal antibodies may also be isolated from phage libraries generated using, *e.g.,* techniques described in McCafferty et al. (Nature 348:552-554, 1990).

Reference herein to "treating" or "treatment of" liver, lung, or kidney fibrosis includes such treatment in the context of any of various diseases or disorders associated with liver, lung, or kidney fibrosis. A disease or disorder is "associated with" liver, lung, or kidney fibrosis if liver, lung, or kidney fibrosis is understood by a clinician to be part of the pathology of the disease or disorder *(e.g.,* an indicator of disease progression and/or a cause of loss of function of the liver, lung, or kidney). "Pathology" in this context includes fibrosis as either a predicted or an actual pathological tissue change as part of disease or disorder progression; accordingly, a subject having a disease or disorder associated with liver, lung, or kidney fibrosis and receiving administration of a PSMP antagonist in accordance with the present disclosure may or may not have fibrosis as an actual physical manifestation of the disease or disorder at the time of treatment. Further, reference herein to treating liver, lung, or kidney fibrosis in a subject and characterization of the liver, lung, or kidney fibrosis as "associated with" a disease or disorder means that the subject being treated either has or is at risk of developing the specified disease or disorder.

Unless the context clearly dictates otherwise, general reference herein to "disease or disorder" includes reference to liver fibrosis, lung fibrosis, kidney fibrosis, graft-versus-host disease (GVHD), or systemic lupus erythematosus (SLE), as well as to particular diseases or disorders associated with liver, lung, or kidney fibrosis (*e.g*., non-alcoholic fatty liver disease (NAFLD), alcoholic liver disease (ALD), primary sclerosing cholangitis (PSC), primary biliary cholangitis (PBC), acute kidney injury (AKI), chronic kidney disease (CKD), lupus nephritis, IgA nephropathy, or membranous glomerulonephritis).

The term "alternative scaffold" refers to a non-antibody protein in which one or more regions may be diversified to produce one or more binding domains that specifically bind to a target molecule *(e.g.,* PSMP). In some embodiments, the binding domain binds the target molecule with specificity and affinity similar to that of an antibody. Exemplary alternative scaffolds include those derived from fibronectin *(e.g.,* Adnectins^{™}), the β-sandwich *(e.g.,* iMab), lipocalin *(e.g.,* Anticalins^{®}), EETI-II/AGRP, BPTI/LACI-D1/ITI-D2 *(e.g.,* Kunitz domains), protein A *(e.g.,* Affibody^{®}), ankyrin repeats (*e.g*., DARPins), gamma-B-crystallin/ubiquitin *(e.g.,* Affilins), CTLD₃ *(e.g.,* Tetranectins), Fynomers, and Avimers. Additional information on alternative scaffolds is provided in Binz et al., Nat. Biotechnol. 23:1257-1268, 2005; Skerra, Current Opin. in Biotech. 18:295-304, 2007; and Silacci et al., J. Biol. Chem. 289:14392-14398, 2014;. An alternative scaffold protein that has been engineered to function as a PSMP antagonist (*e.g*., by specifically binding to and neutralizing PSMP) may be referred to herein an "alternative scaffold PSMP antagonist" or a "PSMP antagonist based on an alternative scaffold."

An "inhibitory polynucleotide" is a DNA or RNA molecule that reduces or prevents expression (transcription or translation) of a second (target) polynucleotide (*e.g*., a gene or mRNA encoding PSMP). Inhibitory polynucleotides include antisense polynucleotides, ribozymes, and external guide sequences. The term "inhibitory polynucleotide" further includes DNA and RNA molecules that encode the actual inhibitory species, such as DNA molecules that encode ribozymes.

The terms "treat" and "treatment" are used broadly to denote therapeutic and prophylactic interventions that favorably alter a pathological state.

The term "effective amount" of a composition, in the context of treatment as described herein, is an amount sufficient to effect any one or more beneficial or desired results. For prophylactic use, beneficial or desired results include eliminating or reducing the risk, lessening the severity, or delaying the outset of a disease or disorder, including biochemical, histological, and/or physical symptoms of the disease or disorder, its complications, and intermediate pathological phenotypes presenting during development of the disease or disorder. For therapeutic use, beneficial or desired results include clinical results such as amelioration of or reducing incidence of one or more symptoms of the disease or disorder, decreasing the dose of other medications required to treat the disease or disorder, enhancing the effect of another medication, delaying the progression of the disease or disorder, and/or producing an improvement in organ function in a patient. For example, in the context of treatment of liver, lung, or kidney fibrosis by administration of a PSMP antagonist to a subject as described herein, an amount of such agent that is sufficient to delay the outset of, reduce the severity of, or reduce the progression of fibrosis in the subject, or sufficient to produce an improvement in liver, lung, or kidney function in the subject, is considered "an effective amount" of the PSMP antagonist.

An effective dosage can be administered in one or more administrations. An effective amount of a composition is administered according to the methods of the present invention in an "effective regime." The term "effective regime" refers to a combination of amount of the composition being administered and dosage frequency adequate to accomplish treatment. For purposes of the present invention, an effective dosage of a composition is an amount sufficient to accomplish prophylactic or therapeutic treatment either directly or indirectly. As is understood in the clinical context, an effective dosage of a composition may or may not be achieved in conjunction with another drug composition. Thus, an "effective amount" may be considered in the context of administering one or more therapeutic agents, and a single agent may be considered to be given in an effective amount if, in conjunction with one or more other agents, a desirable result may be or is achieved.

The term "patient" or "subject," in the context of treating a disease or disorder as described herein, includes mammals such as, for example, humans and other primates. The term also includes domesticated animals such as, *e.g.*, cows, hogs, sheep, horses, dogs, and cats.

The term "combination therapy" refers to a therapeutic regimen that involves the provision of at least two distinct therapies to achieve an indicated therapeutic effect. For example, a combination therapy may involve the administration of two or more chemically distinct active ingredients, or agents, for example, a PSMP antagonist (*e.g.*, an anti-PSMP antibody) and another agent such as, *e.g.,* another anti-inflammatory or anti-fibrotic agent. The distinct therapies constituting a combination therapy may be delivered, *e.g.,* as simultaneous, overlapping, or sequential dosing regimens. In the context of the administration of two or more chemically distinct agents, it is understood that the active ingredients may be administered as part of the same composition or as different compositions. When administered as separate compositions, the compositions comprising the different active ingredients may be administered at the same or different times, by the same or different routes, using the same or different dosing regimens, all as the particular context requires and as determined by the attending physician.

It is understood that wherever embodiments are described herein with the language "comprising," otherwise analogous embodiments described in terms of "consisting of" and/or "consisting essentially of" are also provided.

The term "and/or," *e.g.,* "X and/or Y," shall be understood to mean either "X and Y" or "X or Y" and shall be taken to provide explicit support for both meanings or for either meaning.

Where aspects or embodiments of the invention are described in terms of a Markush group or other grouping of alternatives, the present invention encompasses not only the entire group listed as a whole, but each member of the group individually and all possible subgroups of the main group, but also the main group absent one or more of the group members. The present invention also envisages the explicit exclusion of one or more of any of the group members in the claimed invention.

### BRIEF DESCRIPTION OF THE DRAWINGS

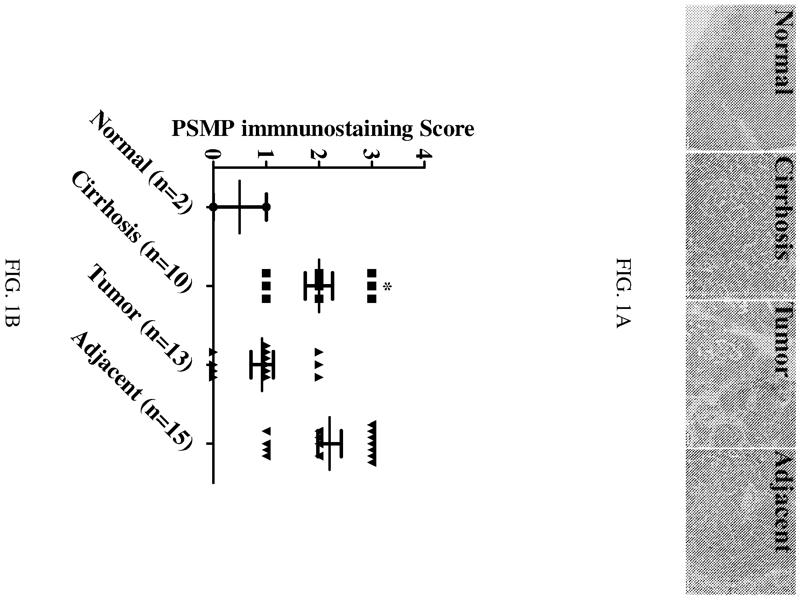

FIGs. 1A and 1B illustrate upregulation of PSMP in cirrhotic and adjacent, nontumor liver tissues *(see* Example 1). Immunohistochemistry was performed as described in Example 6.

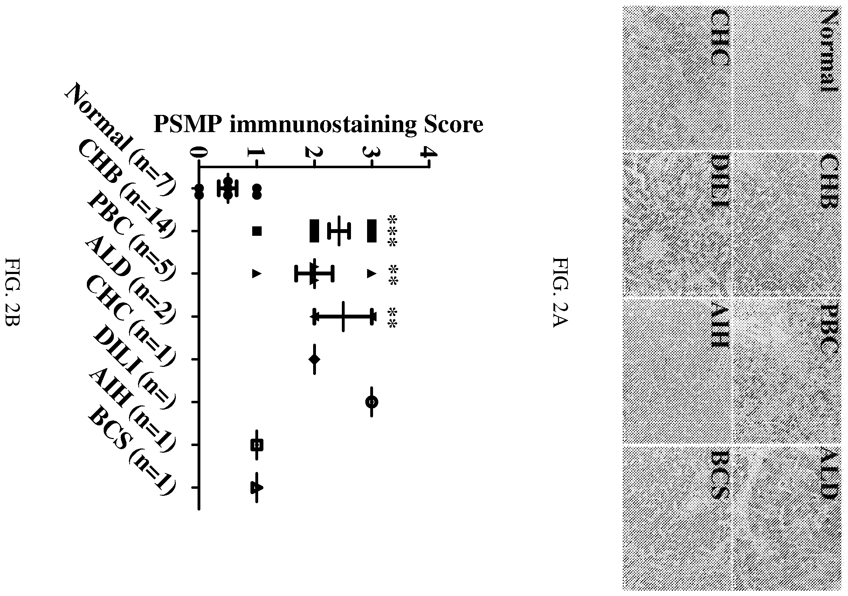

FIGs. 2A and 2B illustrate upregulation of PSMP in human liver fibrotic tissue with different causes *(see* Example 1). Immunohistochemistry was performed as described in Example 6.

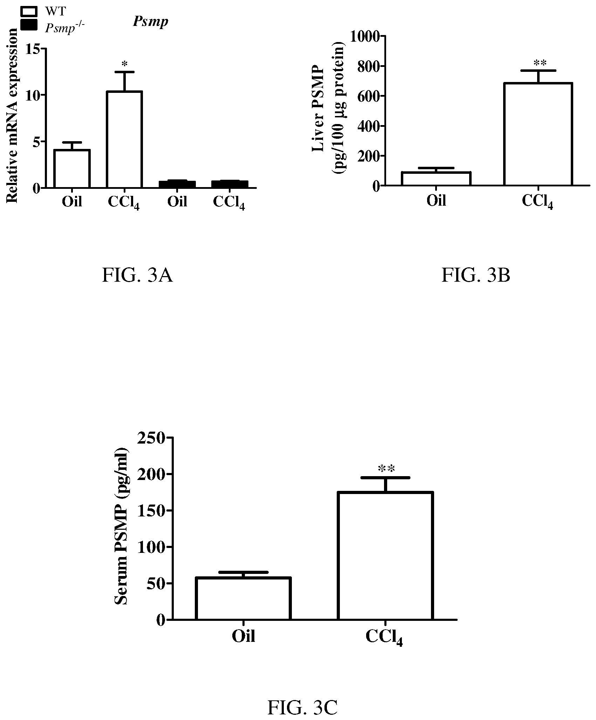

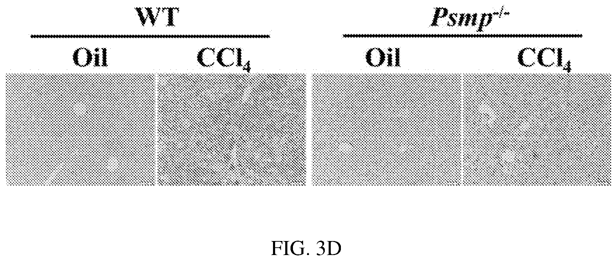

FIGs. 3A-3D illustrate upregulation of PSMP in a mouse model of liver fibrosis induced by CCl₄ *(see* Example 1). Murine CCl₄-induced liver fibrosis model, quantitative real-time PCR assays (FIG. 3A), cytometric bead assays (CBA) (FIGs. 3B and 3C), and immunohistochemistry (FIG. 3D) were performed as described in Example 6.

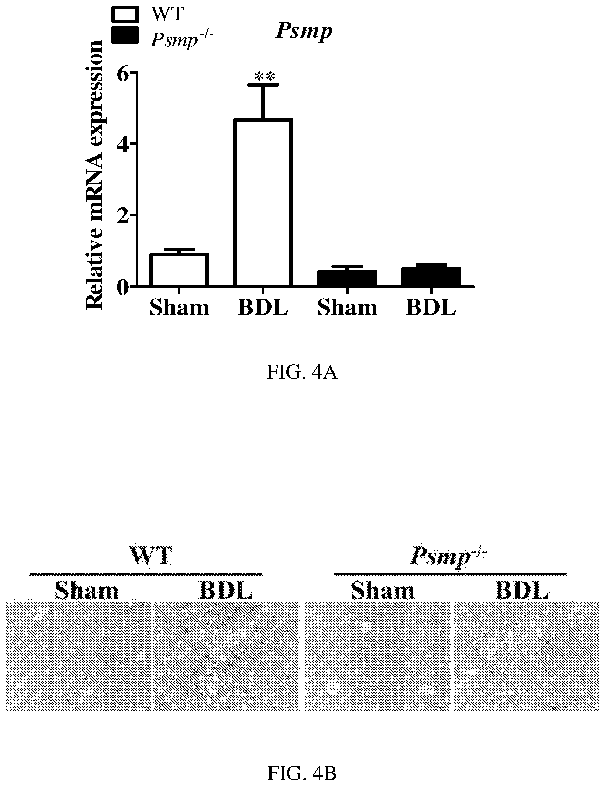

FIGs. 4A and 4B illustrate upregulation of PSMP in a mouse model of liver fibrosis induced by bile duct ligation *(see* Example 1). Murine BDL liver fibrosis model, immunohistochemistry, and quantitative real-time PCR assays were performed as described in Example 6.

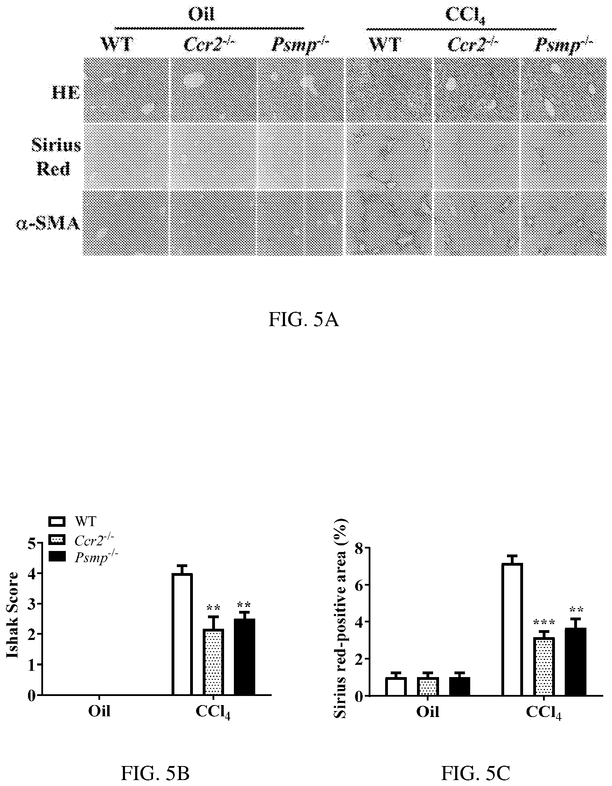

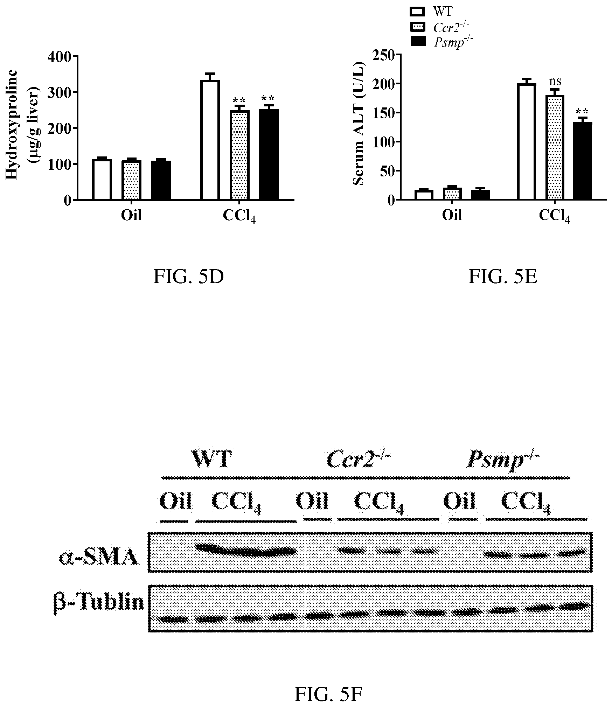

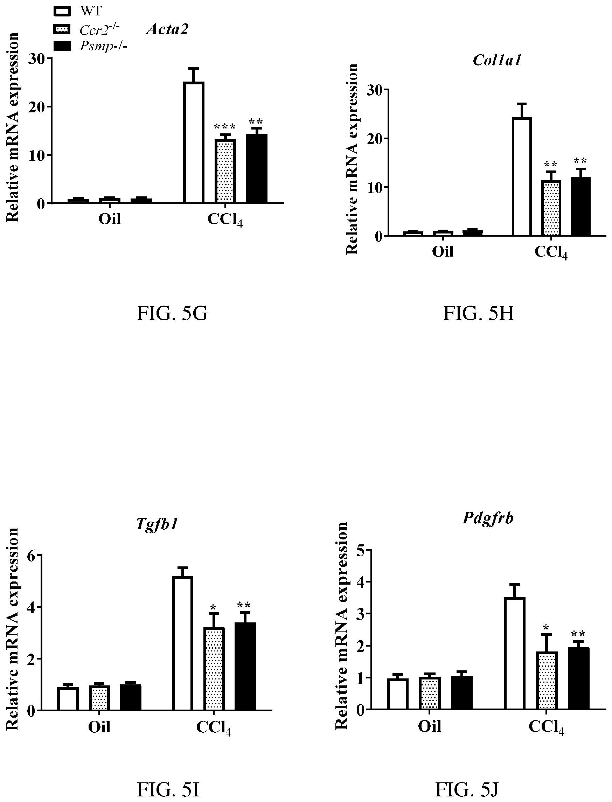

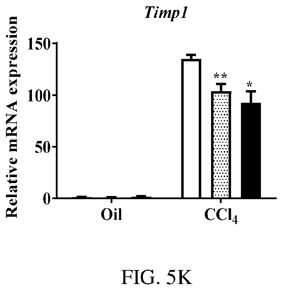

FIGs. 5A-5K illustrate protection against liver fibrosis in PSMP knockout mice using a CCl₄-induced fibrosis model *(see* Example 2). Murine CCl₄-induced liver fibrosis model, immunohistochemistry, immunoblotting analysis, hydroxyproline determination, and quantitative real-time PCR assays were performed as described in Example 6.

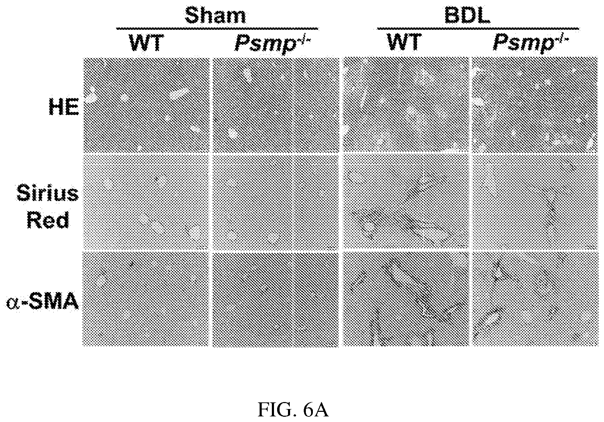

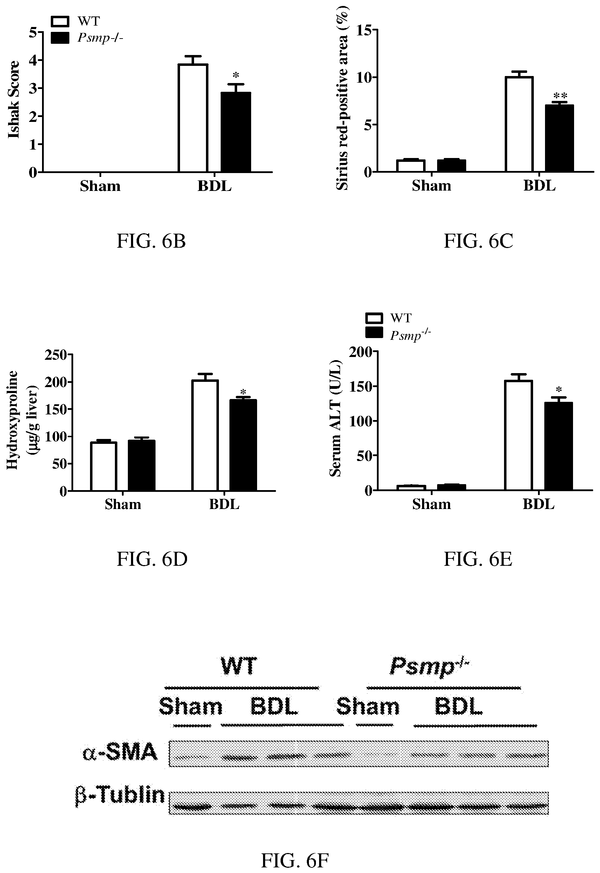

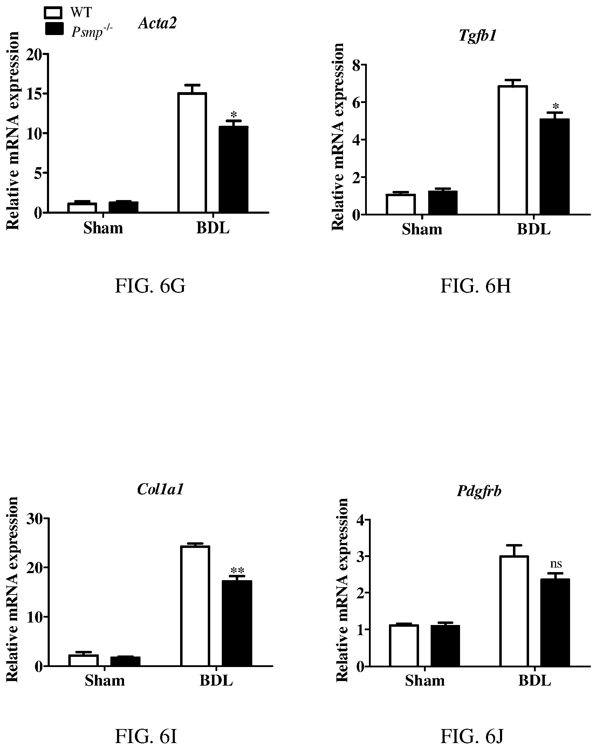

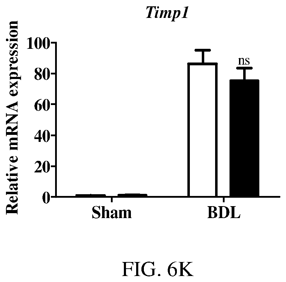

FIGs. 6A-6K illustrate protection against liver fibrosis in PSMP knockout mice using a BDL-induced fibrosis model *(see* Example 2). Murine BDL-induced liver fibrosis model, immunohistochemistry, immunoblotting analysis, hydroxyproline determination, and quantitative real-time PCR assays were performed as described in Example 6.

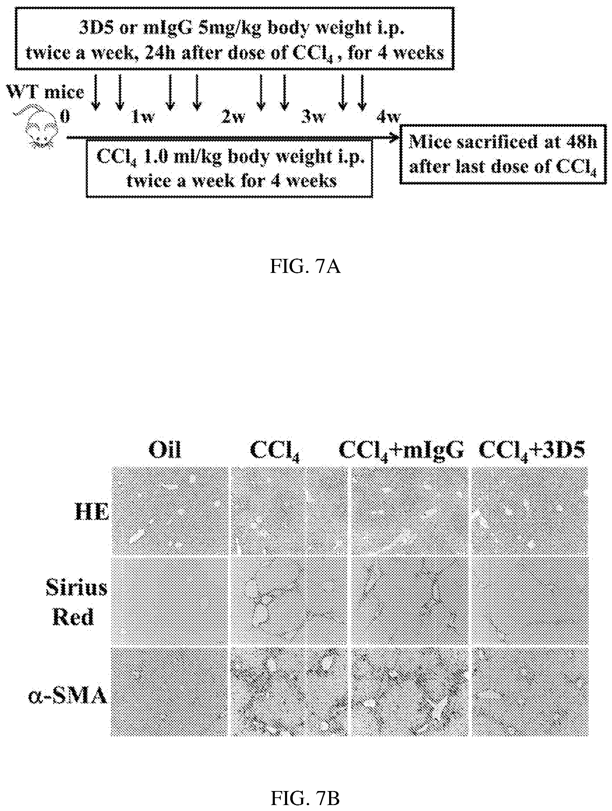

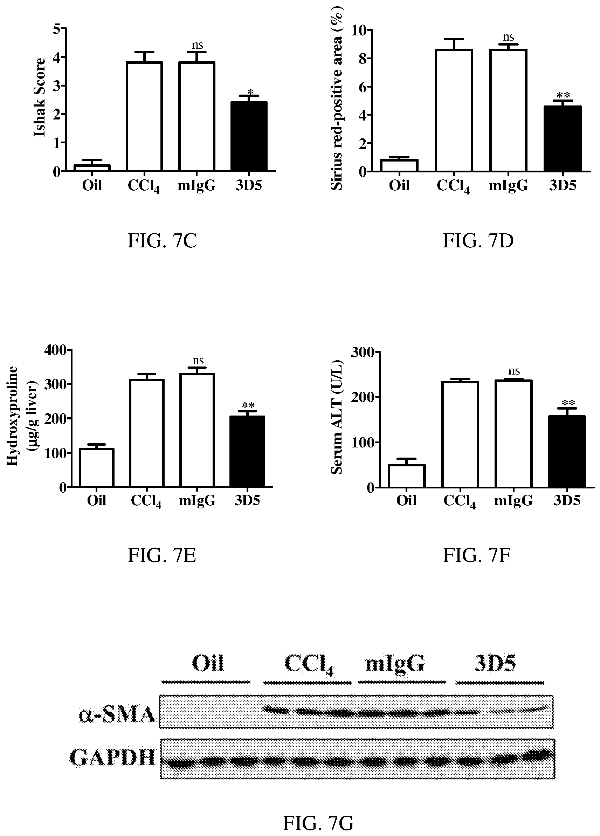

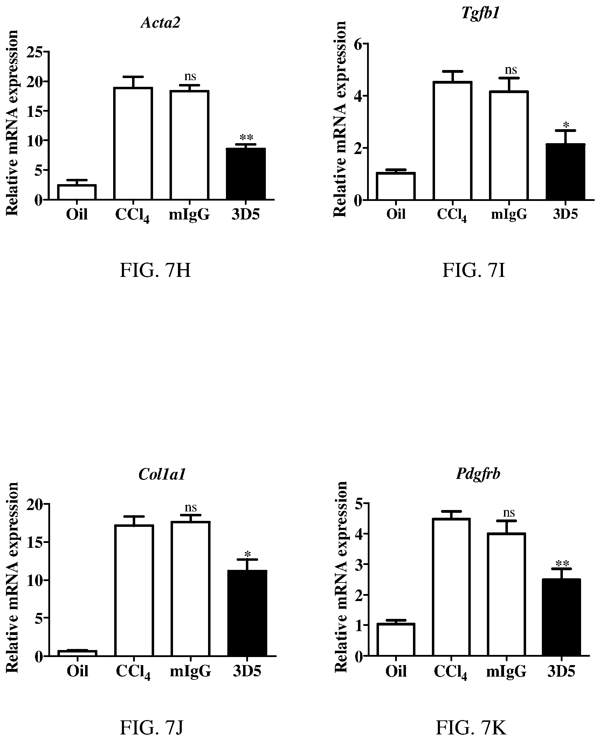

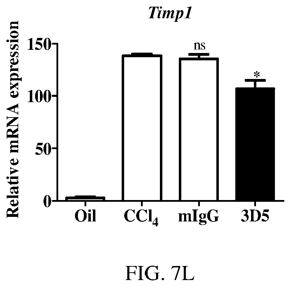

FIGs. 7A-7L illustrate protective effects of a neutralizing anti-PSMP antibody in a CCl₄-induced fibrosis model *(see* Example 3). Murine CCl₄-induced liver fibrosis model, antibody treatments, immunohistochemistry, immunoblotting analysis, hydroxyproline determination, and quantitative real-time PCR assays were performed as described in Example 6.

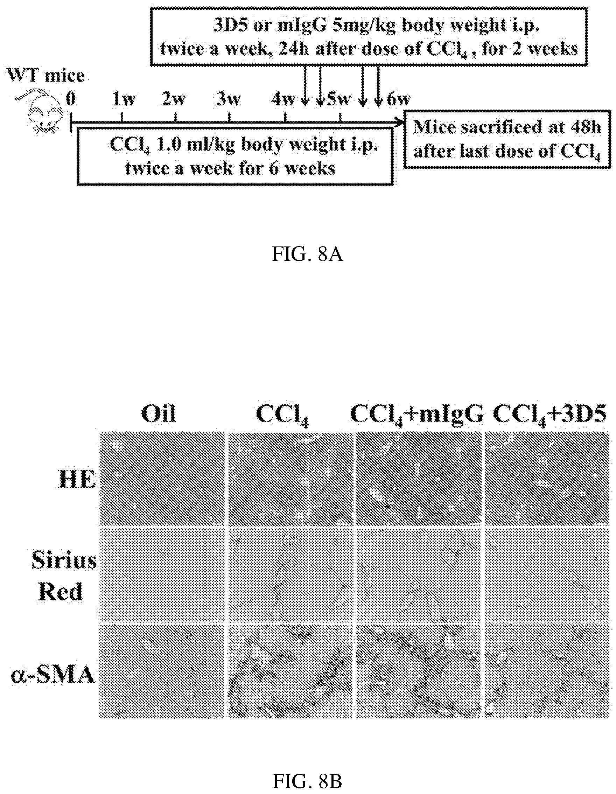

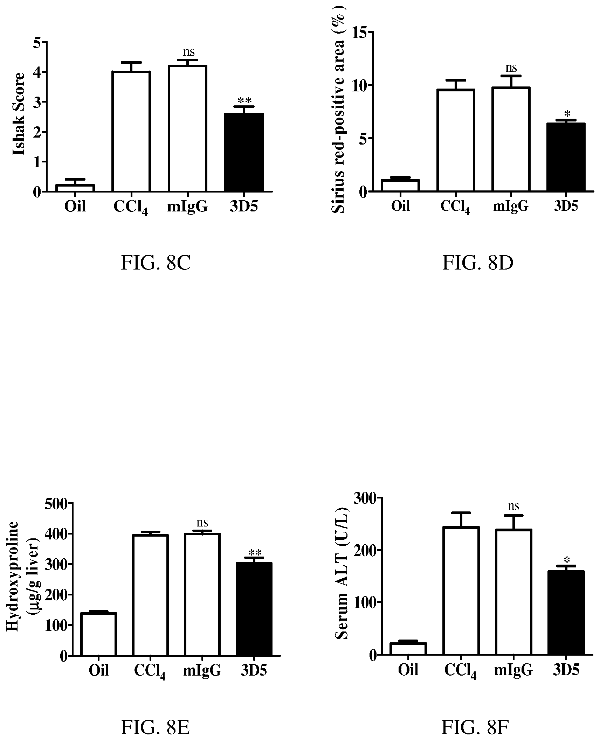

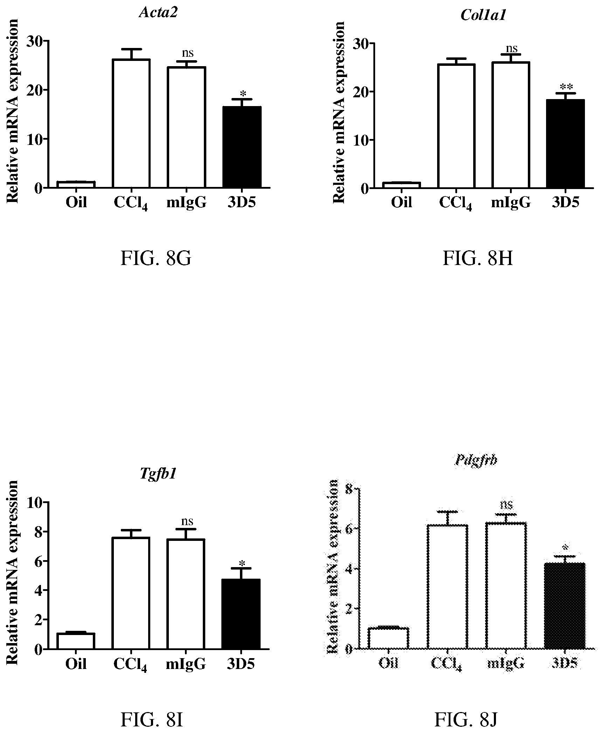

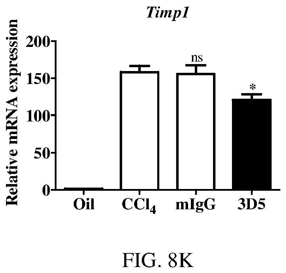

FIGs. 8A-8K illustrate therapeutic effects of a neutralizing anti-PSMP antibody in a CCl₄-induced fibrosis model *(see* Example 3). Murine CCl₄-induced liver fibrosis model, antibody treatments, immunohistochemistry, hydroxyproline determination, and quantitative real-time PCR assays were performed as described in Example 6.

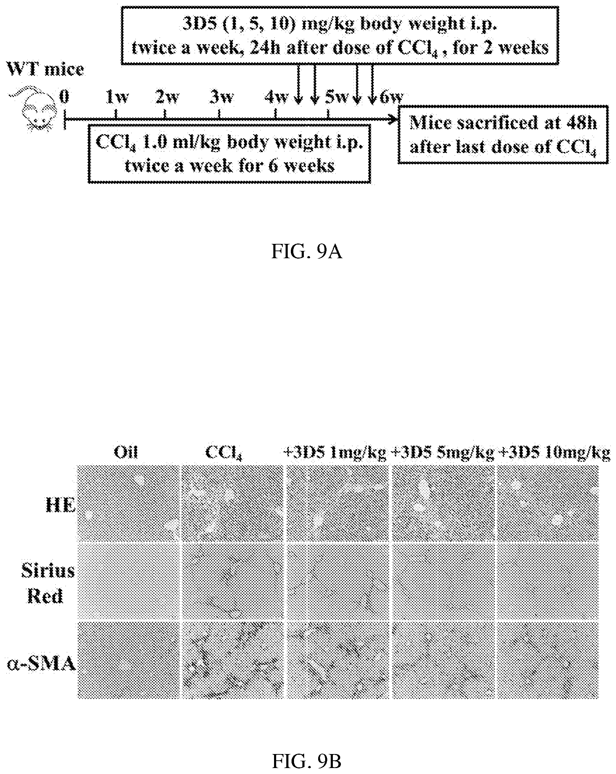

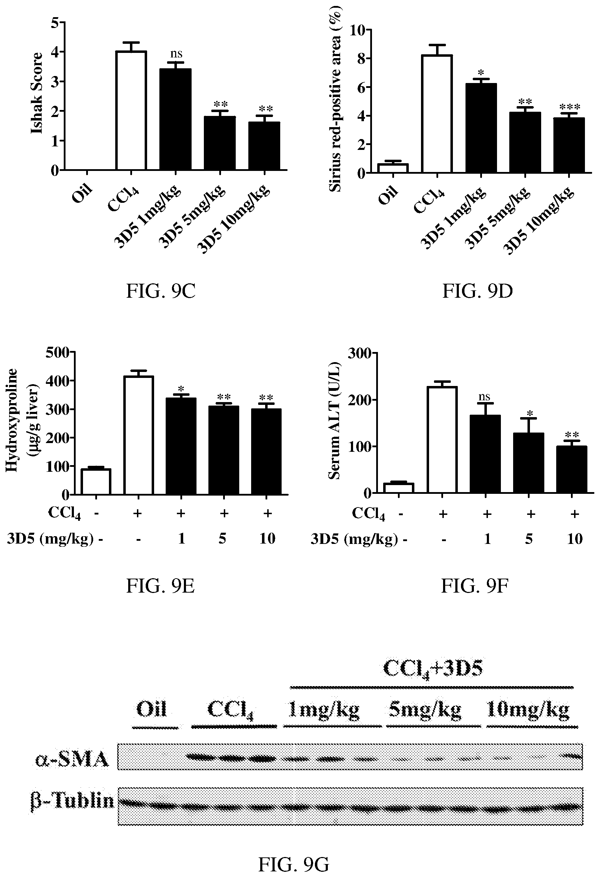

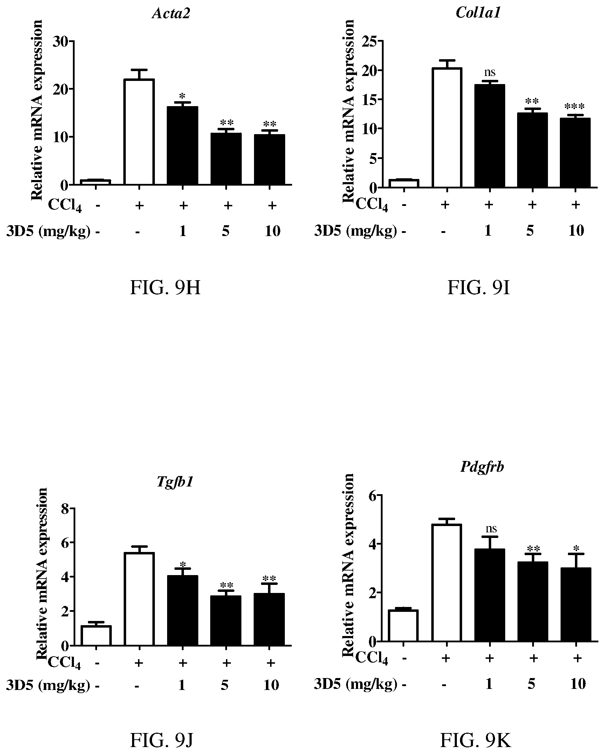

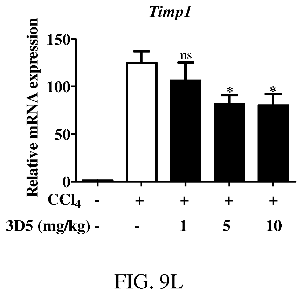

FIGs. 9A-9L illustrate dose-dependent therapeutic effects of a neutralizing anti-PSMP antibody in a CCl₄-induced fibrosis model (*see* Example 3). Murine CCl₄-induced liver fibrosis model, antibody treatments, immunohistochemistry, immunoblotting analysis, hydroxyproline determination, and quantitative real-time PCR assays were performed as described in Example 6.

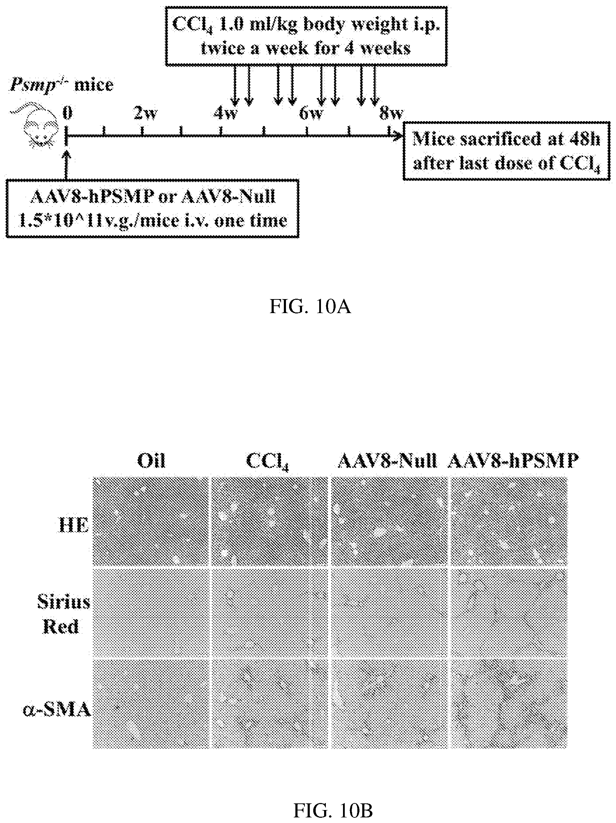

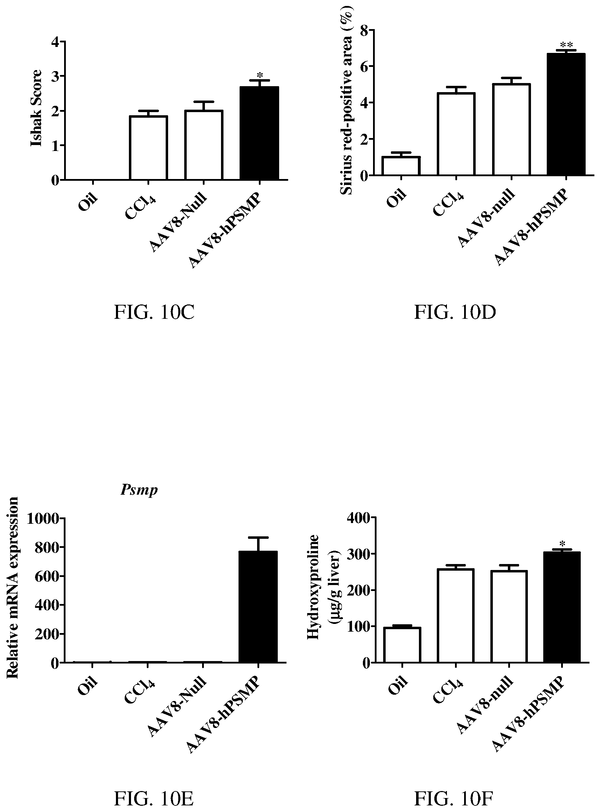

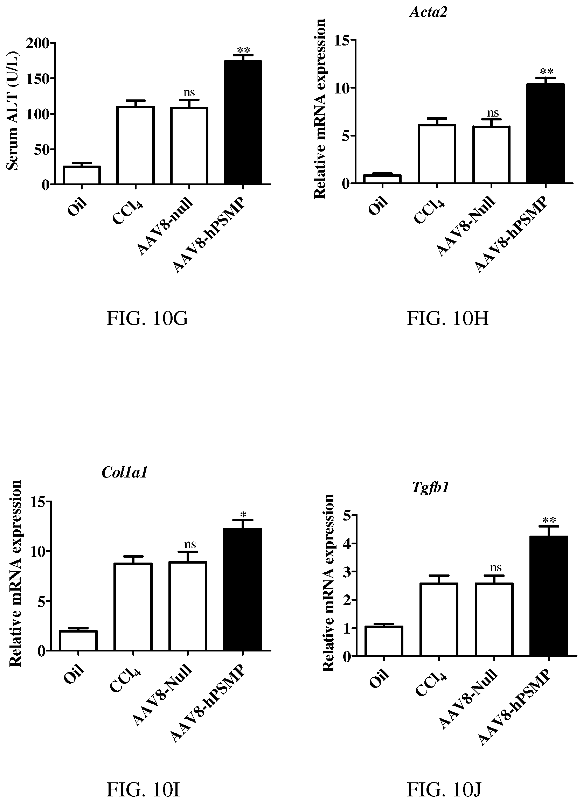

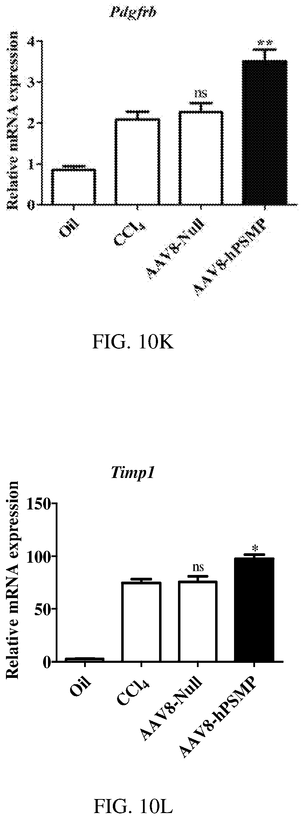

FIGs. 10A-10L illustrate use of an AAV8-hPSMP vector to restore PSMP expression and promote liver fibrosis in PSMP knockout mice *(see* Example 4). Murine CCl₄-induced liver fibrosis model, AAV8 construction and injection, immunohistochemistry, hydroxyproline determination, and quantitative real-time PCR assays were performed as described in Example 6.

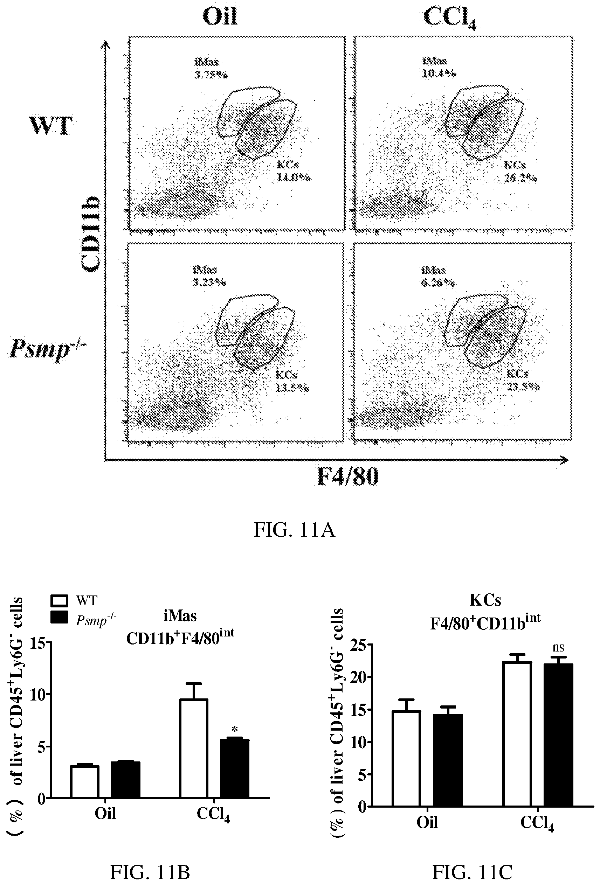

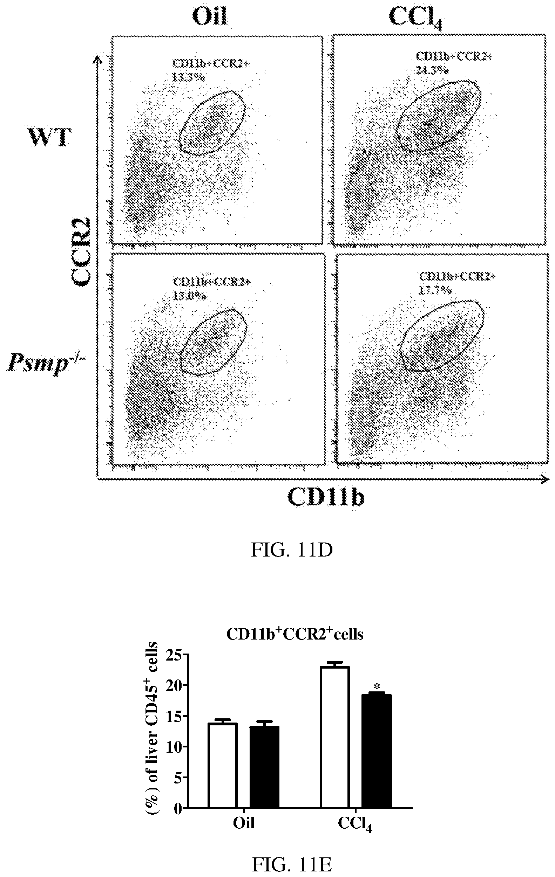

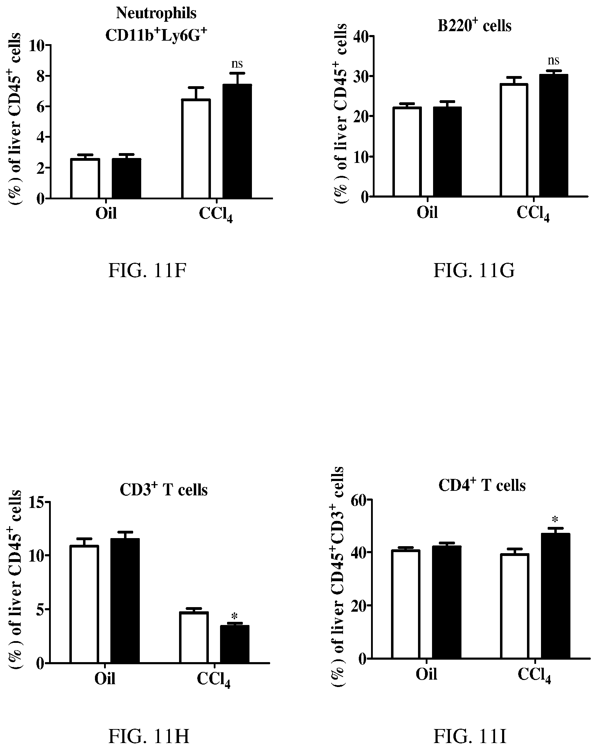

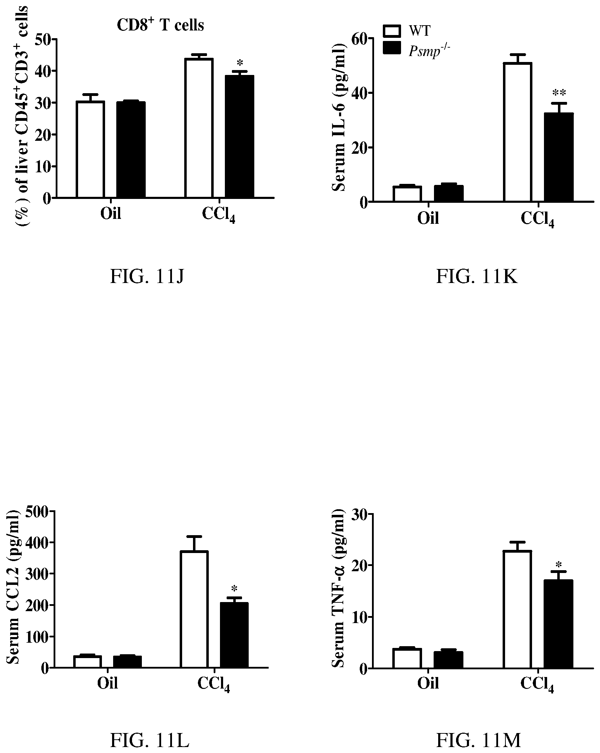

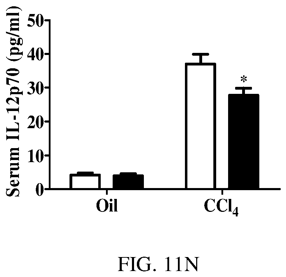

FIGs. 11A-11N illustrate effects of PSMP deficiency on hepatic immune cells and pro-inflammatory cytokine production *(see* Example 5). Reductions in hepatic macrophage infiltration (determined by flow cytometry; FIGs. 11A-11E) and pro-inflammatory cytokine production (determined by cytometric bead assay (CBA); FIGs. 11K-11N) were observed in PSMP knockout mice treated with CCl₄. Other immune cells such as neutrophils, B cells, and T cells were investigated using flow cytometry and found to be not affected (FIGs. 11F-11J). Murine CCl₄-induced liver fibrosis model, flow cytometry, and CBA were performed as described in Example 6.

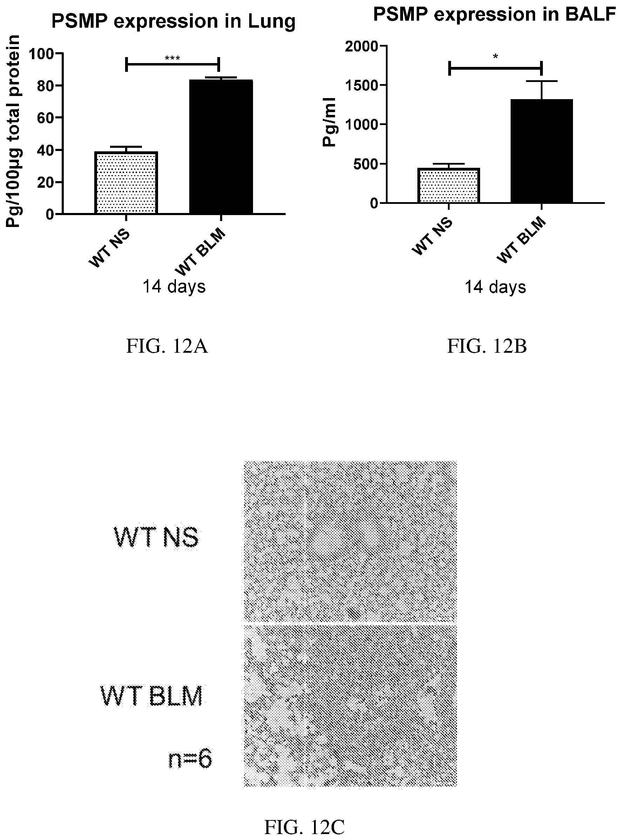

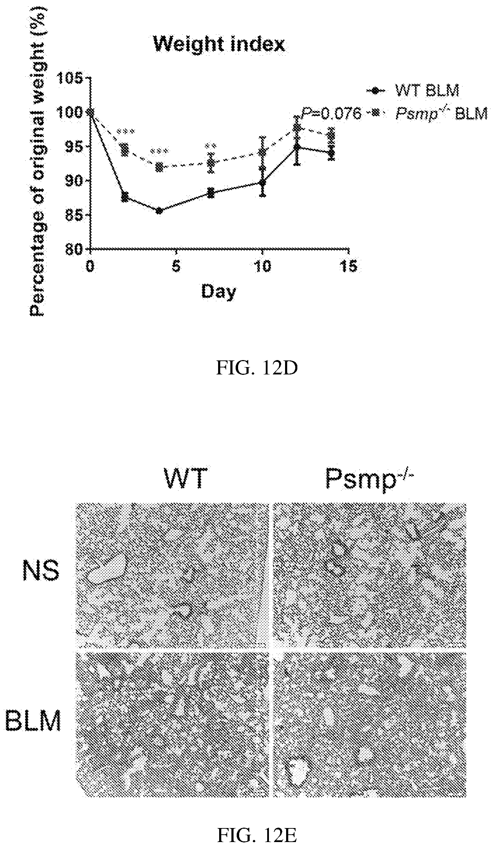

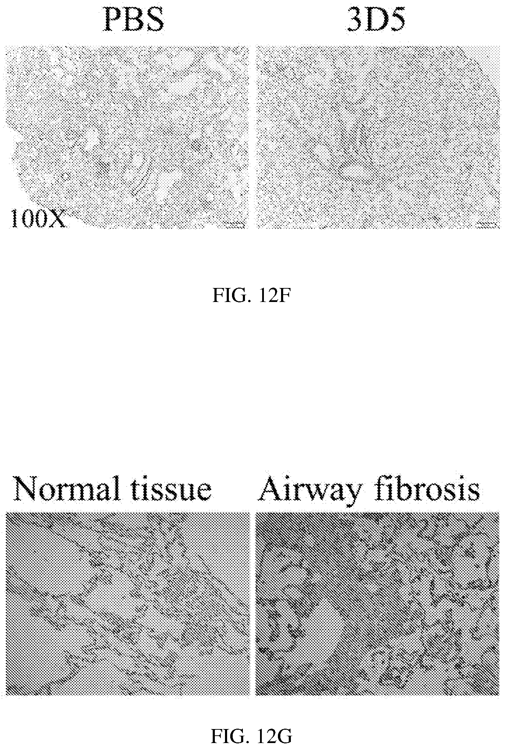

FIGs. 12A-12G illustrate PSMP upregulation and effects of PSMP deficiency during pulmonary fibrosis in a bleomycin mouse model. *See* Example 7. PSMP protein levels in the lung and BALF were measured by cytokine bead assay (FIGs. 12A and 12B). FIG. 12C shows representative lung immunohistochemical staining of PSMP. Weight index was measured for 14 days (FIG. 12D). FIG. 12E shows representative lung histology of Masson's trichrome staining. Expression of α-SMA was determined by immunohistochemistry (FIG. 12F). FIG. 12G shows representative immunohistochemical staining of PSMP in normal and airway fibrosis human lungs. Scale bars, 100 µm. *, *P* < 0.05; **, *P* < 0.01, n≥6/group.

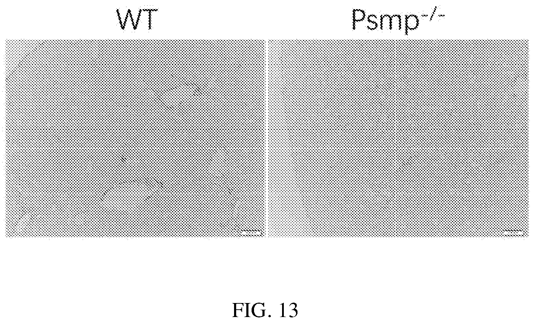

FIG. 13 illustrates a reduction in fibrosis in PSMP knockout mice in a mouse model of nonalcoholic steatohepatitis (NASH). *See* Example 8.

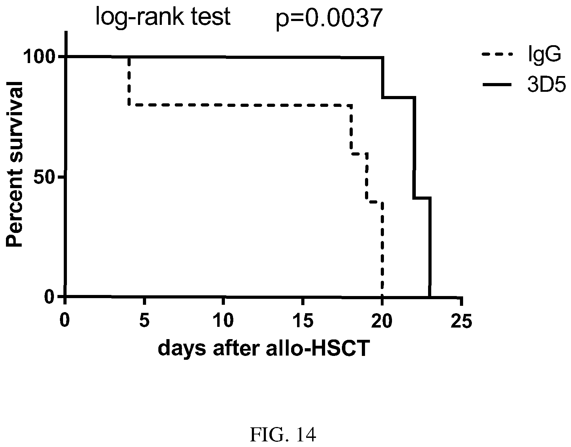

FIG. 14 illustrates a therapeutic effect (extended survival time) of neutralizing anti-PSMP antibody in an acute GVHD (aGVHD) mouse model. *See* Example 9.

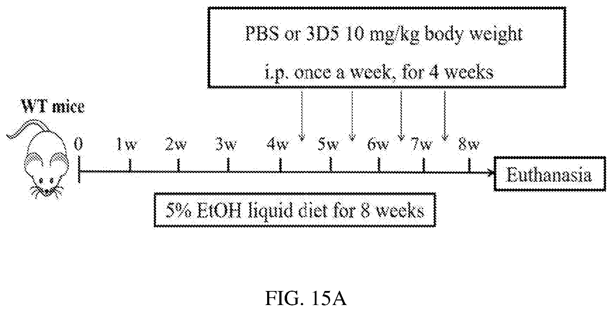

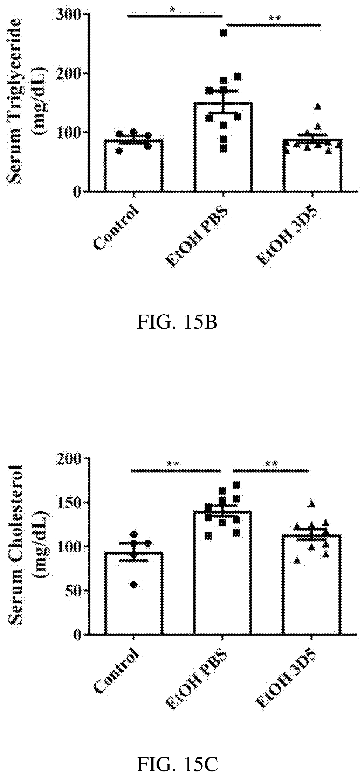

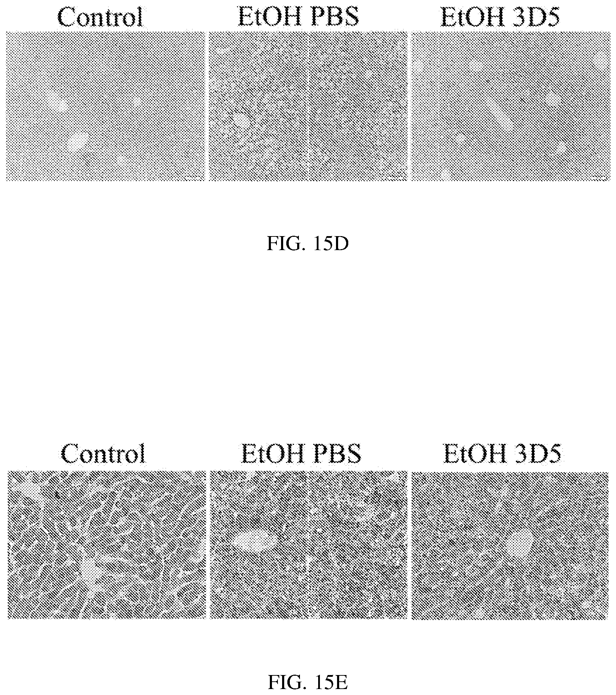

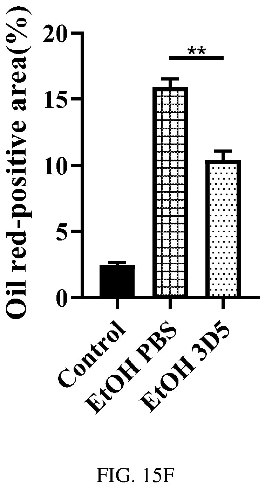

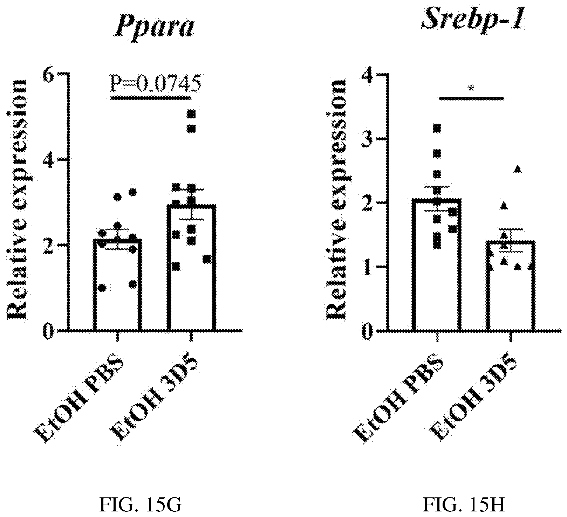

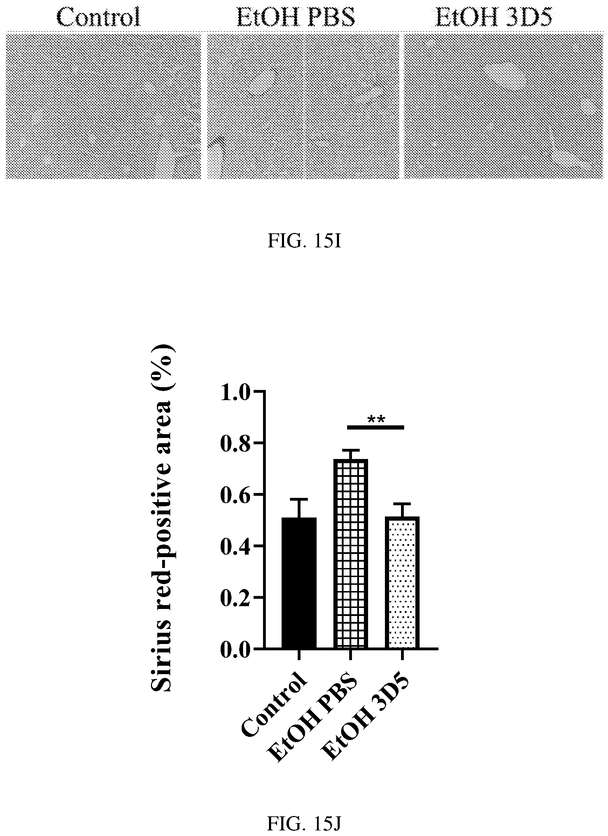

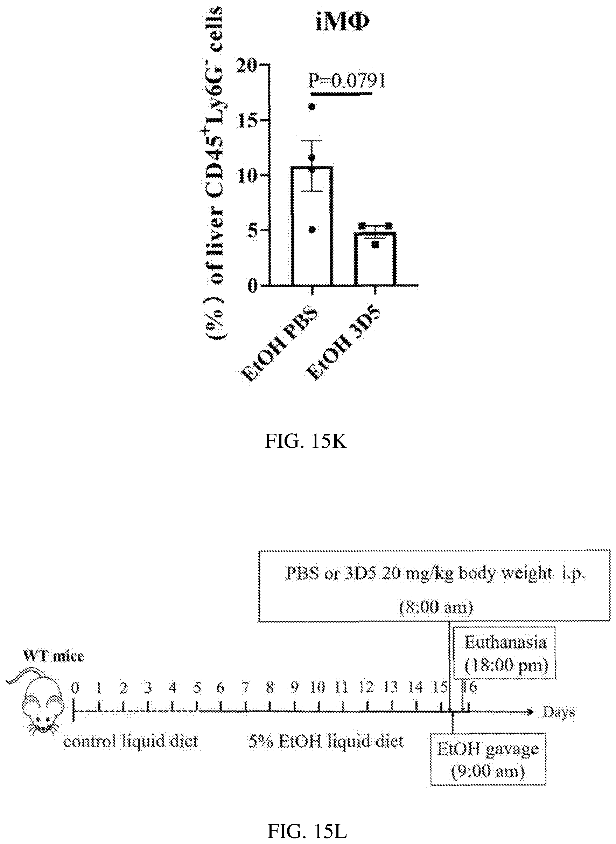

FIGs. 15A-15K illustrate therapeutic effects of a neutralizing anti-PSMP antibody in a chronic ethanol-feeding model. *See* Example 10. FIG. 15A shows a schematic of the experimental design of the PSMP neutralizing antibody 3D5 or PBS treatment in the chronic ethanol-feeding mouse model. Serum triglyceride and cholesterol were measured (FIGs. 15B and 15C). Liver histology was performed by H&E and Oil Red staining (FIGs. 15D and 15E) and quantification of the Oil Red positive area (FIG. 15F). The mRNA levels of hepatic *Ppara* and *Srebp-1* were measured by RT-qPCR (FIGs. 15G and 15H). Further liver histology was performed by Sirius Red staining and its quantification (FIGs. 15I and FIG. 15J). Hepatic non-parenchymal cells were stained and analyzed by flow cytometry. Infiltrating macrophages (iMΦ, CD45⁺ Ly6G⁻ F4/80⁺ CD11b^{high}) were quantified by flow cytometric analysis (FIG. 15K). (Scale bars, 100 µm/50 µm. *, *P* < 0.05; **, *P* < 0.01 by one-way ANOVA , n=3-11/group).

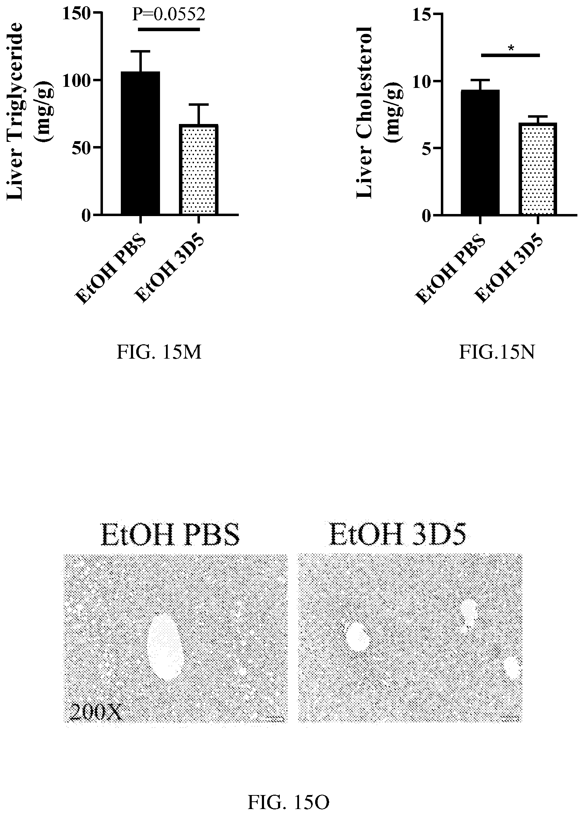

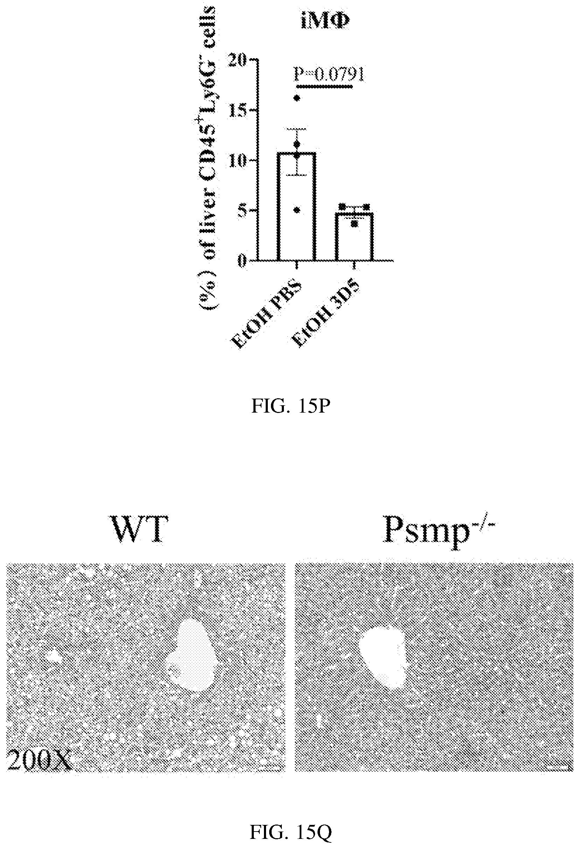

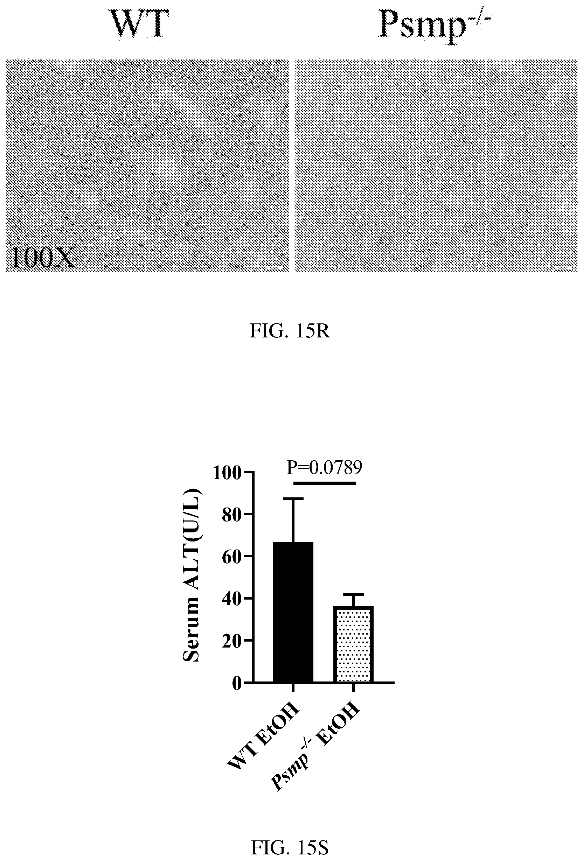

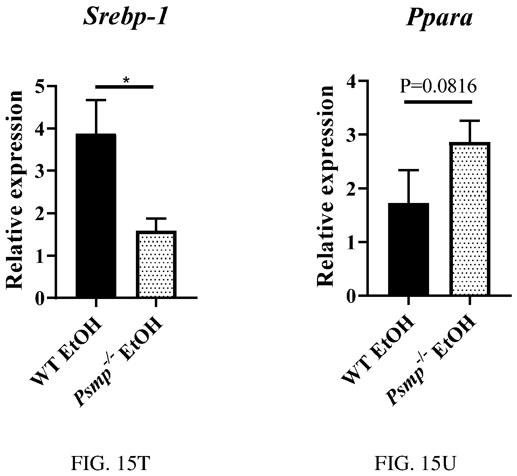

FIGs. 15L-15T illustrate therapeutic effects of a neutralizing anti-PSMP antibody in a chronic-plus-binge alcohol feeding model. *See* Example 10. FIG. 15L shows a schematic of the experimental design of the PSMP neutralizing antibody 3D5 or PBS treatment in the model. Liver triglyceride and cholesterol were measured (FIGs. 15M and 15N). FIG. 15O shows representative H&E staining of liver tissue from PBS- and 3D5-treated mice. Hepatic non-parenchymal cells were stained and analyzed by flow cytometry. Infiltrating macrophages (iMΦ, CD45⁺ Ly6G⁻ F4/80⁺ CD11b^{high}) were quantified by flow cytometric analysis (FIG. 15P). FIGs. 15Q and 15R show representative H&E staining (15Q) and Oil Red O staining (15R) of liver tissue from WT and *Psmp*^{*-*/*-*} mice. Serum levels of ALT were measured (FIG. 15S). The mRNA levels of hepatic *Srebp-1* and *Ppara* were measured by RT-qPCR (FIGs. 15T and 15U). Scale bars, 50 µm. *, *P* < 0.05; **, *P* < 0.01 by one-way ANOVA , n≥4/group.

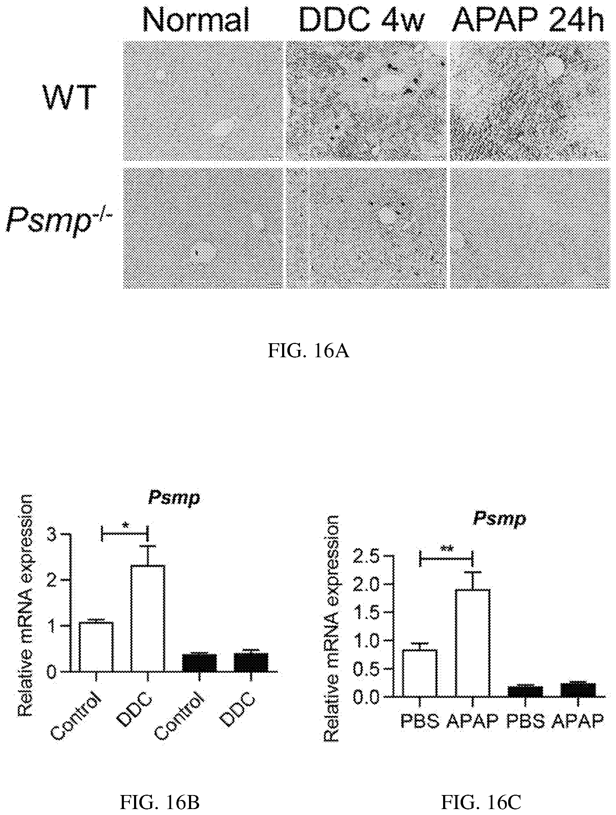

FIGs. 16A-16C illustrate the expression of PSMP in murine liver injury and fibrosis. *See* Example 11. Liver immunohistochemical staining of PSMP (FIG. 16A) and measurement of PSMP mRNA levels by qRT-PCR (FIGs. 16B and 16C) were performed using WT (white bars) and *Psmp*^{*-*/*-*} (black bars) mice treated with DDC-diet or APAP.

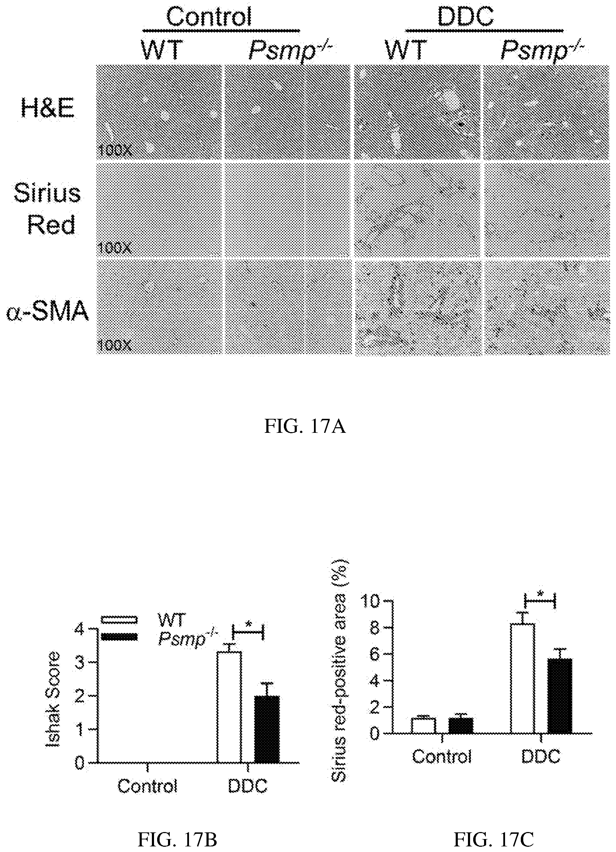

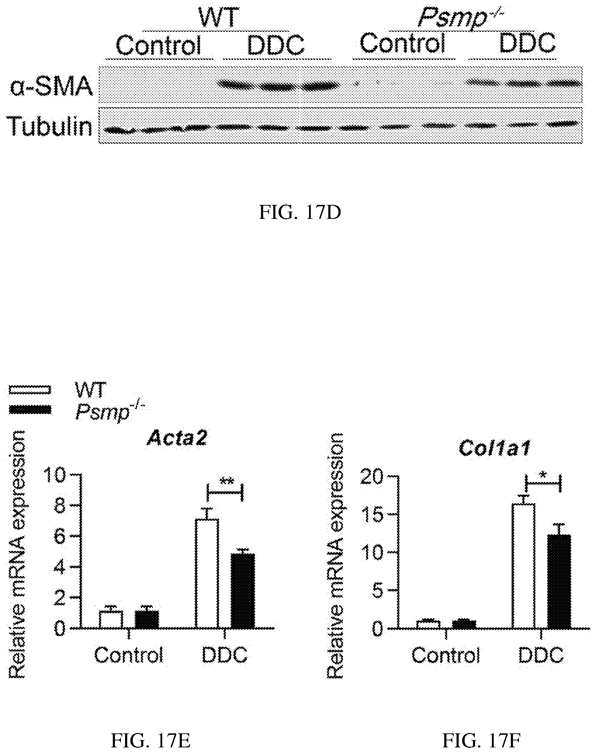

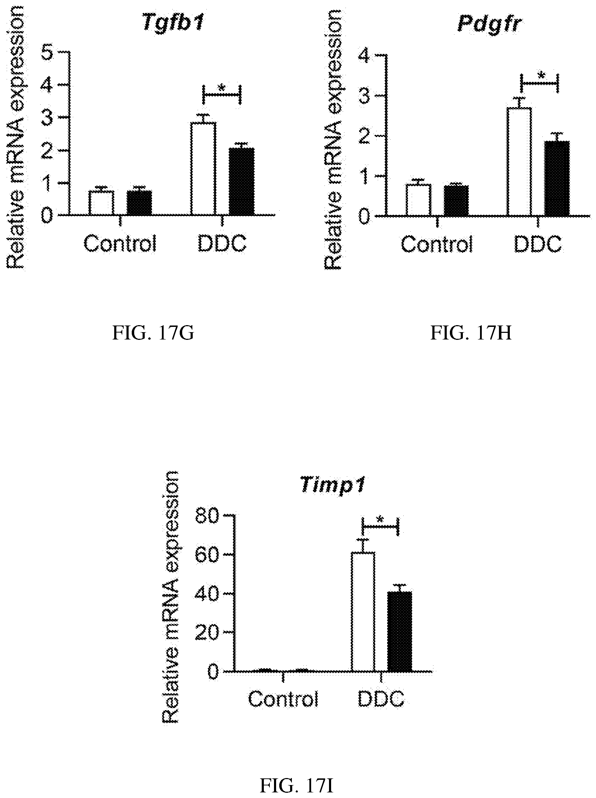

FIGs. 17A-17I illustrate protective effects of PSMP deficiency against liver fibrosis in DDC mice. *See* Example 12. WT and *Psmp*^{*-*/*-*} mice underwent DDC feeding for 4 weeks. Liver histology was performed by H&E and Sirius Red staining (FIG. 17A) and its quantification (FIGs. 17B and 17C). Expression of α-SMA was determined by immunohistochemistry and western blotting (FIGs. 17A and 17D). Hepatic mRNA levels of fibrogenic genes were measured by qRT-PCR (FIGs. 17E-17I).

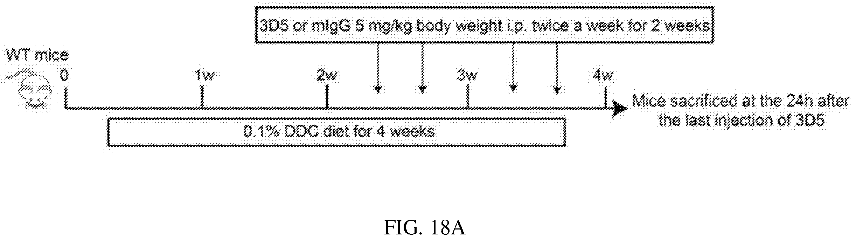

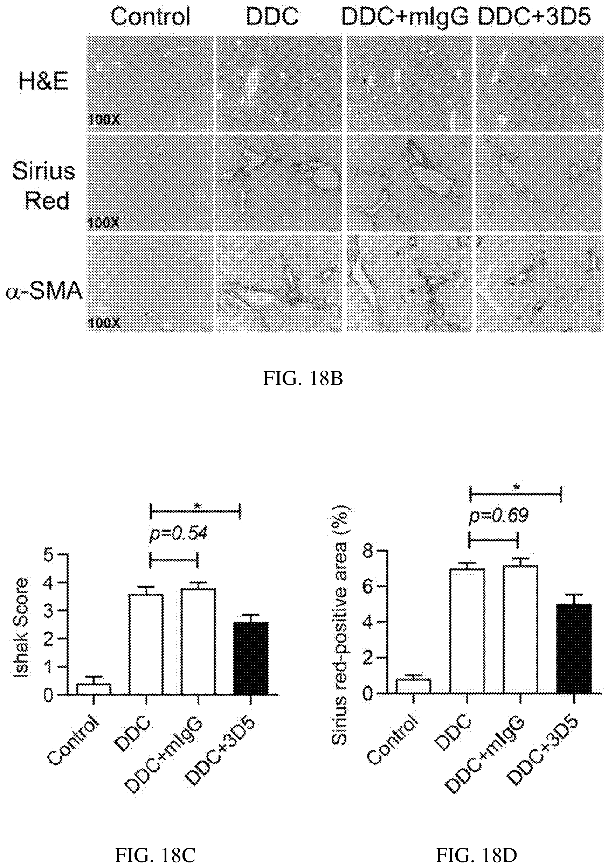

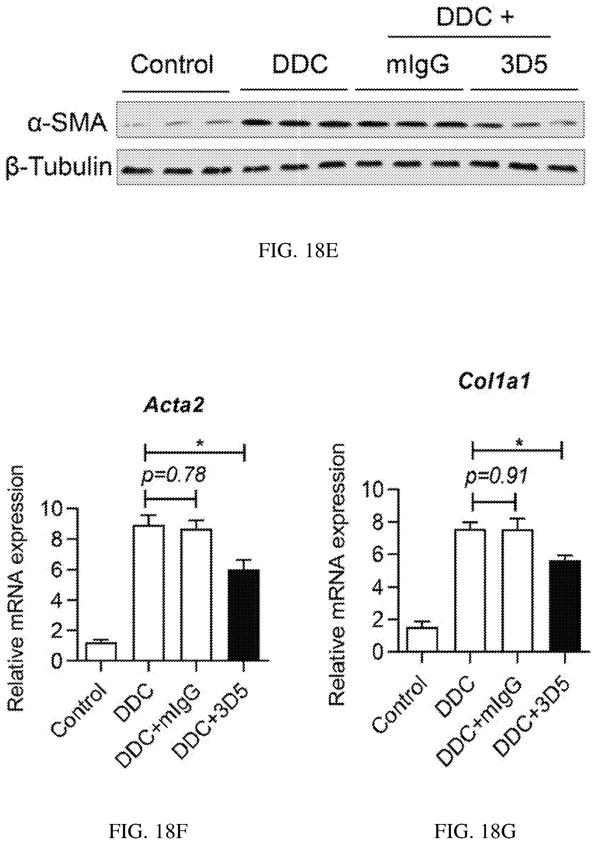

FIGs. 18A-18G illustrate effects of a neutralizing anti-PSMP antibody on murine DDC-induced liver fibrosis. *See* Example 13. FIG. 18A shows a schematic of the experimental design of the PSMP neutralizing antibody 3D5 or mIgG treatment in mice. Liver histology was performed by H&E and Sirius Red staining (FIG. 18B) and its quantification (FIGs. 18C and 18D). Expression of α-SMA was determined by immunohistochemistry and western blotting (FIGs. 18B and 18E). Hepatic mRNA levels of fibrogenic genes were measured by qRT-PCR (FIGs. 18F and 18G).

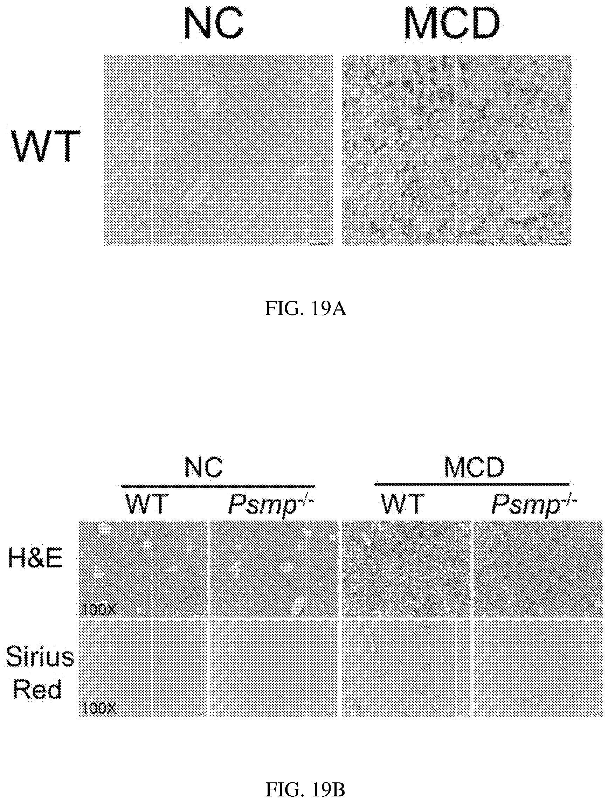

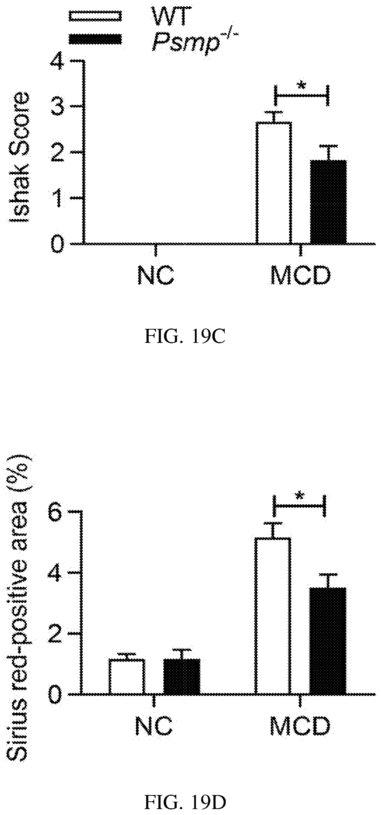

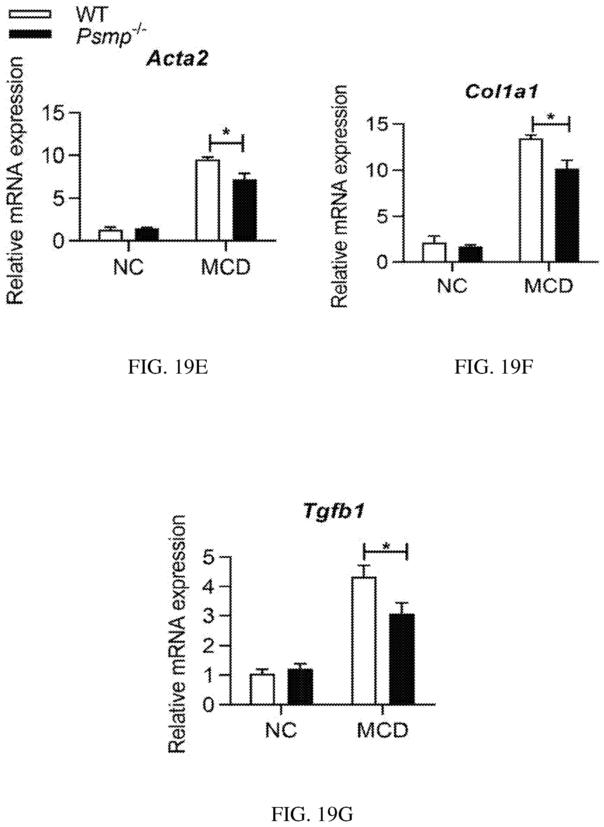

FIGs. 19A-19G illustrate protective effects of PSMP deficiency against liver fibrosis in MCD mice. *See* Example 14. WT and *Psmp*^{*-*/*-*} mice underwent MCD feeding for 6 weeks. FIG. 19A shows representative liver immunohistochemical staining of PSMP in WT mice. Liver histology was performed by H&E and Sirius Red staining (FIG. 19B) and its quantification (FIGs. 19C and 19D). Hepatic mRNA levels of fibrogenic genes were measured by qRT-PCR (FIGs. 19E-19G).

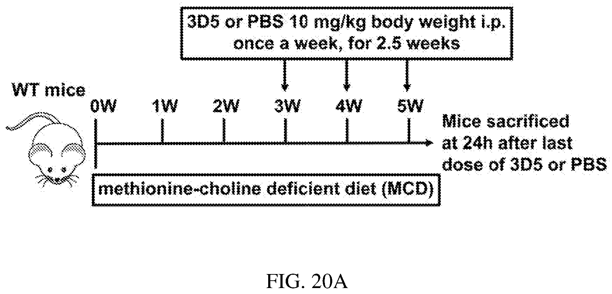

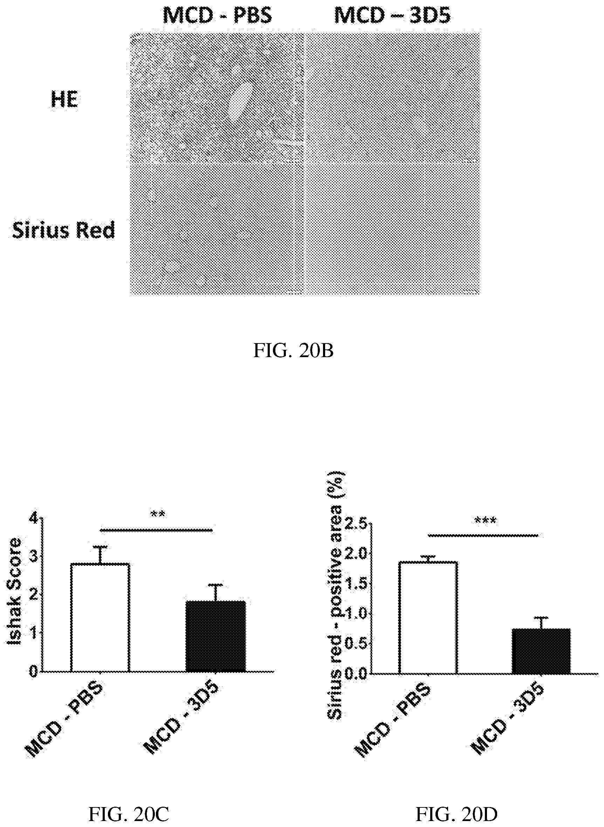

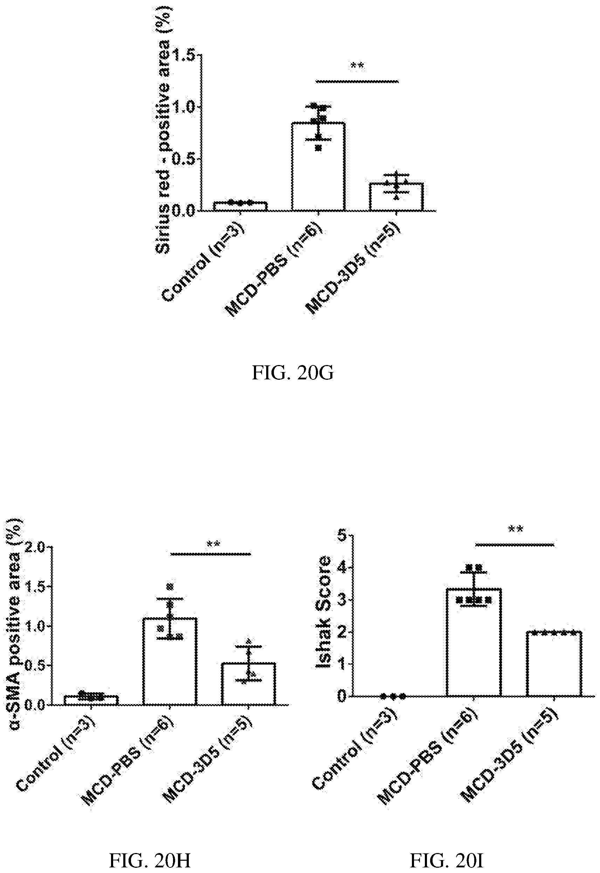

FIGs. 20A-20D illustrate effects of a neutralizing anti-PSMP antibody on murine MCD diet-induced liver fibrosis (treatment from week 4 to week 5.5). *See* Example 15. FIG. 20A shows a schematic of the experimental design of the PSMP neutralizing antibody 3D5 or PBS treatment in mice. Liver histology was performed by H&E and Sirius Red staining (FIG. 20B) and its quantification (FIGs. 20C and 20D). (Scale bars, 100µm. **, *P*<0.01; ***, *P*<0.001 by Student's *t* test, n=5/group.)

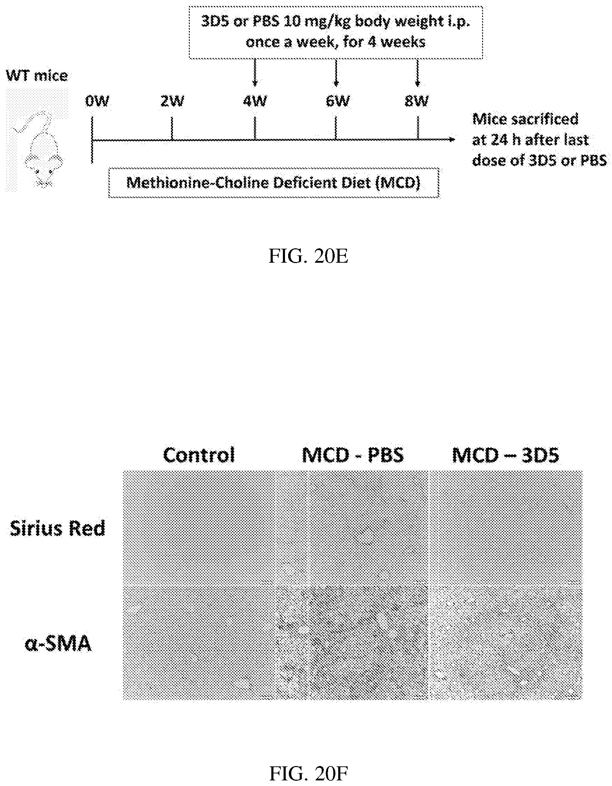

FIGs. 20E-20I illustrate effects of a neutralizing anti-PSMP antibody on murine MCD diet-induced liver fibrosis (treatment from week 5 to week 8). *See* Example 15. FIG. 20E shows a schematic of the experimental design of the PSMP neutralizing antibody 3D5 or PBS treatment in mice. Liver histology was performed by Sirius Red and α-SMA immunohistochemistry staining (FIG. 20F) and its quantification (FIGs. 20G-20I). (Scale bars, 100µm. **, *P*<0.01 by Student's *t* test, control n=3; MCD-PBS n=6; MCD-3D5 n=5.)

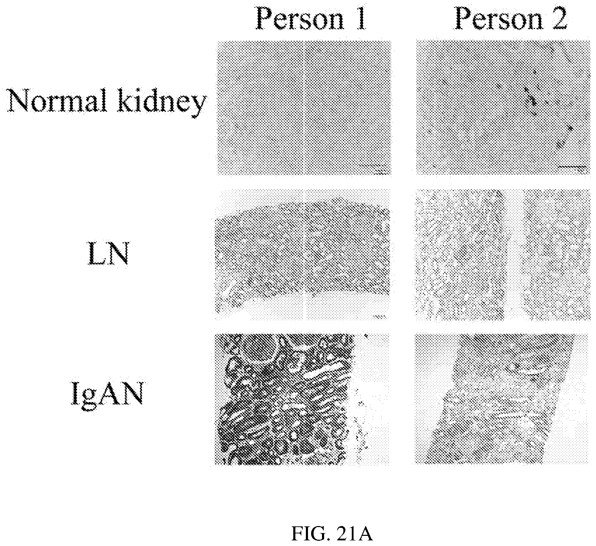

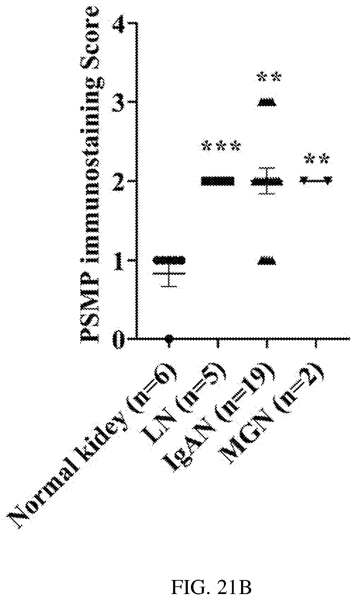

FIGs. 21A and 21B illustrate PSMP expression in different kidney disease tissues. *See* Example 16. FIG. 21A shows representative immunohistochemical staining of PSMP in human normal kidney tissue from adjacent clear cell carcinoma, LN kidney tissue and IgAN kidney tissue. FIG. 21B shows a statistical summary of PSMP immunostaining in different kidney diseases LN: Lupus nephritis, IgAN: IgA nephropathy, MGN: Membranous glomerulonephritis. Scale bars, 100 µm. **, *P* < 0.01; ***, *P* < 0.001 by Student's *t* test.

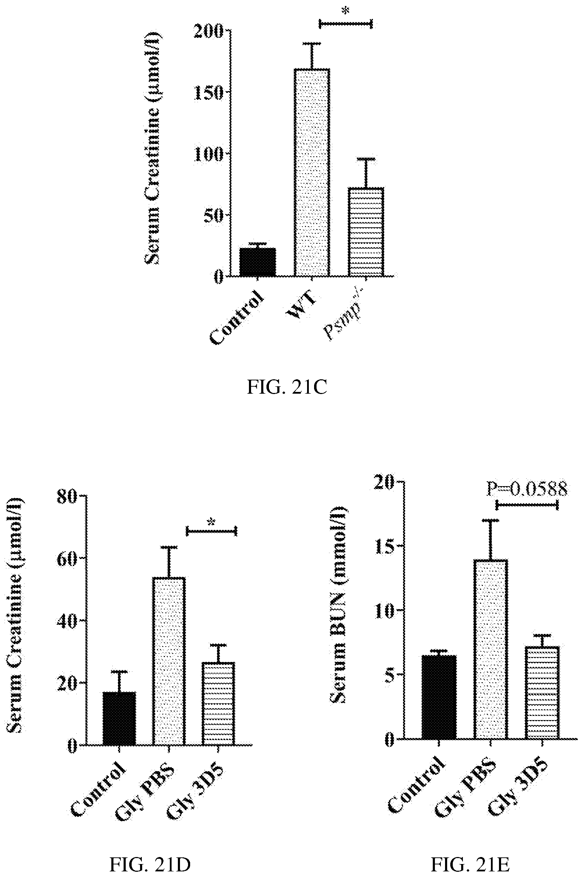

FIGs. 21C-21E illustrate effects of PSMP deficiency or neutralization in a murine model of acute kidney injury (AKI). *See* Example 16. FIG. 21C shows serum creatinine level at 48 h after glycerol injections in *Psmp*^{*-*/*-*} or WT mice. FIGs. 21D and 21E show serum creatinine level (21D) and BUN level (21E) at 48 h after glycerol injections in mice injected PSMP neutralizing antibody 3D5 or PBS. *, *P* < 0.05 by Student's *t* test, *n*=4-6/group.

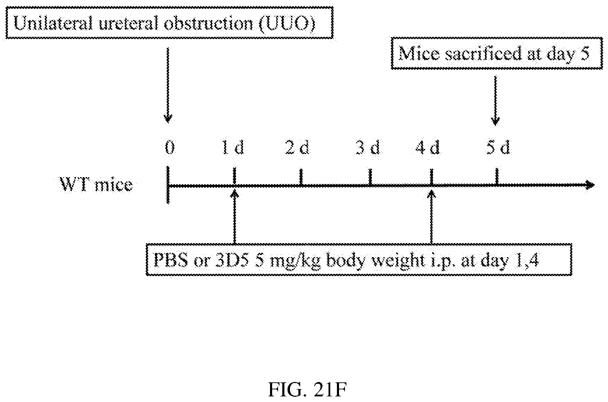

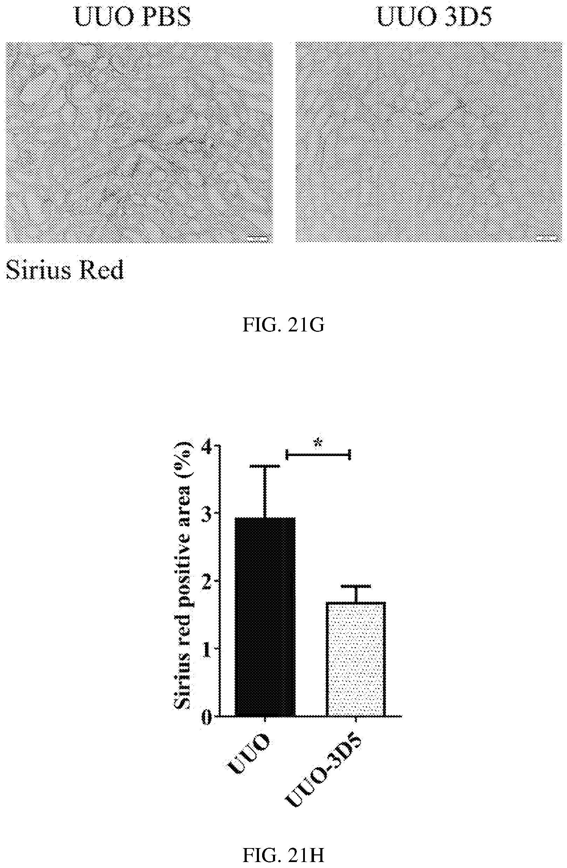

FIGs. 21F-21H illustrate therapeutic effects of a neutralizing anti-PSMP antibody in a murine model of chronic kidney disease (CKD). *See* Example 16. FIG. 21F shows a schematic of the experimental design of the PSMP neutralizing antibody 3D5 or PBS treatment in UUO mice. FIGs. 21G and 21H show representative kidney histology of Sirius Red staining (21G) and its quantification (21H; grey bar - 3D5-treated group, black bar - PBS-treated group). Scale bars, 100 µm. *, *P* < 0.05 by Student's *t* test, *n*=4-6/group.

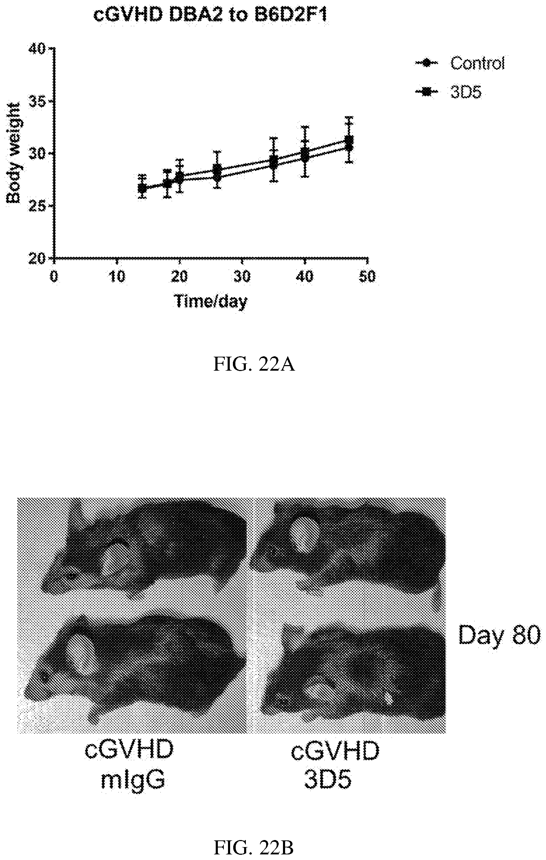

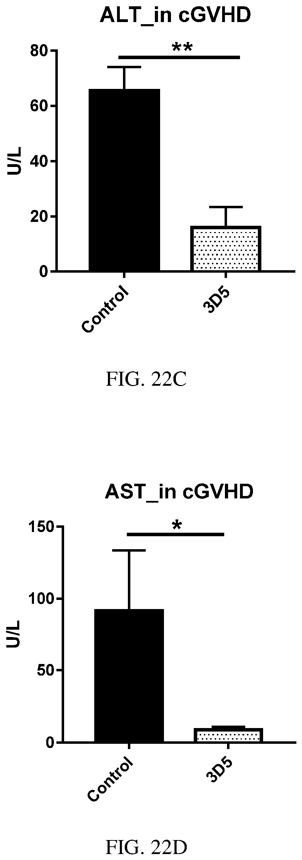

FIGs. 22A-22D illustrate therapeutic effects of a neutralizing anti-PSMP antibody in a chronic GVHD (cGVHD) mouse model. *See* Example 17. FIG. 22A shows body weight monitored over time. FIG. 22B shows fur shedding in the control group compared to mice treated with anti-PSMP antibody 3D5. Serum markers for liver damage, ALT and AST, were measured (FIGs. 22C and 22D). *, *P* < 0.05; **, *P* < 0.01 by Student's t test, *n*≥6/group.

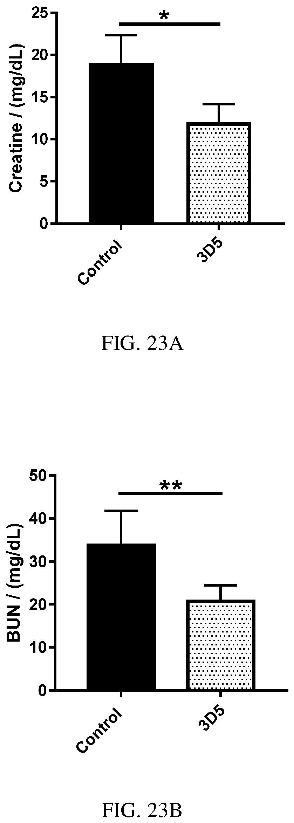

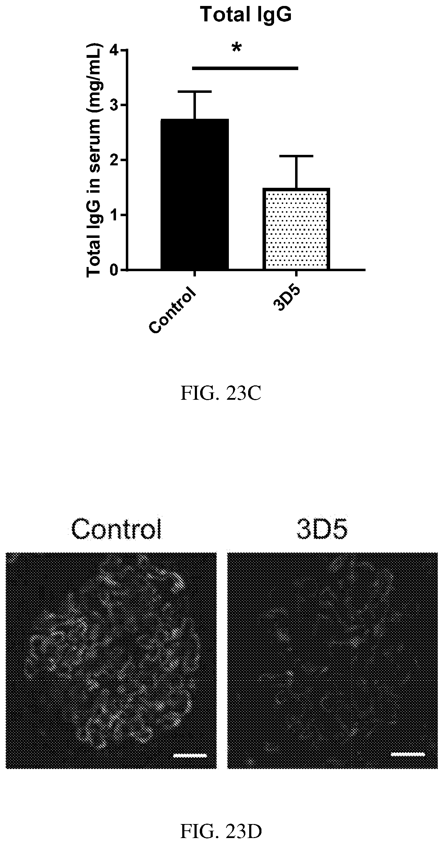

FIGs. 23A-23C illustrate therapeutic effects of a neutralizing anti-PSMP antibody in a lupus nephritis mouse model. *See* Example 18. Renal function markers serum creatinine (FIG. 23A) and blood urea nitrogen (BUN) (FIGs. 23B), the serum total IgG level (FIG. 23C), and autoantibody deposition in the glomerulus (FIG. 23D) were measured. Scale bars, 100 µm. *, *P* < 0.05; **, *P* < 0.01 by Student's *t* test, *n*≥6/group.

### DESCRIPTION OF THE INVENTION

The present disclosure is generally directed to the use of PSMP antagonists, including, *e.g*., neutralizing anti-PSMP antibodies, for the treatment of disease. In particular, in some aspects, the present disclosure is directed to the use of PSMP antagonists for the treatment of liver fibrosis. In other aspects, the present disclosure is directed to the use of PSMP antagonists for the treatment of lung fibrosis. In other aspects, the present disclosure is directed to the use of PSMP antagonists for the treatment of kidney fibrosis. In yet other aspects, the present disclosure is directed to the use of PSMP antagonists for the treatment of a disease or disorder associated with liver, lung, or kidney fibrosis, such as, *e.g.,* a disease or disorder selected from nonalcoholic fatter liver disease (*e.g*., nonalcoholic steatohepatitis (NASH)), alcoholic liver disease (ALD), primary sclerosing cholangitis (PSC), primary biliary cholangitis (PBC), drug-induced lung injury, acute kidney injury (AKI), chronic kidney disease (CKD), lupus nephritis, IgA nephropathy, and membranous glomerulonephritis. In still other aspects, the present disclosure is directed to the use of PSMP antagonists for the treatment of graft-versus-host disease (GVHD) or systemic lupus erythematosus.

Studies described herein indicate that PSMP signaling is involved in the pathogenesis of liver, lung, and kidney fibrosis, and that blocking PSMP *in vivo* can ameliorate fibrosis in disease models. In particular, the present inventors have found that PSMP expression is upregulated in human and murine liver fibrosis *(see* Example 1). Using PSMP knockout mice and liver fibrosis models, the present inventors have also shown that PSMP deficiency significantly attenuates development of liver fibrosis *(see* Example 2), that restoration of hepatic PSMP expression promotes liver fibrosis in a CCR2-dependent manner *(see* Example 4), and that liver fibrosis is significantly ameliorated by neutralizing PSMP signaling using either prophylactic or therapeutic treatment regimens *(see* Example 3). In addition, the present inventors have shown that PSMP is required to establish fibrosis in a model of non-alcoholic steatohepatitis (NASH) *(see* Example 8), that PSMP deficiency protects against liver fibrosis in a MCD diet-induced NASH model *(see* Example 14), and that neutralizing PSMP alleviates liver fibrosis in the MCD diet-induced NASH model *(see* Example 15). The present inventors have also found that neutralizing or deficiency of PSMP alleviates liver steatosis and fibrosis in a chronic ethanol-feeding model and alleviates liver steatosis, liver injury, and inflammation in a chronic-plus-binge alcohol feeding model (also called the NIAAA model) *(see* Example 10). Studies described herein also show that PSMP is upregulated in a DDC-diet-induced liver fibrosis model and in an acute liver injury model *(see* Example 11), that PSMP deficiency protects against liver fibrosis in the DDC model *(see* Example 12), and that neutralizing PSMP alleviates murine DDC-induced liver fibrosis *(see* Example 13). Still further, studies described herein show that PSMP is upregulated in a bleomycin-induced model of lung fibrosis, that PSMP deficiency in this model protects against lung fibrosis, and that neutralization of PSMP signaling alleviates lung fibrosis in this model *(see* Example 7).

The present inventors have also found that PSMP expression is upregulated in human kidney disease, that deficiency and neutralization of PSMP alleviates renal injury in a glycerol-induced rhabdomyolysis model, and that neutralization of PSMP alleviates renal fibrosis in a UUO-induced model of acute kidney injury (AKI) and chronic kidney disease *(see* Example 16).

The present inventors have also found that neutralizing PSMP can effectively treat graft-versus-host disease, as shown by extended survival in an acute GVHD mouse model *(see* Example 9) as well as by the lack of fur shedding and significantly lower levels of markers for liver damage in a chronic GVHD model *(see* Example 17).

The present inventors have also found that neutralizing PSMP can effectively treat lupus nephritis, a common manifestation of systemic lupus erythematosus (SLE), as shown by improved renal function, reduced serum IgG level, and reduced autoantibody deposition in the glomerulus in a mouse lupus model *(see* Example 18).

PSMP antagonists for use within the present disclosure include, for example, molecules that bind to PSMP and reduce that activity of PSMP on cells that express a PSMP receptor such as, *e.g.,* CCR2. Particularly suitable PSMP antagonists include neutralizing anti-PSMP antibodies. Suitable PSMP antagonists also include non-antibody soluble proteins capable of inhibiting the interaction of PSMP with its receptor, including, *e.g*., alternative scaffold proteins and peptide aptamers that specifically bind and neutralize PSMP. In some embodiments, a PSMP antagonist is a nucleic acid aptamer that specifically binds and neutralizes PSMP. In other variations, small molecule antagonists capable of inhibiting the interaction of PSMP with its receptor, or otherwise capable in inhibiting PSMP-induced intracellular signaling through a PSMP receptor, may be employed. Inhibitory polynucleotides targeting the PSMP gene or an mRNA encoding PSMP may also be used.

Neutralizing activity of candidate PSMP antagonists (*e.g*., antibodies or other soluble binding proteins) may be assessed, for example, in chemotaxis assays using cells expressing a PSMP receptor. For example, a chemotaxis assay may utilize cells expressing either or both isoforms of human CCR2 (CCR2A and/or CCR2B, amino acid sequence shown as SEQ ID NO:2 and SEQ ID NO:3, respectively). Chemotaxis assays utilizing CCR2-expressing cells are known in the art. *See, e.g.,* Pei et al., J. Immunol. 192:1878-86, 2014. In one exemplary assay, a plasmid expressing CCR2B (SEQ ID NO:3) (10 µg) is transiently transfected into HEK293 cells by electroporation (120 V for 20 ms) using an electric pulse generator. 48 hours later, the CCR2B-expressing HEK293 cells are used for chemotaxis assay using a 48-well microchemotaxis chamber. PSMP (SEQ ID NO:1 residues 37-139) (7, 70, or 700 ng/ml) is pretreated with a candidate PSMP antagonist (10 or 50 µg/ml) for 30 min. Cells that migrate to the lower part of the filter are fixed and stained with the Three Step Stain Set. Cells are counted in five randomly selected high-power fields (x400) per well. PSMP chemotactic ability toward CCR2B-expressing HEK293 cells in the presence of candidate antagonist is assessed relative to chemotactic ability in the absence of candidate antagonist to determine whether the candidate is effective to neutralize PSMP activity.

In some embodiments, a PSMP antagonist in accordance with the present disclosure is a soluble protein *(e.g.,* an antibody) that specifically binds to PSMP (residues 37-139 of SEQ ID NO: 1). Binding proteins are considered to be specifically binding if (1) they exhibit a threshold level of binding activity, and (2) they do not significantly cross-react with control polypeptide molecules. For example, a threshold level of binding is determined if a protein binds to a PSMP polypeptide, peptide or epitope with an affinity at least 10-fold greater than the binding affinity to a control (non-PSMP) polypeptide. It is preferred that binding proteins (*e.g*., antibodies) used within the disclosure exhibit a binding affinity (Kₐ) of 10⁶ M⁻¹ or greater, preferably 10⁷ M⁻¹ or greater, more preferably 10⁸ M⁻¹ or greater, and most preferably 10⁹ M⁻¹ or greater.

Soluble binding proteins (*e.g*., antibodies) may be assessed for binding activity using any of various known assays. For example, one assay system employs a commercially available biosensor instrument (BIAcore^{™}, Pharmacia Biosensor, Piscataway, NJ), wherein a binding protein *(e.g.,* an anti-PSMP antibody) is immobilized onto the surface of a sensor chip, and a test sample containing a soluble target molecule *(e.g.,* PSMP) is passed through the cell. If the immobilized protein has affinity for the target molecule, it will bind to the target, causing a change in the refractive index of the medium, which is detected as a change in surface plasmon resonance of the gold film. This system allows the determination of on- and off-rates, from which binding affinity can be calculated, and assessment of stoichiometry of binding. Use of this instrument is disclosed, *e.g.,* by Karlsson (J. Immunol. Methods 145:229-240, 1991) and Cunningham and Wells (J. Mol. Biol. 234:554-563, 1993). Binding activity of candidate protein molecules can also be used within other assay systems known in the art. Such systems include Scatchard analysis for determination of binding affinity *(see* Scatchard, Ann. NY Acad. Sci. 51: 660-672, 1949) and calorimetric assays *(see* Cunningham et al., Science 253:545-548, 1991; Cunningham et al., Science 254:821-825, 1991).

In certain embodiments, a PSMP antagonist is a soluble protein (*e.g*., antibody) that competes for binding to PSMP (residues 37-139 of SEQ ID NO:1) with an antibody having the same heavy and light chain variable domains (VH and VL) as monoclonal antibody 3D5. The amino acid sequences of the mAb 3D5 VH and VL domains are shown herein as SEQ ID NO:4 and SEQ ID NO:5, respectively. MAb 3D5 has been shown to effectively neutralize PSMP activity. *See* Pei et al., J. Immunol. 192:1878-86, 2014; *see also* Example 3, *infra.* The ability of a PSMP-binding protein to compete for binding to PSMP with an antibody having the VH and VL domains of mAb 3D5 may be determined by an assay in which a test PSMP-binding protein *(e.g.,* an antibody), or a functional binding fragment thereof, prevents or inhibits specific binding to PSMP of a reference antibody having the VH and VL domains of mAb 3D5 (*i.e.*, having VH and VL domains of SEQ ID NO:4 and SEQ ID NO:5, respectively). Typically, such an assay involves the use of purified target protein *(e.g.,* SEQ ID NO:1 residues 37-139 or a fragment thereof) bound to a solid surface or cells bearing the target protein, an unlabeled test protein (*i.e.,* a PSMP-binding protein or candidate PSMP-binding protein), and a labeled reference antibody. Competitive inhibition is measured by determining the amount of label bound to the solid surface or cells in the presence of the test protein. Usually the test protein is present in excess and/or allowed to bind first. Soluble PSMP-binding proteins identified by competition assay ("competing PSMP-binding proteins") include proteins binding to the same epitope bound by the reference antibody, proteins binding to an epitope overlapping with the epitope bound by the reference antibody, and proteins binding to an epitope sufficiently proximal to the epitope bound by the reference antibody for steric hindrance to occur. Usually, when a competing PSMP-binding protein *(e.g.,* a competing anti-PSMP antibody) is present in excess, it will inhibit specific binding of a reference antibody to PSMP target protein by at least 30%, at least 40%, at least 45%, at least 50%, at least 55%, at least 60%, at least 65%, at least 70%, or at least 75%; in some instances, binding is inhibited by at least 80%, at least 85%, at least 90%, at least 95%, at least 97%, or more. Conversely, when the reference antibody is bound, it will preferably inhibit binding of a subsequently added competing PSMP-binding protein *(e.g.,* a competing anti-PSMP antibody) by at least 30%, at least 40%, at least 45%, at least 50%, at least 55%, at least 60%, at least 65%, at least 70%, or at least 75%; in some instances, binding is inhibited by at least 80%, at least 85%, at least 90%, at least 95%, at least 97%, or more.

The PSMP antagonist for use in accordance with the present invention is an antibody. Antibodies in accordance with the present invention comprise or consist of at least a portion of intact antibodies that retain antigen-binding specificity. Suitable antibodies include, for example, fully human antibodies; humanized antibodies; chimeric antibodies; antibody fragments such as, *e.g.,* Fab, Fab', F(ab)₂, F(ab')₂ and Fv antibody fragments; single-chain antibodies; and monomers or dimers of antibody heavy or light chains or mixtures thereof. Preferred antibodies of the invention are monoclonal antibodies. Antibodies comprising a light chain may comprise kappa or lambda light chain.

In certain embodiments, antibodies for use in the invention include intact immunoglobulins of any isotype including IgA, IgG, IgE, IgD, or IgM (including subtypes thereof). Intact immunoglobulins in accordance with the present invention preferably include intact IgG *(e.g.,* intact IgG1, IgG2, IgG3, or IgG4).

Methods for preparing and isolating polyclonal antibodies, monoclonal antibodies, and antigen-binding antibody fragments thereof are well-known in the art. *See, e.g.,* Current Protocols in Immunology, (Cooligan et al. eds., John Wiley and Sons, Inc. 2006); Sambrook et al., Molecular Cloning: A Laboratory Manual (Cold Spring Harbor, NY, 2nd ed. 1989); and Monoclonal Hybridoma Antibodies: Techniques and Applications (Hurrell ed., CRC Press, Inc., Boca Raton, FL, 1982). Antigen-binding fragments, including scFv, can be prepared using, *e.g*., phage display libraries according to methods known in the art. Methods for preparing recombinant human polyclonal antibodies are disclosed by Wiberg et al., Biotechnol Bioeng. 94:396-405, 2006; Meijer et al., J. Mol. Biol. 358:764-772, 2006; Haurum et al., U.S. Patent Application Publication No. 2002/0009453; and Haurum et al., U.S. Patent Application Publication No. 2005/0180967. As will be evident to persons of ordinary skill in the art, these methods are equally applicable to production of antibodies against PSMP.

As would be evident to one of ordinary skill in the art, polyclonal antibodies for use within the present disclosure can be generated by inoculating any of a variety of warm-blooded animals such as horses, cows, goats, sheep, dogs, chickens, rabbits, mice, and rats with an immunogenic polypeptide or polypeptide fragment. The immunogenicity of an immunogenic polypeptide can be increased through the use of an adjuvant, such as alum (aluminum hydroxide) or Freund's complete or incomplete adjuvant. Polypeptides useful for immunization also include fusion polypeptides, such as fusions of PSMP with an immunoglobulin polypeptide or with maltose binding protein. The polypeptide immunogen may be a full-length molecule or a portion thereof. If the polypeptide portion is hapten-like, it may be advantageously joined or linked to a macromolecular carrier (such as keyhole limpet hemocyanin (KLH), bovine serum albumin (BSA) or tetanus toxoid) for immunization.

In addition, antibodies can be screened against known polypeptides related to the antibody target (*e.g*., orthologs, paralogs, or sequence variants of PSMP) to isolate a population of antibodies that is highly specific for binding to the target protein. Such a lack of crossreactivity with related polypeptide molecules is shown, for example, by the antibody detecting a PSMP polypeptide but not known, related polypeptides using a standard Western blot analysis (Current Protocols in Molecular Biology (Ausubel et al. eds., Green and Wiley and Sons, NY 1993)) or ELISA (enzyme immunoassay) (Immunoassay, A Practical Guide (Chan ed., Academic Press, Inc. 1987)). In another example, antibodies raised against a PSMP polypeptide are adsorbed to related polypeptides adhered to insoluble matrix; antibodies that are highly specific to the PSMP polypeptide will flow through the matrix under the proper buffer conditions. Screening allows isolation of polyclonal and monoclonal antibodies noncross-reactive to known, closely related polypeptides (Antibodies: A Laboratory Manual (Harlow and Lane eds., Cold Spring Harbor Laboratory Press 1988); Current Protocols in Immunology (Cooligan et al. eds., National Institutes of Health, John Wiley and Sons, Inc. 1995). Screening and isolation of specific antibodies is well known in the art. *See* Fundamental Immunology (Paul ed., Raven Press 1993); Getzoff et al., Adv. in Immunol. 43: 1-98, 1988; Monoclonal Antibodies: Principles and Practice (Goding ed., Academic Press Ltd. 1996); Benjamin et al., Ann. Rev. Immunol. 2:67-101, 1984.

Native monoclonal antibodies ("mAbs") can be prepared, for example, by immunizing subject animals (*e.g*., rats or mice) with a purified immunogenic protein or fragment thereof. In a typical procedure, animals are each given an initial intraperitoneal (IP) injection of the purified protein or fragment, typically in combination with an adjuvant (*e.g*., Complete Freund's Adjuvant or RIBI Adjuvant (available from Sigma-Aldrich, St. Louis, MO)) followed by booster IP injections of the purified protein at, for example, two-week intervals. Seven to ten days after the administration of the third booster injection, the animals are bled and the serum is collected. Additional boosts can be given as necessary. Splenocytes and lymphatic node cells are harvested from high-titer animals and fused to myeloma cells (*e.g*., mouse SP2/0 or Ag8 cells) using conventional methods. The fusion mixture is then cultured on a feeder layer of thymocytes or cultured with appropriate medium supplements (including commercially available supplements such as Hybridoma Fusion and Cloning Supplement; Roche Diagnostics, Indianapolis, IN). About 10 days post-fusion, specific antibody-producing hybridoma pools are identified using standard assays (*e.g*., ELISA). Positive pools may be analyzed further for their ability to block or reduce the activity of the target protein. Positive pools are cloned by limiting dilution.

The amino acid sequence of a native antibody can be varied through the application of recombinant DNA techniques. Thus, antibodies can be redesigned to obtain desired characteristics. Modified antibodies can provide, for example, improved stability and/or therapeutic efficacy relative to its non-modified form. The possible variations are many and range from the changing of just one or a few amino acids to the complete redesign of, for example, the variable or constant region. Changes in the constant region will, in general, be made in order to improve or alter characteristics, such as complement fixation, interaction with membranes, and other effector functions. Various changes in a constant region to alter characteristics (e.g., effector function) are known. *See, e.g.,* Morgan et al., Immunology 86:319-324, 1995; Lund et al., J. Immunol. 157:4963-9 157:4963-4969, 1996; Idusogie et al., J. Immunol. 164:4178-4184, 2000; Tao et al., J. Immunol. 143: 2595-2601, 1989; Jefferis et al., Immunological Reviews 163:59-76, 1998; Armour et al., Eur. J. Immunol., 29:2613-2624, 1999; PCT Publication No. WO1999/058572; UK Patent Application No. 9809951.8. Typically, changes in the variable region will be made in order to improve the antigen binding characteristics, improve variable region stability, or reduce the risk of immunogenicity. Phage display techniques can also be employed. *See, e.g.,* Huse et al., Science 246:1275-1281, 1989; Ladner et al., U.S. Patent No. 5,571,698.

For therapeutic antibodies for use in humans, it is usually desirable to humanize non-human regions of an antibody according to known procedures. Methods of making humanized antibodies are disclosed, for example, in U.S. Patent Nos. 5,530,101; 5,821,337; 5,585,089; 5,693,762; and 6,180,370. Methods of making humanized antibodies are also disclosed in, *e.g.,* U.S. Patent No. 7,732,578. Typically, a humanized anti-PSMP antibody comprises the complementarity determining regions (CDRs) of a murine donor immunoglobulin and heavy chain and light chain frameworks of a human acceptor immunoglobulin. Often, framework residues in the human framework regions will be substituted with the corresponding residue from the donor antibody to alter, preferably improve, antigen binding. These framework substitutions are identified by methods well known in the art, *e.g.,* by modeling of the interactions of the CDR and framework residues to identify framework residues important for antigen binding and sequence comparison to identify unusual framework residues at particular positions. *See, e.g.,* Queen et al., U.S. Patent No. 5,585,089; Riechmann et al., Nature 332:323, 1988.

Non-humanized chimeric antibodies can also be used therapeutically (*e.g*., in immunosuppressed patients). Accordingly, in some variations, an antibody for use in accordance with the present invention is a chimeric antibody derived, *inter alia,* from a nonhuman anti-PSMP antibody. Preferably, a chimeric antibody comprises a variable region derived from a mouse or rat antibody and a constant region derived from a human so that the chimeric antibody has a longer half-life and is less immunogenic when administered to a human subject. Methods for producing chimeric antibodies are known in the art. *See e.g.,* Morrison, Science 229:1202, 1985; Oi et al., BioTechniques 4:214, 1986; Gillies et al., J. Immunol. Methods 125:191-202, 1989; U.S. Patent Nos. 5,807,715; 4,816,567; and 4,816,397.

The present invention also encompasses use of fully human antibodies such as, for example, those selected from human antibody expression libraries (*e.g*., phage display libraries); those made in non-human animals (*e.g*., mice) transgenic for a human heavy chain locus and a human light chain locus with the corresponding endogenous immunoglobulin loci inactivated; or those prepared by immortalizing human B lymphocytes producing an antibody against a PSMP target antigen.

Antibodies for use in accordance with the present invention have binding affinities that include a dissociation constant (K_{d}) that is, *e.g.,* less than 5 x 10⁻⁴ M, less than 10⁻⁴ M, less than 5 x 10⁻⁵ M, less than 10⁻⁵ M, less than 5 x 10⁻⁶ M, less than 10⁻⁶ M, less than 5 x 10⁻⁷ M, less than 10⁻⁷ M, less than 5 x 10⁻⁸ M, less than 10⁻⁸ M, less than 5 x 10⁻⁹ M, less than 10⁻⁹ M, less than 5 x 10⁻¹⁰ M, less than 10⁻¹⁰ M, less than 5 x 10⁻¹¹ M, less than 10⁻¹¹ M, less than 5 x 10⁻¹² M, less than 10⁻¹² M, less than 5 x 10⁻¹³ M, less than 10⁻¹³ M, less than 5 x 10⁻¹⁴ M, less than 10⁻¹⁴ M, less than 5 x 10⁻¹⁵ M, or less than 10⁻¹⁵ M.

Antibodies for use in the present invention further include derivatives that are modified, *e.g.,* by the covalent attachment of any type of molecule to the antibody such that covalent attachment does not prevent the antibody from binding to its epitope. Suitable modifications include, for example, fucosylation, glycosylation, acetylation, pegylation, phosphorylation, and amidation. The antibodies and derivatives thereof may themselves by derivatized by known protecting/blocking groups, proteolytic cleavage, linkage to a cellular ligand or other proteins, and the like. In some embodiments of the invention, at least one heavy chain of the antibody is fucosylated. In particular variations, the fucosylation is N-linked. In certain embodiments, at least one heavy chain of the antibody comprises a fucosylated, N-linked oligosaccharide.

Antibodies for use in accordance with the present invention include variants having one or more amino acid substitutions, deletions, or additions relative to a reference antibody such that the variant retains one or more biological properties of the reference antibody. In certain embodiments, an antibody is a variant having one or more amino acid substitutions, deletions, or additions relative to a reference anti-PSMP neutralizing antibody having VH and/or VL sequences as shown in SEQ ID NO:4 and SEQ ID NO:5, respectively, such that the antibody retains the ability of the reference antibody to specifically bind PSMP and neutralize PSMP activity. A skilled person can readily produce variants having one or more amino acid substitutions, deletions, or additions relative to a reference antibody. The techniques for obtaining these variants, including genetic (suppressions, deletions, mutations, *etc*.), chemical, and enzymatic techniques, are known to the person having ordinary skill in the art.

In some embodiments, a neutralizing anti-PSMP antibody for use in accordance with the present invention comprises one or more CDRs of anti-PSMP mAb 3D5. Thus, in certain variations, an antibody comprises a heavy chain CDR (at least one of the CDR-H1, CDR-H2, and CDR-H3 regions) of the 3D5 VH domain (SEQ ID NO:4) and/or a light chain CDR (at least one of the CDR-L1, CDR-L2, and CDR-L3 regions) of the 3D5 VL domain (SEQ ID NO:5). In typical embodiments, the antibody has two or three CDRs of the 3D5 VH domain (SEQ ID NO:4) and/or two or three CDRs of the 3D5 VL domain (SEQ ID NO:5). In some variations, where an antibody has at least one CDR of the 3D5 VH domain, the antibody further comprises at least one CDR of the 3D5 VL domain; in some such embodiments, an antibody has all three heavy chain CDRs and all three light chain CDRs of mAb 3D5 (*i.e.,* CDR-H1, CDR-H2, and CDR-H3 of SEQ ID NO:4 and CDR-L1, CDR-L2, and CDR-L3 of SEQ ID NO:5). The one or more CDRs may be defined, for example, according to the Chothia definition, the Kabat definition, the AbM definition, or the contact definition of CDR. Under the Chothia definition of CDR, CDR-H1, CDR-H2, and CDR-H3 of mAb 3D5 correspond, respectively, to residues 31-35, 50-69, and 99-108 of SEQ ID NO:4, and CDR-L1, CDR-L2, and CDR-L3 of mAb 3D5 correspond, respectively, to residues 24-34, 50-56, and 89-97 of SEQ ID NO:5. In particular variations of an antibody as above, the antibody comprises an VH domain and/or a VL domain having a human immunoglobulin framework region, or a variant thereof having at least 85%, at least 90%, or at least 95% amino acid sequence identity to the human immunoglobulin framework region.

In some embodiments, an anti-PSMP antibody includes (a) a heavy chain variable domain that is at least 60%, at least 65%, at least 70%, at least 75%, at least 80%, at least 85%, at least 90%, at least 95%, at least 96%, at least 97%, at least 98%, at least 99%, or 100% identical to the amino acid sequence of SEQ ID NO:4, and/or (b) a light chain variable domain that is at least 60%, at least 65%, at least 70%, at least 75%, at least 80%, at least 85%, at least 90%, at least 95%, at least 96%, at least 97%, at least 98%, at least 99%, or 100% identical to the amino acid sequence of SEQ ID NO:5.

In some embodiments, an anti-PSMP antibody for use in accordance with the present invention comprises heavy chain CDRs CDR-H1_{Ab}, CDR-H2_{Ab}, and CDR-H3_{Ab}, wherein at least one of the CDRs comprises from zero to three amino acid substitutions relative to a reference set of CDRs CDR-H1_{Ref}, CDR-H2_{Ref}, and CDR-H3_{Ref} in which the reference CDRs are, respectively, a CDR-H1, a CDR-H2, and a CDR-H3 of the 3D5 VH domain (SEQ ID NO:4). In other embodiments, an anti-PSMP antibody for use in accordance with the present invention comprises light chain CDRs CDR-L1_{AB}, CDR-L2_{Ab}, and CDR-L3_{Ab}, wherein at least one of the CDRs comprises from zero to three amino acid substitutions relative to a reference set of CDRs CDR-L1_{Ref}, CDR-L2_{Ref}, and CDR-L3_{Ref} in which the reference CDRs are, respectively, a CDR-L1, a CDR-L2, and a CDR-L3 of the 3D5 VL domain (SEQ ID NO:5). In certain embodiments, an anti-PSMP antibody comprises both sets of heavy chain and light chain CDRs as above. Particularly suitable PSMP antibodies comprise a heavy chain variable domain comprising CDRs CDR-H1_{Ab}, CDR-H2_{Ab}, and CDR-H3_{Ab} and a light chain variable domain comprising CDRs CDR-L1_{AB}, CDR-L2_{Ab}, and CDR-L3_{Ab}, wherein the set of heavy chain CDRs has six or fewer, typically five or fewer, more typically four or fewer, and most typically 3 or fewer amino acid substitutions relative to CDR-H1_{Ref}, CDR-H2_{Ref}, and CDR-H3_{Ref}, and wherein the set of light chain CDRs has six or fewer, typically five or fewer, more typically four or fewer, and most typically 3 or fewer amino acid substitutions relative to CDR-L1_{Ref}, CDR-L2_{Ref}, and CDR-L3_{Ref}. The CDRs as above may be defined, for example, according to the Chothia definition, the Kabat definition, the AbM definition, or the contact definition of CDR. In particular variations of an antibody as above, the VH domain and VL domain each has a human immunoglobulin framework region, or a variant thereof having at least 85%, at least 90%, or at least 95% amino acid sequence identity to the human immunoglobulin framework region.

Anti-PSMP antibodies for use in accordance with the present invention include affinity matured embodiments. Affinity matured antibodies can be produced by procedures known in the art. *See, e.g.,* Marks et al., Bio/Technology 10:779-783, 1992; Barbas et al., Proc Nat. Acad. Sci. USA 91:3809-3813, 1994; Schier et al., Gene 169:147-155, 1995; Yelton et al., J. Immunol. 155:1994-2004, 1995; Jackson et al., J. Immunol. 154:3310-9, 1995; Hawkins et al., J. Mol. Biol. 226:889-896, 1992; and PCT Publication No. WO 2004/058184. In some embodiments, an anti-PSMP antibody is derived by affinity maturation of an antibody comprising one or more CDRs of mAb 3D5 *(e.g.,* derived by affinity maturation of an antibody comprising (i) a VH domain having a CDR-H1, a CDR-H2, and a CDR-H3 of SEQ ID NO:4 and (ii) a VL domain having a CDR-L1, a CDR-L2, and a CDR-L3 of SEQ ID NO:5); in some such variations, the CDRs are defined according to the Chothia definition, the Kabat definition, the AbM definition, or the contact definition of CDR.

One method for adjusting the affinity of an antibody is termed "library scanning mutagenesis." Generally, library scanning mutagenesis is carried out as follows. One or more amino acid positions in at least one CDR *(e.g.,* in two, three, four, five, or six CDRs) are replaced with two or more (such as 3, 4, 5, 6, 7, 8, 9, 10, 11, 12, 13, 14, 15, 16, 17, 18, 19, or 20) amino acids using art recognized methods. This generates small libraries of clones (in some embodiments, one for every amino acid position that is analyzed), each with a complexity of two or more members (if two or more amino acids are substituted at every position). Generally, the library also includes a clone comprising the native (unsubstituted) amino acid. A small number of clones, *e.g*., about 20-80 clones (depending on the complexity of the library), from each library are screened for binding affinity to the target polypeptide (or other binding target), and candidates with increased, the same, decreased, or no binding are identified. Binding affinity may be determined, *e.g*., using Biacore^{™} surface plasmon resonance analysis, which detects differences in binding affinity of about 2-fold or greater. Biacore^{™} is particularly useful when the starting antibody already binds with a relatively high affinity, for example a K_{D} of about 10 nM or lower.

In some embodiments of the invention, an antibody comprises an immunoglobulin constant region *(e.g.,* an immunoglobulin heavy chain constant region such as, *e.g.,* an Fc region). In some such embodiments, the constant region is a variant of a naturally occurring (*e.g*., wild-type) constant region, such as, for example, a constant region that has increased or decreased affinity to a human Fc gamma receptor, or that has reduced activities (compared to the unmodified antibody) in any one or more of the following: triggering complement mediated lysis, stimulating antibody-dependent cell mediated cytotoxicity (ADCC), or activating microglia. Different modifications of the constant region may be used to achieve optimal level and/or combination of effector functions. *See, e.g.,* Morgan et al., Immunology 86:319-324, 1995; Lund et al., J. Immunol. 157:4963-9 157:4963-4969, 1996; Idusogie et al., J. Immunol. 164:4178-4184, 2000; Tao et al., J. Immunol. 143: 2595-2601, 1989; and Jefferis et al., Immunological Reviews 163:59-76, 1998. In some embodiments, the constant region is modified as described in Armour et al., Eur. J. Immunol., 29:2613-2624, 1999; PCT Publication No. WO1999/058572, and/or UK Patent Application No. 9809951.8. In other embodiments, the constant region is aglycosylated for N-linked glycosylation. In some embodiments, the constant region is aglycosylated for N-linked glycosylation by mutating the glycosylated amino acid residue or flanking residues that are part of the N-glycosylation recognition sequence in the constant region. For example, N-glycosylation site N297 may be mutated to A, Q, K, or H. *See* Tao et al., J. Immunol. 143: 2595-2601, 1989; Jefferis et al., Immunological Reviews 163:59-76, 1998. The constant region may also be aglycosylated for N-linked glycosylation enzymatically (such as, *e.g*., removing carbohydrate by enzyme PNGase), or by expression in a glycosylation deficient host cell. Other suitable variant constant regions include sequences based on chimeric domains derived from two or more human immunoglobulin heavy chain CH2 domains that retain FcRn and FcyRIIb binding activity and which do not trigger significant complement dependent lysis or cell-mediated destruction of the target. *See* described in PCT Publication No. WO 99/58572.

In some embodiments, a PSMP antagonist for use in accordance with the present disclosure is a non-antibody protein based on an alternative scaffold. *See, e.g.,* Silverman et al., Nat. Biotechnol. 23:1556-61, 2005; Zahnd et al., J. Mol. Biol. 369:1015-28, 2007; U.S. Patent No. 7,115,396; Binz et al., Nat. Biotechnol. 23:1257-1268, 2005; Skerra, Current Opin. in Biotech. 18:295-304, 2007; Silacci et al., J. Biol. Chem. 289:14392-14398, 2014. In some embodiments, an alternative scaffold antagonist specifically binds and neutralizes PSMP. In particular variations, an alternative scaffold PSMP antagonist is an Adnectin^{™}, an iMab, a lipocalin, a Kunitz domain, an Affibody^{®}, an ankyrin repeat, an Affilin, a Tetranectin, a Fynomer, or an Avimer protein. PSMP-binding proteins based on an alternative scaffold may be prepared, *e.g.,* by screening expression libraries *(e.g.,* phage display, ribosome display) comprising one or more randomized or semi-randomized regions within an alternative scaffold framework. *See, e.g.,* Koide et al., J. Mol. Biol. 284:1141-1151, 1998; Zahnd et al., *supra.*

In other embodiments, a PSMP antagonist for use in accordance with the present disclosure is a peptide aptamer *(e.g.,* a peptide aptamer that specifically binds and neutralizes PSMP). Peptide aptamers generally consist of a variable peptide loop *(e.g.,* about five to 20 amino acids), typically embedded as a loop within a small and stable protein scaffold. Peptide aptamers may be prepared by selecting the aptamer for its binding affinity with the specific target from a random pool or library of peptides. For example, peptide aptamers can be isolated from random peptide libraries by yeast two-hybrid screens *(see, e.g.,* Xu et al., Proc. Natl. Acad. Sci. USA 94:12473, 1997) or phage display libraries. Peptide aptamers are reviewed, for example, in Reverdatto et al., Curr. Top. Med. Chem. 15:1082-1101, 2015.

In yet other embodiments, a PSMP antagonist is a nucleic acid aptamer *(e.g.,* a nucleic acid aptamer that specifically binds and neutralizes PSMP). Generally, a nucleic acid aptamer is an oligonucleotide, such as ribonucleic acid (RNA) or single-strand deoxyribonucleic acid (ssDNA), that can bind to a target with high affinity and specificity due to its specific three-dimensional structure. Nucleic acid aptamers that bind specifically to a target macromolecule can be readily isolated from libraries of such oligomers by technologies such as SELEX. *See, e.g.,* Stoltenburg et al., Biamal. Eng. 24:381, 2007. Nucleic acid aptamers are reviewed, for example, in Ni et al., Curr. Med. Chem. 18:4206-4214, 2011; and Esposito et al., Discovery Medicine 11:487-496, 2011.

In accordance with certain aspects of the present disclosure, a PSMP antagonist is used to treat liver fibrosis. In some embodiments, the liver fibrosis to be treated has progressed to liver cirrhosis. In other non-mutually exclusive embodiments, the fibrosis is associated with a disease or disorder characterized by or known to lead to fibrosis of the liver. For example, the liver fibrosis to be treated may be associated with chronic alcohol abuse (*e.g*., alcoholic liver disease (ALD), alcoholic hepatitis, alcoholic cirrhosis), chronic viral hepatitis (*e.g*., hepatitis B, C, or D), fat accumulation in the liver (nonalcoholic fatty liver disease (*e.g*., nonalcoholic steatohepatitis (NASH)), iron buildup in the body (hemochromatosis), cystic fibrosis, copper accumulation in the liver (Wilson's disease), poorly formed bile ducts (biliary atresia), alpha-1 antitrypsin deficiency, an inherited disorder of sugar metabolism (*e.g*., galactosemia, glycogen storage disease), a genetic digestive disorder (*e.g*., Alagille syndrome), an autoimmune liver disease (*e.g*., autoimmune hepatitis, primary sclerosing cholangitis (PSC), primary biliary cholangitis (PBC, previously known as primary biliary cirrhosis), an infection *(e.g.,* syphilis, brucellosis), drug-induced liver injury *(e.g.,* methotrexate- or isoniazid-induced livery injury), or Budd-Chiari syndrome.

In accordance with other aspects of the present disclosure, a PSMP antagonist is used to treat lung (pulmonary) fibrosis. In some embodiments, the fibrosis is associated with a disease or disorder characterized by or known to lead to fibrosis of the lung. For example, the lung fibrosis to be treated may be associated with dermatomyositis, polymyositis, mixed connective tissue disease, systemic lupus erythematosus, rheumatoid arthritis, sarcoidosis, scleroderma, pneumonia, chronic radiation pneumonitis, a pneumoconiosis, an infection, or drug-induced lung injury. In some embodiments in which the lung fibrosis is associated with a pneumoconiosis, the pneumoconiosis is caused or exacerbated by exposure to silica dust, asbestos fibers, a hard metal dust, coal dust, grain dust, or bird or animal droppings. In some embodiments in which the lung fibrosis is associated with drug-induced lung injury, the lung injury is induced by a chemotherapy drug (*e.g*., methotrexate, cyclophosphamide), a heart medication (*e.g*., a drug used to treat irregular heartbeats such as, for example, amiodarone), an antibiotic *(e.g.,* nitrofurantoin, ethambutol), or an anti-inflammatory drug *(e.g.,* rituximab, sulfasalazine).

In yet other aspects of the present disclosure, a PSMP antagonist is used to treat nonalcoholic fatty liver disease (NAFLD). In some such variations, the NAFLD is nonalcoholic steatohepatitis (NASH).

In yet other aspects of the present disclosure, a PSMP antagonist is used to treat alcoholic liver disease (ALD).

In yet other aspects of the present disclosure, a PSMP antagonist is used to treat primary sclerosing cholangitis (PSC).

In yet other aspects of the present disclosure, a PSMP antagonist is used to treat primary biliary cholangitis (PBC).

In still other aspects of the present disclosure, a PSMP antagonist is used to treat kidney fibrosis. In some embodiments, the fibrosis is associated with a disease or disorder characterized by or known to lead to fibrosis of the kidney. For example, the kidney fibrosis to be treated may be associated with IgA nephropathy, membranoproliferative glomerulonephritis, membranous glomerulonephritis, crescentic glomerulonephritis, diabetic nephropathy, hypertension nephropathy, lupus nephritis, hepatic nephropathy, polycystic kidney disease, Alport syndrome, Fabry disease, primary hyperoxaluria, cystinosis; coenzyme Q10-related gene mutations causing focal segmental glomerulosclerosis (FSGS), complement 3 glomerulonephritis, acute or subacute immune complex glomerulonephritis, renal vasculitis, systemic lupus erythematosus (SLE), recent-onset renal artery stenosis (fibromuscular or vasculitic), diabetic kidney disease, chronic urate nephropathy, toxic nephropathies, bacterial pyelonephritis, viral nephropathies, multiple myeloma, or obstructive nephropathy.

In still other aspects of the present disclosure, a PSMP antagonist is used to treat acute kidney injury (AKI) or chronic kidney disease (CKD). In some embodiments in which a PSMP antagonist is used to treat CKD, the CKD is caused by IgA nephropathy, membranoproliferative glomerulonephritis, membranous glomerulonephritis, crescentic glomerulonephritis, diabetic nephropathy, hypertension nephropathy, lupus nephritis, hepatic nephropathy, polycystic kidney disease, Alport syndrome, Fabry disease, primary hyperoxaluria, cystinosis; coenzyme Q10-related gene mutations causing focal segmental glomerulosclerosis (FSGS), complement 3 glomerulonephritis, acute or subacute immune complex glomerulonephritis, renal vasculitis, systemic lupus erythematosus (SLE), recent-onset renal artery stenosis (fibromuscular or vasculitic), diabetic kidney disease, chronic urate nephropathy, toxic nephropathies, bacterial pyelonephritis, viral nephropathies, multiple myeloma, or obstructive nephropathy.

In still other aspects of the present disclosure, a PSMP antagonist is used to treat graft-versus-host disease (GVHD). In some embodiments, a PSMP antagonist is used to treat acute GVHD (aGVHD). In other embodiments, a PSMP antagonist is used to treat chronic GVHD (cGVHD).

In yet other aspects of the present disclosure, a PSMP antagonist is used to treat systemic lupus erythematosus (SLE).

In yet other aspects of the present disclosure, a PSMP antagonist is used to treat lupus nephritis.

In yet other aspects of the present disclosure, a PSMP antagonist is used to treat IgA nephropathy.

In yet other aspects of the present disclosure, a PSMP antagonist is used to treat membranous glomerulonephritis.

In still other aspects of the present disclosure, a PSMP antagonist is used to treat a disease or disorder associated with liver fibrosis, such as, *e.g.,* chronic alcohol abuse *(e.g.,* alcoholic liver disease (ALD), alcoholic hepatitis, alcoholic cirrhosis), chronic viral hepatitis *(e.g.,* hepatitis B, C, or D), fat accumulation in the liver (nonalcoholic fatty liver disease *(e.g.,* nonalcoholic steatohepatitis (NASH)), iron buildup in the body (hemochromatosis), cystic fibrosis, copper accumulation in the liver (Wilson's disease), poorly formed bile ducts (biliary atresia), alpha-1 antitrypsin deficiency, an inherited disorder of sugar metabolism (*e.g*., galactosemia, glycogen storage disease), a genetic digestive disorder (*e.g*., Alagille syndrome), an autoimmune liver disease (*e.g*., autoimmune hepatitis, primary sclerosing cholangitis (PSC), primary biliary cholangitis (PBC, previously known as primary biliary cirrhosis), an infection *(e.g.,* syphilis, brucellosis), drug-induced liver injury *(e.g.,* methotrexate- or isoniazid-induced livery injury), or Budd-Chiari syndrome.

In still other aspects of the present disclosure, a PSMP antagonist is used to treat a disease or disorder associated with lung fibrosis, such as, *e.g*., dermatomyositis, polymyositis, mixed connective tissue disease, systemic lupus erythematosus, rheumatoid arthritis, sarcoidosis, scleroderma, pneumonia, chronic radiation pneumonitis, a pneumoconiosis, an infection, or drug-induced lung injury. In some embodiments in which a PSMP antagonist is used to treat pneumoconiosis, the pneumoconiosis is caused or exacerbated by exposure to silica dust, asbestos fibers, a hard metal dust, coal dust, grain dust, or bird or animal droppings. In some embodiments in which a PSMP antagonist is used to treat drug-induced lung injury, the lung injury is induced by a chemotherapy drug (*e.g*., methotrexate, cyclophosphamide, bleomycin), a heart medication (*e.g*., a drug used to treat irregular heartbeats such as, for example, amiodarone), an antibiotic (*e.g*., nitrofurantoin, ethambutol), or an anti-inflammatory drug (*e.g*., rituximab, sulfasalazine).

In still other aspects of the present disclosure, a PSMP antagonist is used to treat a disease or disorder associated with kidney fibrosis, such as, *e.g.,* IgA nephropathy, membranoproliferative glomerulonephritis, membranous glomerulonephritis, crescentic glomerulonephritis, diabetic nephropathy, hypertension nephropathy, lupus nephritis, hepatic nephropathy, polycystic kidney disease, Alport syndrome, Fabry disease, primary hyperoxaluria, cystinosis; coenzyme Q10-related gene mutations causing focal segmental glomerulosclerosis (FSGS), complement 3 glomerulonephritis, acute or subacute immune complex glomerulonephritis, renal vasculitis, systemic lupus erythematosus (SLE), recent-onset renal artery stenosis (fibromuscular or vasculitic), diabetic kidney disease, chronic urate nephropathy, toxic nephropathies, bacterial pyelonephritis, viral nephropathies, multiple myeloma, or obstructive nephropathy.

In each of the embodiments of the treatment methods described herein, the PSMP antagonist is delivered in a manner consistent with conventional methodologies associated with management of the disease or disorder for which treatment is sought. In accordance with the disclosure herein, an effective amount of the antagonist is administered to a subject in need of such treatment for a time and under conditions sufficient to treat the disease or disorder.

Subjects for administration of PSMP antagonists as described herein include patients at high risk for developing a particular disease or disorder (*e.g*., liver fibrosis, lung fibrosis, kidney fibrosis, or a disease or disorder associated with liver or lung fibrosis) as well as patients presenting with an existing disease or disorder. In certain embodiments, the subject has been diagnosed as having the disease or disorder for which treatment is sought. Further, subjects can be monitored during the course of treatment for any change in the disease or disorder *(e.g.,* for an increase or decrease in clinical symptoms of the disease or disorder). Also, in some variations, the subject does not suffer from another disease or disorder requiring treatment that involves inhibiting PSMP signaling pathways.

In prophylactic applications, pharmaceutical compositions or medicants are administered to a patient susceptible to, or otherwise at risk of, a particular disease or disorder in an amount sufficient to eliminate or reduce the risk or delay the outset of the disease or disorder. In therapeutic applications, compositions or medicants are administered to a patient suspected of, or already suffering from, such a disease in an amount sufficient to cure or at least partially arrest the symptoms of the disease or disorder and its complications. An amount adequate to accomplish this is referred to as a therapeutically or pharmaceutically effective dose or amount. In both prophylactic and therapeutic regimes, agents are usually administered in several dosages until a sufficient response (*e.g*., inhibition of fibrosis or a biomarker of fibrosis) has been achieved. Typically, the response is monitored and repeated dosages are given if the desired response starts to fade.

To identify subject patients for treatment according to the methods of the invention, accepted screening methods may be employed to determine risk factors associated with specific diseases or disorders or to determine the status of an existing disease or disorder identified in a subject. Such methods can include, for example, determining whether an individual has relatives who have been diagnosed with a particular disease. Screening methods can also include, for example, conventional work-ups to determine familial status for a particular disease known to have a heritable component. For example, various liver diseases are also known to have certain inheritable components. *See, e.g.,* Scorza et al., International Journal of Hepatology, Volume 2014, Article ID 713754, 11 pages (genetic defects causing early chronic liver involvement); Severson et al., World J. Gastroenterol. 22:6742-6756, 2016 (genetic factors affecting development of nonalcoholic fatty liver disease). Toward this end, molecular diagnostic assays (*e.g*., nucleic-acid-based diagnostic assays) can be routinely employed to identify individuals carrying genetic markers associated with a disease of interest. Screening may also be implemented as indicated by known patient symptomology, age factors, related risk factors, *etc.* These methods allow the clinician to routinely select patients in need of the methods described herein for treatment. In accordance with these methods, administration of a PSMP antagonist may be implemented as an independent treatment program or as a follow-up, adjunct, or coordinate treatment regimen to other treatments.

For administration, the PSMP antagonist is formulated as a pharmaceutical composition. A pharmaceutical composition comprising a PSMP antagonist can be formulated according to known methods to prepare pharmaceutically useful compositions, whereby the therapeutic molecule is combined in a mixture with a pharmaceutically acceptable carrier. A composition is said to be a "pharmaceutically acceptable carrier" if, when combined with an active ingredient, it allows the active ingredient to retain biological activity and its administration can be tolerated by a recipient patient. Examples include, , any of the standard pharmaceutical carriers such as a phosphate buffered saline solution, water, emulsions such as oil/water emulsion, and various types of wetting agents. Preferred diluents for aerosol or parenteral administration are phosphate buffered saline (PBS) or normal (0.9%) saline. Compositions comprising such carriers are formulated by well-known conventional methods *(see, e.g.,* Gennaro (ed.), Remington's Pharmaceutical Sciences (Mack Publishing Company, 19th ed. 1995); Allen (ed.), Remington: The Science and Practice of Pharmacy (Pharmaceutic Press, 22nd revised ed.).) Formulations may further include one or more excipients, preservatives, solubilizers, buffering agents, albumin to prevent protein loss on vial surfaces, *etc.* Monospecific antagonists can be individually formulated or provided in a combined formulation.

A pharmaceutical composition comprising a PSMP antagonist is administered to a subject in an effective amount. According to the methods of the present disclosure, an antagonist may be administered to subjects by a variety of administration modes, including, for example, by intramuscular, subcutaneous, intravenous, intra-atrial, intra-articular, parenteral, intranasal, intrapulmonary, transdermal, intrapleural, intrathecal, and oral routes of administration. For prevention and treatment purposes, an antagonist may be administered to a subject in a single bolus delivery, via continuous delivery (*e.g*., continuous transdermal delivery) over an extended time period, or in a repeated administration protocol *(e.g.,* on an hourly, daily, or weekly basis). The exact dose will be determined by the clinician according to accepted standards, taking into account the nature and severity of the condition to be treated, patient traits, *etc.* Determination of dose is within the level of ordinary skill in the art.

Determination of effective dosages in this context is typically based on animal model studies followed up by human clinical trials and is guided by determining effective dosages and administration protocols that significantly reduce the occurrence or severity of the subject disease or disorder in model subjects. Effective doses of the compositions of the present invention vary depending upon many different factors, including means of administration, target site, physiological state of the patient, whether the patient is human or an animal, other medications administered, whether treatment is prophylactic or therapeutic, as well as the specific activity of the composition itself and its ability to elicit the desired response in the individual. Usually, the patient is a human, but in some diseases, the patient can be a nonhuman mammal. Typically, dosage regimens are adjusted to provide an optimum therapeutic response, *i.e.,* to optimize safety and efficacy. Accordingly, a therapeutically or prophylactically effective amount is also one in which any undesired collateral effects are outweighed by beneficial effects. For administration of a PSMP antagonist, a dosage typically ranges from about 0.1 µg to 100 mg/kg or 1 µg/kg to about 50 mg/kg, and more usually 10 µg to 5 mg/kg of the subject's body weight. In more specific embodiments, an effective amount of the agent is between about 1 µg/kg and about 20 mg/kg, between about 10 µg/kg and about 10 mg/kg, or between about 0.1 mg/kg and about 5 mg/kg. Dosages within this range can be achieved by single or multiple administrations, including, *e.g*., multiple administrations per day or daily, weekly, bi-weekly, or monthly administrations. For example, in certain variations, a regimen consists of an initial administration followed by multiple, subsequent administrations at weekly or bi-weekly intervals. Another regimen consists of an initial administration followed by multiple, subsequent administrations at monthly or bi-monthly intervals. Alternatively, administrations can be on an irregular basis as indicated by monitoring of fibrosis and/or clinical symptoms of the disease or disorder.

Particularly suitable animal models for evaluating efficacy of a PSMP antagonist for treatment of liver, lung, or kidney fibrosis are generally known in the art. For example, one model of liver fibrosis uses CCL₄ injection. *See, e.g.,* You et al., Mol. Med. Rep. 12:5594-5600, 2015. CCL₄ toxic liver fibrosis can be induced, for example, in 6-8-week-old male mice by intraperitoneal (i.p.) injections of CCl₄ *(e.g.,* 1.0 ml/kg body weight, dissolved in corn oil at a ratio of 1:9) twice a week for 4 or 6 weeks. Another exemplary model of liver fibrosis uses bile duct ligation (BDL) to induce cholestasis in mice. *See, e.g.,* Yongping et al., J. Ethnopharmacol. 169:200-209, 2015. Using this model, mice *(e.g.,* 6-8-week-old male mice) develop cholestasis and associated fibrosis over a period *(e.g.,* 14 days) following ligation of the common bile duct.

One suitable model for lung fibrosis is the bleomycin-induced pulmonary fibrosis model. *See, e.g.,* Rangarajan et al., Nat. Med. 8:1121-1127, 2018; doi: 10.1038/s41591-018-0087-6. For bleomycin-induced pulmonary fibrosis, mice (*e.g*., 6-8-week-old female C57Bl/6J mice) are anesthetized before tracheotomy and injected with bleomycin (*e.g*., a single injection containing 3.5 U/kg of bleomycin), and pulmonary fibrosis develops over a period (*e.g., 14* days) following injection.

A suitable model for kidney (renal) fibrosis is the Unilateral Ureteral Obstruction (UUO) model, in which renal fibrosis is caused by tubular injury as a result of obstructed urine flow. *See, e.g.,* Elena Martinez-Klimova et al., Biomolecules 9:141, 2019.

Animal models of diseases or disorders associated with liver, lung, or kidney fibrosis are also known. An exemplary model of alcoholic liver fibrosis is described, *e.g*., by Ambade et al. (Hepatology, 2018, doi: 10.1002/hep.30249). For nonalcoholic steatohepatitis (NASH), various dietary, chemical, and genetic models may be used. *See generally, e.g.,* Hansen et al., Drug Discov Today, 22, 1707, 2017. For example, one NASH model is a combined model of chemical and dietary challenges by the administration of streptozotocin (STZ) with a high fat diet (HFD). In the STZ-HFD model, NASH is induced by injection of STZ *(e.g.,* a single subcutaneous injection of 200 µg STZ in male mice at two days after birth) and feeding with HFD *ad libitum,* typically after four weeks of age for eight weeks. Another exemplary NASH model is a nutrient-deficient dietary model-the methionine-choline deficient (MCD) model-commonly used in preclinical NASH research. In the MCD model, NASH is induced by MCD feeding *ad libitum.* Typically, MCD mice develop hepatic macrovesicular steatosis and infiltration of inflammatory cells after one to three weeks of dieting and robust perisinusoidal fibrosis occurs from the fifth to seventh weeks. For cholestatic disease, such as primary sclerosing cholangitis (PSC) and primary biliary cholangitis (PBC), an exemplary model is chronic feeding of 3,5-diethoxycarbonyl-1,4-dihydrocollidine (DDC).

The Unilateral Ureteral Obstruction (UUO) model of renal fibrosis *(see* Elena Martinez-Klimova *et al., supra*) is the most common rodent model used to study acute kidney injury (AKI) and chronic kidney disease (CKD). In addition, a popular mouse model for AKI following rhabdomyolysis is glycerol injection into the leg muscle. *See, e.g.,* Yanqiu et al., Stem Cell Research & Therapy 5:80, 2014. For lupus nephritis, a well-known model is the DBA/2-B6D2F1 model, in which DBA/2 mice spleen cells are transfused into B6D2F1 mice. *See, e.g.,* Via et al., J Immunol. 139:1840-1849, 1987.

Animal models for evaluating efficacy of a PSMP antagonist for treatment of GVHD are also generally known. *See generally, e.g.,* Schroeder et al., Dis. Model Mech. 4:318-333, 2011. In one exemplary acute GVHD (aGVHD) model, BABL/c mice (*e.g.,* 6-8-week-old males) act as the recipient and C57BL/6 mice are the donors. The spleen CD3+ T cells and bone marrow cells from the C57BL/6 mice are separated after mice are sacrificed. Before cell infusion (*e.g*., 12 hours before), recipient mice are exposed to radiation (for example, two Co⁶⁰ radiation doses of 4 Gy each, for a total of 8 Gy). CD3+ cells and bone marrow cells are mixed at a ratio of, *e.g.,* 5x10⁶ to 1x10⁷, and then are injected intravenously into the BABL/c mice. During the aGVHD period, typically defined as one month from infusion, mouse weight and survival rate are monitored every two days. In addition, a well-known chronic GVHD (cGVHD) mouse model is the DBA/2→B6D2F1 model (also a model for systemic lupus erythematosus (SLE) and lupus nephritis, *see, e.g.,* Via *et al., supra*), established by the transfusion of DBA/2 mice spleen cells into B6D2F1 mice. *See generally, e.g.,* Kim et al., J. Immunol. 181:7380-7389, 2008.

Dosage of the pharmaceutical composition may be varied by the attending clinician to maintain a desired concentration at a target site. For example, if an intravenous mode of delivery is selected, local concentration of the agent in the bloodstream at the target tissue may be between about 1-50 nanomoles of the composition per liter, sometimes between about 1.0 nanomole per liter and 10, 15, or 25 nanomoles per liter depending on the subject's status and projected measured response. Higher or lower concentrations may be selected based on the mode of delivery, *e.g*., trans-epidermal delivery versus delivery to a mucosal surface. Dosage should also be adjusted based on the release rate of the administered formulation, *e.g.*, nasal spray versus powder, sustained release oral or injected particles, transdermal formulations, *etc.* To achieve the same serum concentration level, for example, slow-release particles with a release rate of 5 nanomolar (under standard conditions) would be administered at about twice the dosage of particles with a release rate of 10 nanomolar.

A pharmaceutical composition comprising a PSMP antagonist can be furnished in liquid form, in an aerosol, or in solid form. Liquid forms are illustrated by injectable solutions, aerosols, droplets, topological solutions and oral suspensions. Exemplary solid forms include capsules, tablets, and controlled-release forms. The latter form is illustrated by miniosmotic pumps and implants. *See, e.g.,* Bremer et al., Pharm. Biotechnol. 10:239, 1997; Ranade, "Implants in Drug Delivery," in Drug Delivery Systems 95-123 (Ranade and Hollinger, eds., CRC Press 1995); Bremer et al., "Protein Delivery with Infusion Pumps," in Protein Delivery: Physical Systems 239-254 (Sanders and Hendren, eds., Plenum Press 1997); Yewey et al., "Delivery of Proteins from a Controlled Release Injectable Implant," in Protein Delivery: Physical Systems 93-117 (Sanders and Hendren, eds., Plenum Press 1997). Other solid forms include creams, pastes, other topological applications, and the like.

Liposomes provide one means to deliver therapeutic polypeptides to a subject, *e.g.*, intravenously, intraperitoneally, intrathecally, intramuscularly, subcutaneously, or via oral administration, inhalation, or intranasal administration. Liposomes are microscopic vesicles that consist of one or more lipid bilayers surrounding aqueous compartments. *See, generally,* Bakker-Woudenberg et al., Eur. J. Clin. Microbiol. Infect. Dis. 12 (Suppl. 1):S61, 1993; Kim, Drugs 46:618, 1993; Ranade, "Site-Specific Drug Delivery Using Liposomes as Carriers," in Drug Delivery Systems 3-24 (Ranade and Hollinger, eds., CRC Press 1995). Liposomes are similar in composition to cellular membranes and as a result, liposomes can be administered safely and are biodegradable. Depending on the method of preparation, liposomes may be unilamellar or multilamellar, and liposomes can vary in size with diameters ranging from 0.02 µm to greater than 10 µm. A variety of agents can be encapsulated in liposomes: hydrophobic agents partition in the bilayers and hydrophilic agents partition within the inner aqueous space(s). *See, e.g.,* Machy et al., Liposomes In Cell Biology And Pharmacology (John Libbey 1987); Ostro et al., American J. Hosp. Pharm. 46:1576, 1989. Moreover, it is possible to control the therapeutic availability of the encapsulated agent by varying liposome size, the number of bilayers, lipid composition, as well as the charge and surface characteristics of the liposomes.

Liposomes can adsorb to virtually any type of cell and then slowly release the encapsulated agent. Alternatively, an absorbed liposome may be endocytosed by cells that are phagocytic. Endocytosis is followed by intralysosomal degradation of liposomal lipids and release of the encapsulated agents (*see* Scherphof et al., Ann. N.Y. Acad. Sci. 446:368, 1985). After intravenous administration, small liposomes (0.1 to 1.0 µm) are typically taken up by cells of the reticuloendothelial system, located principally in the liver and spleen, whereas liposomes larger than 3.0 µm are deposited in the lung. This preferential uptake of smaller liposomes by the cells of the reticuloendothelial system has been used to deliver therapeutic agents to the liver.

The reticuloendothelial system can be circumvented by several methods including saturation with large doses of liposome particles, or selective macrophage inactivation by pharmacological means (*see* Claassen et al., Biochim. Biophys. Acta 802:428, 1984). In addition, incorporation of glycolipid- or polyethelene glycol-derivatized phospholipids into liposome membranes has been shown to result in a significantly reduced uptake by the reticuloendothelial system (*see* Allen et al., Biochim. Biophys. Acta 1068:133, 1991; Allen et al., Biochim. Biophys. Acta 1150:9, 1993).

Liposomes can also be prepared to target particular cells or organs by varying phospholipid composition or by inserting receptors or counter-receptors into the liposomes. For example, liposomes, prepared with a high content of a nonionic surfactant, have been used to target the liver. *See, e.g.,* Japanese Patent 04-244,018 to Hayakawa et al.*;* Kato et al., Biol. Pharm. Bull. 16:960, 1993. These formulations were prepared by mixing soybean phospatidylcholine, α-tocopherol, and ethoxylated hydrogenated castor oil (HCO-60) in methanol, concentrating the mixture under vacuum, and then reconstituting the mixture with water. A liposomal formulation of dipalmitoylphosphatidylcholine (DPPC) with a soybeanderived sterylglucoside mixture (SG) and cholesterol (Ch) has also been shown to target the liver. *See* Shimizu et al., Biol. Pharm. Bull. 20:881, 1997.

Alternatively, various targeting counter-receptors can be bound to the surface of the liposome, such as antibodies, antibody fragments, carbohydrates, vitamins, and transport proteins. For example, for targeting to the liver, liposomes can be modified with branched type galactosyllipid derivatives to target asialoglycoprotein (galactose) receptors, which are exclusively expressed on the surface of liver cells. *See* Kato and Sugiyama, Crit. Rev. Ther. Drug Carrier Syst. 14:287, 1997; Murahashi et al., Biol. Pharm. Bull.20:259, 1997. In a more general approach to tissue targeting, target cells are prelabeled with biotinylated antibodies specific for a counter-receptor expressed by the target cell. *See* Harasym et al., Adv. Drug Deliv. Rev. 32:99, 1998. After plasma elimination of free antibody, streptavidin-conjugated liposomes are administered. In another approach, targeting antibodies are directly attached to liposomes. *See* Harasym *et al., supra.*

Antibodies and other soluble proteins can be encapsulated within liposomes using standard techniques of protein microencapsulation. *See, e.g.,* Anderson et al., Infect. Immun. 31:1099, 1981; Anderson et al., Cancer Res. 50:1853, 1990; Cohen et al., Biochim. Biophys. Acta 1063:95, 1991; Alving et al. "Preparation and Use of Liposomes in Immunological Studies," in Liposome Technology (Vol. III) 317 (Gregoriadis, ed., CRC Press, 2nd ed. 1993); Wassef et al., Meth. Enzymol. 149:124, 1987. As noted above, therapeutically useful liposomes may contain a variety of components. For example, liposomes may comprise lipid derivatives of poly(ethylene glycol). *See* Allen et al., Biochim. Biophys. Acta 1150:9, 1993.

Degradable polymer microspheres have been designed to maintain high systemic levels of therapeutic proteins. Microspheres are prepared from degradable polymers such as poly(lactide-co-glycolide) (PLG), polyanhydrides, poly (ortho esters), nonbiodegradable ethylvinyl acetate polymers, in which proteins are entrapped in the polymer. *See, e.g.,* Gombotz and Pettit, Bioconjugate Chem. 6:332, 1995; Ranade, "Role of Polymers in Drug Delivery," in Drug Delivery Systems 51-93 (Ranade and Hollinger, eds., CRC Press 1995); Roskos and Maskiewicz, "Degradable Controlled Release Systems Useful for Protein Delivery," in Protein Delivery: Physical Systems 45-92 (Sanders and Hendren, eds., Plenum Press 1997); Bartus et al., Science 281:1161, 1998; Putney and Burke, Nature Biotechnology 16:153, 1998; Putney, Curr. Opin. Chem. Biol. 2:548, 1998. Polyethylene glycol (PEG)-coated nanospheres can also provide carriers for intravenous administration of therapeutic proteins. *See, e.g.,* Gref et al., Pharm. Biotechnol. 10:167, 1997.

Other dosage forms can be devised by those skilled in the art, as shown by, *e.g.,* Ansel and Popovich, Pharmaceutical Dosage Forms and Drug Delivery Systems (Lea & Febiger, 5th ed. 1990); Gennaro (ed.), Remington's Pharmaceutical Sciences (Mack Publishing Company, 19th ed. 1995); Ranade and Hollinger, Drug Delivery Systems (CRC Press 1996); and Allen (ed.), Remington: The Science and Practice of Pharmacy (Pharmaceutic Press, 22nd revised ed.).

Pharmaceutical compositions may be supplied as a kit comprising a container that comprises a PSMP antagonist composition as described herein. A pharmaceutical composition can be provided, for example, in the form of an injectable solution for single or multiple doses, or as a sterile powder that will be reconstituted before injection. Alternatively, such a kit can include a dry-powder disperser, liquid aerosol generator, or nebulizer for administration of a pharmaceutical composition. Such a kit may further comprise written information on indications and usage of the pharmaceutical composition.

**Table 1: Exemplary Sequences**

| **SEQ ID NO** | **Sequence** | **Description/ Comments** |
|---|---|---|
| 1 | MALRMLWAGQAKGILGGWGIICLVMSLLLQHPGVYSKCYFQA QAPCHYEGKYFTLGESWLRKDCFHCTCLHPVGVGCCDTSQHP IDFPAGCEVRQEAGTCQFSLVQKSDPRLPCKGGGPDPEWGSA NTPVPGAPAPHSS | Human PSMP protein, Signal sequence underlined |
| 2 | MLSTSRSRFIRNTNESGEEVTTFFDYDYGAPCHKFDVKQIGA QLLPPLYSLVFIFGFVGNMLVVLILINCKKLKCLTDIYLLNL AISDLLFLITLPLWAHSAANEWVFGNAMCKLFTGLYHIGYFG GIFFIILLTIDRYLAIVHAVFALKARTVTFGVVTSVITWLVA VFASVPGIIFTKCQKEDSVYVCGPYFPRGWNNFHTIMRNILG LVLPLLIMVICYSGILKTLLRCRNEKKRHRAVRVIFTIMIVY FLFWTPYNIVILLNTFQEFFGLSNCESTSQLDQATQVTETLG MTHCCINPIIYAFVGEKFRSLFHIALGCRIAPLQKPVCGGPG VRPGKNVKVTTQGLLDGRGKGKSIGRAPEASLQDKEGA | Human CCR2A protein |
| 3 | MLSTSRSRFIRNTNESGEEVTTFFDYDYGAPCHKFDVKQIGA QLLPPLYSLVFIFGFVGNMLVVLILINCKKLKCLTDIYLLNL AISDLLFLITLPLWAHSAANEWVFGNAMCKLFTGLYHIGYFG GIFFIILLTIDRYLAIVHAVFALKARTVTFGVVTSVITWLVA VFASVPGIIFTKCQKEDSVYVCGPYFPRGWNNFHTIMRNILG LVLPLLIMVICYSGILKTLLRCRNEKKRHRAVRVIFTIMIVY FLFWTPYNIVILLNTFQEFFGLSNCESTSQLDQATQVTETLG MTHCCINPIIYAFVGEKFRRYLSVFFRKHITKRFCKQCPVFY RETVDGVTSTNTPSTGEQEVSAGL | Human CCR2B protein |
| 4 | EVQLQQSGPELVKPGASVKISCKASGYSFTGYFMNWVMQSHG KSLEWIGRINPYNGDTFYNQKFKGKATLTVDKSSSTAHMELR SLASEDSAVYYCARGGDGYYAFAYWGQGTLVTVSA | mAb 3D5 VH domain protein, Chothia CDRs underlined |
| 5 | NIVMTQSPKSMSMSVGERVTLSCRASENVGTYVSWYQQKPEQ SPKLLIYGASNRYTGVPDRFTGSGSATDFTLTISSVQAEDLA DYHCGQSYSYMYTFGGGTKLEIKR | mAb 3D5 VL domain protein, Chothia CDRs underlined |
| 6 | gaggttcagctgcagcagtctggacctgagctggtgaagcct ggggcttcagtgaagatatcctgcaaggcttctggttactca tttactggctactttatgaactgggtgatgcagagccatgga aagagccttgagtggattggacgtattaatccttacaatggt gatactttctacaaccagaagttcaagggcaaggccacattg actgtagacaaatcctctagtacagcccacatggagctccgg agcctggcatctgaggactctgcagtctattattgtgcaaga ggtggggatggttactacgcgtttgcttactggggccaaggg actctggtcactgtctctgca | mAb 3D5 VH domain DNA |
| 7 | aacattgtaatgacccaatctcccaaatccatgtccatgtca gtaggagagagggtcaccttgagctgcagggccagtgagaat gtgggtacttatgtatcctggtatcaacagaaaccagagcag tctcctaaactgctgatatacggggcatccaaccggtacact ggggtccccgatcgcttcacaggcagtggatctgcaacagat ttcactctgaccatcagcagtgtgcaggctgaagaccttgca gattatcactgtggacagagttacagctatatgtacacgttc ggaggggggaccaagctggaaataaaacgg | mAb 3D5 VL domain DNA |

The invention is further illustrated by the following non-limiting examples.

### Example 1: PSMP expression is upregulated in human and murine liver fibrosis

To evaluate whether PSMP expression is associated with liver disease, PSMP levels were examined by immunohistochemistry in tissue microarrays containing different liver disease tissues. PSMP was significantly upregulated in cirrhotic and adjacent, nontumour liver tissues (FIGs. 1A and 1B). This finding was confirmed in human liver fibrotic tissue with different causes including hepatitis B virus (HBV)-induced cirrhosis (n=14), hepatitis C virus (HCV)-induced cirrhosis (n=1), primary biliary cirrhosis (n=5), and alcohol-induced cirrhosis (n=2). Immunohistochemical analyses revealed that hepatic PSMP expression was significantly elevated in patients with cirrhosis compared to normal human liver tissues (FIGs. 2A and 2B). Similar to the human data, the expression of PSMP was also markedly increased in mouse models of liver fibrosis induced by CCl₄ and BDL compared with the livers from the control mice (FIGs. 3A-3D, 4A, and 4B). These data indicate that PSMP is upregulated in cirrhotic livers, raising the hypothesis that the activation of PSMP signaling might be involved in the pathogenesis of liver fibrosis.

### Example 2: Deficiency of PSMP protects against liver fibrosis in mice

To further examined the role of PSMP signaling in liver fibrosis, fibrogenesis was then investigated in PSMP knockout mice subjected to a toxic fibrosis mouse model induced by CCl₄ treatment. Mice were repetitively exposed to CCl₄ for 4 weeks (2 times/week), *Psmp*^{*-*/-} mice displayed significantly attenuated liver injury and fibrosis assessed by Hematoxylin and eosin (H&E), Sirius Red staining and hepatic hydroxyproline content compared with oil controls comparable with *Ccr2*^{*-*/*-*} mice (FIGs. 5A-5D). Consistently, upregulation of α-SMA, a marker of HSC activation, was notably decreased in *Psmp*^{*-*/*-*} mice as assessed by immunohistochemistry and immunoblotting concordant with the *Ccr2*^{*-*/-}mice (FIGs. 5A and 5F). The serum levels of alanine aminotransferase (ALT) showed a clear tendency of reduction in *Psmp*^{*-*/*-*} mice, indicative of improvement of liver damage (FIG. 5E). Like *Ccr2*^{*-*/*-*} mice, the hepatic mRNA expression of prototypical profibrotic genes (*Acta2, Collal, Tgfbl, Timp1* and *Pdgfrb*) were reduced in CCl₄-induced *Psmp*^{*-*/*-*} mice (FIGs. 5G-5K).

To further analyze the role of PSMP in liver fibrosis, the BDL mouse model of liver fibrosis, another well-established model, was employed. Following BDL, *Psmp*^{*-*/*-*} mice also displayed a significant reduction of the Sirius Red-positive area, hydroxyproline content, and α-SMA expression (FIGs. 6A-6D and 6F). Serum ALT level was also elevated in *Psmp*^{*-*/*-*} mice after BDL (FIG. 6E). Significant reduction of *Acta2, Collal, Tgfbl, Timp1* and *Pdgfrb* mRNA levels was also found in *Psmp*^{*-*/*-*} mice (FIGs. 6G-6K).

Taken together, these results suggest that activation of the PSMP signaling is likely involved in the pathogenesis of hepatic fibrosis.

### Example 3: Neutralization of PSMP signalling alleviates murine liver fibrosis

On the basis of PSMP deficiency significantly attenuating development of liver fibrosis in mice, the effect of specific PSMP-neutralizing antibody, 3D5, on liver fibrosis induced by CCl₄ was examined. First, to investigate the protective effects of 3D5 on mice with 4 weeks of CCl₄-induced liver fibrosis, the mice were treated with 3D5 after each CCl₄ injection (FIG. 7A). H&E and Sirius Red staining assays showed reduced liver injury and fibrosis after 3D5 treatment compared with the control and mIgG-treated groups (FIGs. 7B-7D). Accordingly, 3D5 notably reduced α-SMA expression in the fibrotic livers (FIGs. 7B and 7G). Moreover, the liver hydroxyproline content was significantly lower in the 3D5-treated groups than in the control and mIgG-treated groups (FIG. 7E). Serum levels of ALT were significantly lower in 3D5-treated groups than in the control and mIgG-treated groups, indicative of improvement of liver function (FIG. 7F). The qRT-PCR showed that 3D5 also significantly reduced the mRNA levels of fibrogenic genes (*Acta2, Collal, Tgfbl, Timpl* and *Pdgfrb*) (FIGs. 7H-7L).

Moreover, to evaluate the therapeutic potential of 3D5 in 6 weeks of CCl₄-induced liver fibrosis, 3D5 treatment was used in mice from the 4th week to the 6th week with CCl₄ treatment (FIG. 8A). Mice with 3D5 treatment displayed a significant reduction of the Sirius Red-positive area, hydroxyproline content, α-SMA expression, serum ALT compared with control and mIgG-treated groups (FIGs. 8B-8F). Consistently, significant reduction of *Acta2, Colla1, Tgfb1, Timp1,* and *Pdgfrb* mRNA levels was also observed in mice treated with 3D5 (FIGs. 8G-8K).

Different doses of 3D5 (1 mg/kg, 5 mg/kg, and 10 mg/mL) were used to treat mice with CCl₄-induced liver fibrosis (FIG. 9A). H&E staining and Sirius Red staining assay showed that mice with liver fibrosis and treated with 3D5 had dose-dependently milder liver fibrosis than did the control and mIgG-treated groups (FIGs. 9B-9E). The levels of α-SMA expression, serum ALT, mRNA expression of fibrogenic genes (*Acta2, Collal, Tgfbl, Timp1* and *Pdgfrb*) in CCl₄-treated mice were also dose-dependently lower after 3D5 treatment (FIGs. 9B and 9F-9L).

These results suggest that blockage of PSMP signaling significantly ameliorates the pathogenesis of liver fibrosis in mice.

### Example 4: AAV8-hPSMP restores hepatic PSMP expression and promotes liver fibrosis in a CCR2-dependent manner

To further explore the role of PSMP in liver fibrosis, AAV8 (a gene vector isolated from rhesus monkeys, which is used to transduce hepatocytes because of its high affinity for liver cells) was used to overexpress human PSMP in PSMP^{-/-} mice (FIG. 10A). As expected, PSMP^{-/-} mice that received AAV8-hPSMP had strongly increased levels of expression of hPSMP compared with those receiving a control AAV8-Null (FIG. 10E). Next, using this approach, the consequences of PSMP overexpression under conditions of chronic liver damage was examined. Four weeks after the AAV8 injection, the mice were treated with CCl₄ for another four weeks to induce liver fibrosis (FIG. 10A). Significant augment was found in liver injury and fibrosis that had been elicited by PSMP overexpression compared to the effects observed in mice that were injected with the AAV8-Null (FIGs. 10B-10D, 10F-10L).

The data further show that PSMP overexpression promotes liver fibrosis development.

### Example 5: PSMP deficiency inhibits hepatic macrophages infiltration and pro-inflammatory cytokines production induced by CCL₄ challenge

The infiltration of macrophages into the liver upon chronic injury has been convincingly linked to the progression of liver inflammation and fibrosis in mice and humans. Therefore, the composition of immune cells in the liver after CCl₄ treatment was investigated by flow cytometric analysis. Infiltrating hepatic macrophages (iMΦ, CD11b⁺F4/80^{int}) and CD11b⁺CCR2⁺ cells accumulation was significantly reduced in *Psmp*^{-/-} mice (FIGs. 11A-11E). Parallel to the intrahepatic macrophages, other immune cells such as neutrophils (CD11b⁺Ly6G⁺), B cells and T cells (CD3⁺,CD4⁺, CD8⁺) were also investigated and were found to be not affected except for some elevation or decrease of T cells in the PSMP-knockout mice (FIGs. 11F-11J).

Next, the consequences of reduced hepatic macrophage accumulation due to PSMP deficiency in the liver were investigated. An important function of hepatic macrophages during disease progression is the secretion of proinflammatory cytokines. *Psmp*^{*-*/*-*} mice had lower serum concentrations of CCL2, TNF-α, IL-6 and IL-12 upon CCl₄ treatment, which is in line with the reduced numbers of intrahepatic macrophages (FIGs. 11K-11N).

These data indicate that PSMP may promote fibrosis through affecting the infiltrating of macrophages and then the production of proinflammatory cytokines.

### Example 6: Materials and Methods for Liver Fibrosis Studies

This Example describes materials and methods for the liver fibrosis studies described in Examples 1-5.

### Human samples

All human liver samples were collected at Hospital. Cirrhosis samples were collected from HBV-induced cirrhosis, HCV-induced cirrhosis, primary biliary cirrhosis, alcoholic-induced cirrhosis. Normal liver tissue was collected from patients receiving hepatic resections for non-tumoral diseases, including hepatic adenoma and focal nodula hyperplasia.

### Animals

Specific pathogen-free C57BL/6 mice were purchased from Peking Experimental Animal Center (Beijing, China). PSMP knockout mice were C57BL/6 background. CCR2 knockout mice were C57BL/6 background and obtained from Professor Yu Zhang (Department of Immunology, Peking University Health Science Center). Mice with double knockout (DKO) of PSMP and CCR2 were obtained by crossing PSMPKO and CCR2KO mice. All animal experiments were carried out according to the Guidelines for the Care and Use of Laboratory Animals and were approved by the Ethics Committee of Peking University Health Science Center.

### Murine liver fibrosis models

For the toxic liver fibrosis, 6-8-week-old male mice received intraperitoneal (i.p.) injections of CCl₄ (1.0 ml/kg body weight, dissolved in corn oil at a ratio of 1:9) or vehicle (corn oil) twice a week for 4 or 6 weeks. The mice were sacrificed two days after the final CCl₄ (Aladdin, Shanghai, China) injection.

For the induction of cholestatic liver fibrosis, 6-8-week-old male mice underwent ligation of the common bile duct. Sham-operated mice underwent the same procedure but without ligation. Bile duct ligation (BDL) mice developed cholestasis and associated fibrosis over a 14-day period.

### Antibody treatments

Monoclonal hybridoma cells expressing neutralizing antibody for PSMP (3D5; Pei et al., J. Immunol. 192:1878-86, 2014) were injected into the mouse abdominal cavity and ascites was obtained. mAb 3D5 in the ascites was purified using protein G. For protective treatment, the mice were injected (i.p.) with 5 mg/kg of 3D5 twice a week after each CCl₄ injection for whole 4 weeks. For therapeutic treatment, the mice were injected (i.p.) with 5 mg/kg of 3D5 twice a week after each CCl₄ injection from the 4th week to the 6th week.

### Mouse adeno-associated virus 8 (AAV8) construction and injection

The AAV8 delivery system overexpressing human PSMP gene in mouse livers is constructed by Vigene Bioscience (Shangdong, China). The empty associated adenovirus (AAV8-Null) served as a control. Titers of the vector genome were measured by qPCR with vector-specific primers. The mice were injected with 100 *µ*l of virus containing 2 × 10¹¹ AAV8 vector genomes via the tail vein.

### Cytometric bead assay (CBA)

Microspheres (A37304, Life Technologies) were coated with polyclonal rabbit anti-PSMP antibody according to the manufacturer's instructions. The coated beads were blocked with 20% FBS for 30 min and then added to the samples for 2 hours at room temperature, and the monoclonal anti-PSMP antibodies were then added for 1 hour. The mixture was suspended with PE goat anti-mouse antibody (eBiocience) for 30 min at room temperature. The data were acquired by flow cytometry.

The cytokines IL-6, CCL2, TNF-α, and IL-12p70 were detected with a BD^{™} Cytometric Bead Array (CBA) Mouse Inflammation Kit (552364, BD Biosciences) according to the manufacturer's instructions. The data were acquired by flow cytometry.

### Liver histological and immunohistological staining

Liver specimens were preserved in 10% formalin and cut into 4 µm-thick sections. Sections were then stained with hematoxylin and eosin (H&E) by a normal procedure and with Sirius Red staining for 1 hour. Sirius Red staining was used to grade the extent of fibrosis according to Ishak scoring system. Hepatic fibrosis was graded according to Ishak scoring system: Grade 0 - no fibrosis; grade 1 - fibrous expansion of some portal areas with or without short fibrous septa; grade 2 - fibrous expansion of most portal areas, with or without short fibrous septa; grade 3 - fibrous expansion of most portal areas with occasional portal to portal (P-P) bridging; grade 4 - fibrous expansion of portal areas with marked bridging [portal to portal (P-P) as well as portal to central (P-C)]; grade 5 - marked bridging (P-P and/or P-C) with occasional nodules (incomplete cirrhosis); grade 6 - cirrhosis, probable or definite. The cumulative histologic scores could have ranged from 0 (entirely normal) to 6 (severe disease with cirrhosis).

### Immunohistochemistry

For immunohistochemistry (IHC), liver specimens were fixed in 10% buffered formalin. PSMP IHC staining was performed as described by Pei et al., J. Immunol. 192:1878-86, 2014. For α-SMA IHC staining, monoclonal antibody to α-SMA (Abcam, ab7817) was used.

### Immunoblotting analysis

Proteins in lysates were resolved by SDS-PAGE electrophoresis and transferred to PVDF membrane (Millipore Corporation, MA, USA). Blots were incubated with Rabbit polyclonal antibody to α-SMA visualized them by enhanced chemiluminescence (ECL) system. The densitometric measurement of each band was analyzed using Quantity-One Protein Analysis Software (Bio-Rad Laboratories, USA) and normalized to GAPDH (β-actin) as internal control.

### Quantitative real-time PCR assay

The tissue or cells were homogenized in Trizol (Invitrogen). Reverse transcription (RT) reactions were performed by using the RT MasterMix system (ABM Inc.). Real-time PCR was carried out using SYBR Green PCR mix (ABM Inc.). All gene expressions were normalized to that of Gapdh.

### Determining liver hydroxyproline

Liver samples, obtained immediately after the mice were killed, were subjected to a modified acid hydrolysis protocol to determine their hydroxyproline levels. The hydroxyproline contents of liver are expressed as mg of hydroxyproline per gram of liver.

### Serum ALT

The liver function of mice with liver injury was monitored based on serum levels of AST and ALT. Enzyme activities are expressed as units per liter (U/L).

### Flow cytometry

Liver mononuclear cells were taken and blocked in 5% fetal bovine serum for 20 minutes, then stained with CD45-FITC, CD11b-PE, CCR2-APC, Ly6C-PerCP Cy5.5, Ly6G-PerCP Cy5.5, F4/80-APC, and CD11c-APC (BioLegend) at 4°C for 30 minutes. Data were acquired from FACS caliber (BD Biosciences) and analyzed using FlowJo 7.6 (TreeStar, Ashland, OR, USA).

### Statistical analysis

All data are expressed as the mean ± standard error of the mean. Statistical differences between two groups were analyzed by the Student's t test with GraphPad Prism 6.0 (GraphPad Software, San Diego, CA, USA). Statistical significance differences between two groups are presented by *0.01 < p < 0.05, **0.001 < p < 0.01, and ***p < 0.001.

### Example 7: PSMP Expression and Effects of PSMP Deficiency or Neutralization during Pulmonary Fibrosis

PSMP expression and effects of PSMP deficiency during pulmonary fibrosis were analyzed using a bleomycin-model of fibrosis. Neutralization of PSMP signaling alleviates bleomycin-induced pulmonary fibrosis in a mouse model.

### Methods

Six to eight-week-old female C57Bl/6J (WT) mice were used in this study. Animals were anesthetized intraperitoneally before tracheotomy. A single injection containing 3.5U/kg of bleomycin (BLM) (Nippon Kayaku Co. Ltd, Japan) diluted in Saline, or Saline only (NS) (control group), was instilled intratracheally. Fourteen days after bleomycin, animals were euthanized to perform bronchoalveolarlavage and collect lung samples for biochemical and histologic analysis. The total content of lung collagen was measured by the Masson's trichome stain following the manufacturer's instructions. For the treatment, the mice were injected (i.p.) with 10 mg/kg of PSMP neutralizing antibody 3D5 or PBS once a week for three weeks after the single BLM injection.

Student's t-test was used for comparisons between two groups. All tests utilized one-tailed methodology. A P value of less than 0.05 was considered significant. Replicates consisted of at least three independent experiments.

### Results

In the lung tissue of animals instilled with bleomycin, an upregulation of PSMP was observed (FIGs. 12A and 12C), which was consistent with the phenomenon observed in bronchoalveolarlavage (FIG. 12B). As compared with WT animals, PSMP^{-/-} mice showed decreased body weight loss (FIG 12D.) Consistent with this, the increase in lung collagen content observed in WT animals instilled with bleomycin was significantly reduced in PSMP^{-/-} mice (FIG. 12E). Mice treated with 3D5 displayed a significant reduction of α-SMA expression, a marker of fibroblast activation compared with the PBS-treated mice (FIG. 12F). Immunohistochemical analysis revealed that there is marked staining for PSMP in airway fibrosis human lung, while no staining of PSMP in normal tissue (FIG. 12G).

### Example 8: Deficiency of PSMP protects against liver fibrosis in a nonalcoholic steatohepatitis (NASH) model

### Methods

Two-day-old infant mice were subcutaneously administered 200µg of streptozotocin (STZ, Sigma, MO, USA). The high-fat diet (HFD, composed of 60 kcal% fat) started when mice reached 4 weeks of age and continued throughout the experiment period until 12 weeks. Fibrosis was evaluated by Sirius Red staining with paraffin section.

### Results

In the liver tissue of PSMP^{-/-} mice, a significant reduction of fibrosis was observed (FIG. 13).

### Example 9: Treatment of acute GVHD with anti-PSMP neutralizing antibody

### Methods

In this study, 6-8-week-old male BABL/c mice acted as recipient and C57BL/6 mice were donors. The spleen CD3+ T cells and bone marrow cells from C57BL/6 mice were separated after mice sacrificed. 12 hours before infusion of the cells into BABL/c mice, recipient mice were exposed to two doses of Co⁶⁰ radiation (4 Gy each dose, for a total of 8 Gy). CD3+ cells and bone marrow cells were mixed at a ratio of 5x10⁶ to 1x10⁷ and then were injected intravenously into BABL/c mice. During a period of one month from infusion (the acute GVHD (aGVHD) period), mouse weight and survival rate were monitored every two days for each of the two groups (mAb 3D5 treatment and IgG control groups).

The neutralizing antibody of PSMP, 3D5, was injected intraperitoneally every week after cell infusion with a dose of 5 mg/kg, and mouse IgG was used as a negative control treatment.

### Results

Anti-PSMP neutralizing antibody extended the survival time of aGVHD mice. Mice reaching more than 30% weight loss were defined as unable to survive and were sacrificed. As shown in FIG. 14, aGVHD mice died at about the 19th day after infusion, while mice in the 3D5 treated group died at about the 24^{th} day after infusion. The survival time of aGVHD mice was summarized in two groups with mouse IgG or 3D5 treated, and the significant difference was calculated. The survival time of aGVHD mice showed a significant difference between IgG and 3D5 treated group.

### Example 10: Neutralization or deficiency of PSMP signaling alleviates liver steatosis and fibrosis in chronic ethanol-feeding model

### Methods

### Murine models

The chronic ethanol-feeding model used the commercially available control and ethanol Lieber-DeCarli diets, which are equicaloric and have the same composition with respect to fat (35% of calories) and protein (18% of calories). The content of carbohydrates is 47% of total calories (dextrin-maltose) in the control diet and 11% of total calories in the ethanol diet, where up to 36% of carbohydrate calories are replaced by ethanol.

For the chronic ethanol-feeding model with ethanol Lieber-DeCarli diet, 10-week-old female mice (~22 g of body weight) were acclimatized to the liquid diets by feeding them the control diet for 5 d. The percentage of ethanol-derived calories was progressively increased over a period of one week and then maintained on the 5% ethanol diet for eight weeks. Mice were pair-fed throughout the entire experiment.

For the chronic-plus-binge alcohol feeding model, 8-week-old female mice (~20 g of body weight) were acclimatized to the liquid diets by feeding them the control diet for 5 d. The percentage of ethanol-derived calories was progressively increased over a period of one week and then maintained on the 5% ethanol diet for ten days. At day 11, mice were gavaged a single dose of ethanol (5g/kg body weight, 31.5% ethanol). The gavage was always performed in the early morning. After gavage, mice were kept on ethanol diet and kept in the cages on the warm blanket with circulating water. After gavage, mice were slow-moving, but conscious and regained normal behavior within 4-6 h. The mice were always euthanized 9 h post gavage.

For antibody treatment, the mice were injected (i.p.) with 10 mg/kg of PSMP neutralizing antibody 3D5 or PBS once a week for 4 weeks (beginning at week 5 through 8 of alcohol feeding, at the middle of one week) in the chronic ethanol-feeding model. For the antibody treatment in the chronic-plus-binge alcohol feeding model, the mice were injected (i.p.) with 20 mg/kg of 3D5 or PBS one hour before gavage.

### Serum lipid contents

Serum triglyceride or cholesterol contents were determined using triglyceride or cholesterol assay kit (Zhong Sheng, Beijing, China).

### Histopathology

To examine hepatic morphology and assess liver fibrosis, hematoxylin and eosin (H&E) staining and Sirius Red staining were performed. Liver fibrosis was quantified by Sirius Red staining. Liver specimens were fixed in 10% neutral buffered formalin, embedded in paraffin and cut into 4 µm sections. For analysis of fat accumulation in the liver, 10 µm sections were cut from frozen samples and stained with Oil Red O (Solarbio Science & Technology, Co, Ltd. Beijing, China) for 10 min. These slides were rinsed in water and counterstained with Mayer's hematoxylin, and analyzed by light microscopy. The positive area was calculated in at least 10 magnification (×200) fields.

### Liver immunohistochemical staining

For immunohistochemistry staining, sections were blocked with 10% goat serum solution for 30 min at room temperature, followed by incubation with primary antibodies against α-SMA (Abcam, ab32575) at 4°C overnight. Sections were incubated with secondary antibodies (ZSGB-Bio) and 3,3-diaminobenzidine (DAB), followed by brief counterstaining with H&E staining, and mounted with neutral gum.

### Biochemical assays

Serum levels of alanine aminotransferase (ALT) were measured using standard enzymatic procedures according to the manufacturer's instructions (Nanjing Jiancheng Bioengineering Institute, Nanjing, China). Serum and liver triglyceride or cholesterol contents were determined using triglyceride or cholesterol assay kit (Zhong Sheng, Beijing, China).

### Reverse transcription reactions and quantitative real time polymerase chain reaction (RT-qPCR)

The tissue or cells were homogenized in TRIzol (Life Technologies, 15596018). Reverse transcription reactions were performed using the Hifair II 1st Strand cDNA Synthesis SuperMix (Yeasen Biotech, 11120ES60). Quantitative real-time polymerase chain reaction was carried out using Hieff qPCR SYBR Green Master mix (Yeasen Biotech, 11202ES03). All gene expression levels were normalized to that of Gapdh.

### Results

Analyses of serum lipid content confirmed that 3D5 treatment mice had lower levels of triglycerides and cholesterol than PBS treatment mice after ethanol feeding (FIGs. 15B and 15C). Liver hematoxylin and eosin (H&E) and Oil Red O staining revealed that 3D5 treatment ethanol-fed mice had a lower degree of steatosis and fibrosis than PBS treatment ethanol-fed mice (FIGs. 15D-15F). The hepatic expression levels of *Ppara* and *Srebp-1* mRNA were measured by RT-qPCR. In agreement with these findings, 3D5 treatment had an expression of downregulated *Srebp-1* and upregulated *Ppara* than PBS treatment in ethanol-fed mice (FIGs. 15G and 15H). Liver fibrosis was further determined by Sirius Red staining. As illustrated in FIGs. 15I and 15J, in the PBS treatment group, mice had greater Sirius Red than 3D5 treatment mice. Furthermore, the composition of immune cells in the liver was investigated by flow cytometric analysis in a chronic-plus-binge alcohol feeding model. Infiltrating hepatic macrophages (iMΦ, F4/80⁺ CD11b^{high}) accumulation were reduced in 3D5 treatment mice (FIG. 15K).

In a chronic-plus-binge alcohol feeding model (FIG. 15L), analyses of liver lipid content confirmed that 3D5 treatment mice also had lower levels of triglycerides and cholesterol than PBS treatment mice after ethanol feeding (FIGs. 15M and 15N). Liver hematoxylin and eosin (H&E) revealed that 3D5 treatment ethanol-fed mice had a lower degree of steatosis than PBS treatment ethanol-fed mice (FIG. 15O). The composition of immune cells in the liver was also investigated by flow cytometric analysis. Infiltrating hepatic macrophages (iMΦ, F4/80⁺ CD11b^{high}) accumulation were reduced in 3D5-treated mice (FIG. 15P).

In a chronic-plus-binge alcohol feeding model, Psmp^{-/-} mice displayed significantly attenuated liver injury and steatosis, as assessed by H&E and Oil red O staining, compared with WT mice (FIGs. 15Q and 15R). The serum levels of alanine aminotransferase (ALT) showed a decrease in Psmp^{-/-} mice, indicating improvement of liver damage (FIG. 15S). Consistently, Psmp^{-/-} mice had an expression of downregulated *Srebp-1* and upregulated *Ppara* compared with WT mice (FIGs. 15T and 15U).

### Example 11: PSMP expression is upregulated in murine liver injury and fibrosis

### Methods

For the induction of acute liver injury, 6- to 8-week-old male WT and *Psmp*^{*-*/*-*} mice were induced by a single i.p. injection of Acetaminophen (APAP, 300 mg/kg body weight, dissolved in warm PBS (55°C) (Sigma-Aldrich) (n=5/group). Control mice received a similar volume of vehicle. Mice were euthanized 24 h after injection.

For the 5-diethoxycarbonyl-1,4-dihydrocollidine (DDC) diet, 8-week-old male WT mice were fed a diet supplemented with 0.1% DDC (Sigma-Aldrich, St Louis, MO) for 4 weeks (n=5/group). Control mice received a standard mouse diet. For antibody treatment, mice were injected (i.p.) with 5 mg/kg of PSMP neutralizing antibody 3D5 or mIgG twice a week from the 3rd week to the 4th week.

### Results

PSMP expression was markedly increased in mouse model of liver fibrosis induced by DDC-diet or mouse acute liver injury model by acetaminophen (APAP) treatment compared with the livers from the control mice (FIGs 16A-16C).

### Example 12: PSMP deficiency protects against liver fibrosis in DDC mice

To analyze the role of PSMP in liver fibrosis due to other causes, the DDC-diet model (*see* Example 11, Methods) was employed. The DDC-diet model recapitulates clinical features of human biliary fibrosis. After 4 weeks of DDC feeding, *Psmp*^{*-*/*-*} mice also displayed a significant reduction of the Sirius Red-positive area and α-SMA expression (FIGs. 17A-17D). A significant reduction in mRNA levels of *Acta2, Colla1, Tgfb1, Timp1* and *Pdgfr* in *Psmp*^{*-*/*-*}mice was also observed (FIGs. 17E-17I).

### Example 13: Neutralization of PSMP signaling alleviates murine DDC-induced liver fibrosis

To evaluate the therapeutic potential of 3D5 on liver fibrosis induced by DDC diet (*see* Example 11, Methods), we used 3D5 treatment in mice from the 3rd week to the 4th week (FIG. 18A). H&E and Sirius Red staining assays showed reduced liver injury and fibrosis after 3D5 treatment compared with that of the mIgG treatment group (FIGs. 18B-18D). Accordingly, 3D5 notably reduced α-SMA expression in the fibrotic livers (FIGs. 18B and 18E). Quantitative real-time PCR (qRT-PCR) assays showed that 3D5 also significantly reduced the mRNA levels of fibrogenic genes (*Acta2, Col1a1*) (FIGs. 18F and 18G).

### Example 14: PSMP deficiency protects against liver fibrosis in MCD mice

### Methods

For the methionine-choline-deficient (MCD) diet, 8-week-old male WT and *Psmp*^{*-*/-} mice were fed a MCD diet (Research Diets, A02082002B) for 6 weeks (n=5/group). Control mice received a standard mouse diet.

### Results

A methionine-choline-deficient (MCD) diet-induced mouse nonalcoholic steatohepatitis (NASH) model was constructed. After 6 weeks of MCD feeding, PSMP expression was markedly increased in WT mice (FIG. 19A). *Psmp*^{*-*/*-*} mice showed a significant decrease in the area stained with Sirius Red compared to WT mice (FIGs. 19B-19D). A significant decrease in mRNA expression levels of *Acta2, Colla1* and *Tgfb1* in the liver of *Psmp*^{*-*/*-*} mice was also detected (FIGs. 19E-19G).

### Example 15: PSMP neutralizing antibody treatment alleviates murine liver fibrosis in MCD-induced NASH model

### Methods

For antibody treatment, 8-week-old male mice were fed the methionine and choline deficient (MCD) diet for 5.5 weeks. The mice were injected (i.p.) with 10 mg/kg of PSMP neutralizing antibody 3D5 or PBS once a week from the 4th week to the 5.5th week. For another antibody treatment, 8-week-old male mice were fed the MCD diet for 8 weeks. The mice were injected (i.p.) with 10 mg/kg of PSMP neutralizing antibody 3D5 or PBS once a week from the 5th week to the 8th week.

### Results

To evaluate the therapeutic potential of PSMP neutralizing antibody 3D5 on liver fibrosis induced by MCD diet, we used 3D5 treatment in mice from the 4th week to the 5.5th week (FIG. 20A). H&E and Sirius Red staining assays and its quantification showed reduced liver injury and fibrosis after 3D5 treatment compared with that of the PBS treatment group (FIGs. 20B-20D). Another 3D5 treatment in MCD mice was from the 5th week to the 8th week (FIG. 20E). Sirius Red and α-SMA immunohistochemistry staining and its quantification showed reduced liver fibrosis after 3D5 treatment compared with that of the PBS treatment group (FIGs. 20F-20I).

### Example 16: Deficiency or neutralization of PSMP alleviates murine acute kidney injury (AKI) and chronic kidney disease (CKD)

### Methods

### Murine models

In the UUO groups, left ureter of 6-week-old male mice (~20g) were exposed through a left-flank incision on the abdomen and ligated twice with a 4-0 silk suture. For treatment, the mice were injected (i.p.) with 5 mg/kg of 3D5 or PBS after UUO at day 1, 4. After 5 days, the mice were sacrificed.

In the glycerol induced rhabdomyolysis group, mice were injected intramuscularly with 7.5 ml/kg 50% glycerol diluted with PBS into their thigh muscles. For treatment, the mice were injected (i.p.) with 5 mg/kg of 3D5 or PBS after injection of glycerol. After 48 h, the mice were sacrificed.

Subsequently, the left kidney was removed, cut transversely, and either fixed in 4% paraformaldehyde for histopathology or snap-frozen in liquid nitrogen for other experiments.

### Biochemical assays

The determination of serum creatinine and blood urea nitrogen levels is of great value in helping to ascertain the renal function in the clinical setting. Serum levels of creatinine and blood urea nitrogen (BUN) were measured using assay kit according to the manufacturer's instructions (Nanjing Jiancheng Bioengineering Institute, Nanjing, China).

### Histopathology and immunohistochemical analysis

Paraformaldehyde-fixed kidney tissues were embedded in paraffin and sectioned at 4 µm. The sections were dewaxed in xylene, rehydrated in decreasing ethanol concentrations, and stained with Sirius Red. For immunohistochemistry staining, sections were blocked with 10% goat serum solution for 30 min at room temperature, followed by incubation with primary antibodies against α-SMA (Abcam, ab32575) at 4°C overnight. Sections were incubated with secondary antibodies (ZSGB-Bio) and 3, 3-diaminobenzidine (DAB), followed by brief counterstaining with H&E staining, and mounted with neutral gum. The positive area was calculated in at least 10 magnification (×200) fields.

### Results

### PSMP expression is upregulated in human kidney disease

To determine whether PSMP expression is associated with kidney disease, PSMP levels were initially examined by immunohistochemistry. PSMP showed negative or weak expression in the human normal kidney tissue from adjacent clear cell carcinoma. PSMP was upregulated in renal tubules of lupus nephritis, IgA nephritis, and membranous glomerulonephritis (FIGs. 21A and 21B).

### Deficiency and neutralization of PSMP alleviates the glycerol induced rhabdomyolysis

*Psmp*^{*-*/*-*} mice of glycerol induced rhabdomyolysis displayed significantly attenuated renal injury as assessed by serum creatinine compared with WT mice (FIG. 21C). Simultaneously, the therapeutic effects of PSMP neutralizing antibody 3D5 on mice of glycerol-induced rhabdomyolysis were investigated, as determined using serum creatinine and BUN levels (FIGs. 21D and 21E).

### Neutralization of PSMP signaling alleviates renal fibrosis in UUO model

To investigate the therapeutic effects of PSMP neutralizing antibody 3D5 on mice of UUO-induced renal fibrosis, mice were treated with 3D5 after UUO at day 1 and day 4 (FIG. 21F). Sirius Red staining assays showed reduced renal fibrosis after 3D5 treatment compared with the PBS-treated group (FIGs. 21G and 21H; grey bar - 3D5-treated group, black bar - PBS-treated group).

### Example 17: Treatment of chronic GVHD with anti-PSMP neutralizing antibody

Chronic GVHD is a syndrome of variable clinical features presenting as an autoimmune-like syndrome. cGVHD organ damage in mice can involve skin, gut, liver, lung, salivary glands, or oral mucosa. *See generally, e.g.,* Schroeder et al., Dis. Model Mech. 4:318-333, 2011.

### Methods

In this study, a chronic GVHD (cGVHD) model was established by infusion of 7x10⁷ spleen cells from 6-week-old male DBA2 mice into 6-week-old female B6D2F1 mice without irradiation. Anti-PSMP neutralizing antibody 3D5 was injected intraperitoneally every week after cell infusion with a dose of 2.5 mg/kg, and mouse IgG was used as a negative control treatment. Mice were monitored for body weight and appearance, and markers of liver function were detected at day 80 after spleen cell infusion.

### Results

Anti-PSMP neutralizing antibody 3D5 reversed the progress of cGVHD mice. As shown in FIG.22A, there was no difference in body weight between the control group and the 3D5-treated group. At the day 40 after spleen cells infusion, fur shedding was observed on the eyelid region of the control group mice but not the neutralizing antibody 3D5 group. From day 40 to day 80, the fur shedding was gradually severe as shown in FIG. 22B, but fur shedding was not observed in the neutralizing antibody 3D5 group. At day 80 after spleen cell infusion, peripheral blood from the two groups was collected and markers of liver function were detected. The markers for liver damage, ALT and AST, in the serum samples from the control group were significantly higher than those from the 3D5-treated group (FIGs. 22C and 22D).

### Example 18: Treatment of lupus with anti-PSMP neutralizing antibody

### Methods

The male DBA/2-female B6D2F1 graft-versus-host disease model also is a well-known murine lupus nephritis model. In this study, a murine lupus model was established by infusion of 7x10⁷ spleen cells from 6-week-old male DBA/2 mice into 6-week-old female B6D2F1 mice. Mouse IgG or anti-PSMP neutralizing antibody 3D5 was injected intraperitoneally every week after cell infusion with a dose of 2.5 mg/kg. The markers of kidney function were detected at day 80 after spleen cell infusion. For ELISA to detect total IgG in serum, mice were bled from tail vein and serum samples were obtained. The total IgG was detected via Mouse IgG ELISA kit from Sigma (catalog no. 11333151001) following the manufacturer's protocol. To detect autoantibody deposition in the glomerulus, kidneys were embedded and snap-frozen in liquid nitrogen. Sections (8 µm) were fixed with acetone and stained with FITC-conjugated anti-mouse IgG (BD Biosciences). Fluorescence was examined by confocal microscopy.

### Results

Because the expression of PSMP was found to be upregulated in kidney tissue from lupus nephritis patients (*see* Example 16 and FIGs. 21A and 21B), the effects of PSMP neutralizing antibody 3D5 in a murine lupus model was explored. At day 80 after spleen cell infusion, the renal function of 3D5-treated mice was detected and compared with the control group. The PSMP neutralizing antibody 3D5 group had significantly improved kidney function relative to the control group as shown by the level of serum creatinine and blood urea nitrogen (BUN) (FIGs. 23A and 23B). Renal injury in lupus may be related to the autoantibody deposited in the kidney tissue. This study showed that the PSMP neutralizing antibody 3D5 could reduce the serum total IgG level and autoantibody deposition in the glomerulus compared with the control group (FIGs. 23C and 23D), indicating that the neutralizing antibody 3D5 against PSMP had a renal protecting potential during lupus.

From the foregoing, it will be appreciated that, although specific embodiments of the invention have been described herein for purposes of illustration, various modifications may be made without deviating from the scope of the invention, which is defined by the appended claims

## Claims

1. A PC3-secreted microprotein (PSMP) antagonist for use in the treatment of liver, lung, or kidney fibrosis, wherein the PSMP antagonist is an antibody that specifically binds to PSMP.

2. The PSMP antagonist for use of claim 1, wherein the PSMP antagonist is for use in the treatment of liver fibrosis, optionally wherein the liver fibrosis has progressed to liver cirrhosis.

3. The PSMP antagonist for use of claim 2, wherein the liver fibrosis is hepatitis B virus (HBV)-induced, hepatitis C virus (HCV)-induced, or alcohol-induced liver fibrosis.

4. The PSMP antagonist for use of claim 2, wherein the liver fibrosis is associated with a disease selected from the group consisting of nonalcoholic fatty liver disease (NAFLD), alcoholic liver disease (ALD), primary sclerosing cholangitis (PSC), and primary biliary cholangitis (PBC), optionally wherein the nonalcoholic fatty liver disease is nonalcoholic steatohepatitis (NASH).

5. The PSMP antagonist for use of claim 1, wherein the PSMP antagonist is for use in the treatment of lung fibrosis, optionally wherein the lung fibrosis is associated with a druginduced lung injury.

6. The PSMP antagonist of claim 1, wherein the PSMP antagonist is for use in the treatment of kidney fibrosis,
optionally wherein the kidney fibrosis is associated with a disease or disorder selected from the group consisting of lupus nephritis, IgA nephropathy, and membranous glomerulonephritis.

7. A PC3-secreted microprotein (PSMP) antagonist for use in the treatment of a disease selected from the group consisting of nonalcoholic fatty liver disease (NAFLD), alcoholic liver disease (ALD), primary sclerosing cholangitis (PSC), and primary biliary cholangitis (PBC), wherein the PSMP antagonist is an antibody that specifically binds to PSMP.

8. The PSMP antagonist for use of claim 7, wherein nonalcoholic fatty liver disease is nonalcoholic steatohepatitis (NASH).

9. A PC3-secreted microprotein (PSMP) antagonist for use in the treatment of acute kidney injury (AKI) or chronic kidney disease (CKD), wherein the PSMP antagonist is an antibody that specifically binds to PSMP,
optionally wherein the AKI is rhabdomyolysis-induced,
optionally wherein the CKD is caused by a disease or disorder selected from the group consisting of lupus nephritis, IgA nephropathy, and membranous glomerulonephritis.

10. A PC3-secreted microprotein (PSMP) antagonist for use in the treatment of a disease or disorder selected from the group consisting of graft-versus-host disease (GVHD), systemic lupus erythematosus (SLE), and lupus nephritis wherein the PSMP antagonist is an antibody that specifically binds to PSMP.

11. The PSMP antagonist for use of any one of claims 1, 7, 9, and 10, wherein the antibody competes for binding to PSMP with a second antibody comprising a heavy chain variable domain (VH) having the amino acid sequence shown in SEQ ID NO:4 and a light chain variable domain (VL) having the amino acid sequence shown in SEQ ID NO:5.

12. The PSMP antagonist for use of claim 11 wherein the antibody comprises a VH domain comprising complementarity determining regions (CDRs) CDR-H1_{Ab}, CDR-H2_{Ab}, and CDR-H3_{Ab}, wherein said set of VH CDRs has three or fewer amino acid substitutions relative to a reference set of CDRs CDR-H1_{Ref}, CDR-H2_{Ref}, and CDR-H3_{Ref} in which
CDR-H1_{Ref} is a CDR-H1 of SEQ ID NO:4;
CDR-H2_{Ref} is a CDR-H2 of SEQ ID NO:4; and
CDR-H3_{Ref} is a CDR-H3 of SEQ ID NO:4;
optionally wherein each VH CDR is defined according to the Chothia definition of CDR, whereby
CDR-H1_{Ref} has the amino acid sequence shown in residues 31-35 of SEQ ID NO:4;
CDR-H2_{Ref} has the amino acid sequence shown in residues 50-69 of SEQ ID NO:4; and
CDR-H3_{Ref} has the amino acid sequence shown in residues 99-108 of SEQ ID NO:4.

13. The PSMP antagonist for use of claim 12, wherein the antibody comprises a VL domain comprising complementarity determining regions (CDRs) CDR-L1_{Ab}, CDR-L2_{Ab}, and CDR-L3_{Ab}, wherein said set of VL CDRs has three or fewer amino acid substitutions relative to a reference set of CDRs CDR-L1_{Ref}, CDR-L2_{Ref}, and CDR-L3_{Ref} in which
CDR-L1_{Ref} is a CDR-L1 of SEQ ID NO:5;
CDR-L2_{Ref} is a CDR-L2_of SEQ ID NO:5; and
CDR-L3_{Ref} is a CDR-L3_of SEQ ID NO:5;
optionally wherein each VL CDR is defined according to the Chothia definition of CDR, whereby
CDR-L1_{Ref} has the amino acid sequence shown in residues 24-34 of SEQ ID NO:5;
CDR-L2_{Ref} has the amino acid sequence shown in residues 50-56 of SEQ ID NO:5; and
CDR-L3_{Ref} has the amino acid sequence shown in residues 89-97 of SEQ ID NO:5.

14. The PSMP antagonist for use of claim 11, wherein the antibody is a humanized antibody, a chimeric antibody, or a human antibody.

15. The PSMP antagonist of any one of claims 11 to 13, wherein the antibody is a single chain antibody.

## Patentansprüche

1. Antagonist des PC3-sekretierten Mikroproteins (PSMP) zur Verwendung bei der Behandlung von Leber-, Lungen- oder Nierenfibrose, wobei der PSMP-Antagonist ein Antikörper ist, der spezifisch an PSMP bindet.

2. PSMP-Antagonist zur Verwendung nach Anspruch 1, wobei der PSMP-Antagonist zur Verwendung bei der Behandlung von Leberfibrose dient, wobei optional die Leberfibrose zu Leberzirrhose fortgeschritten ist.

3. PSMP-Antagonist zur Verwendung nach Anspruch 2, wobei die Leberfibrose durch das Hepatitis-B-Virus (HBV) ausgelöste, durch das Hepatitis-C-Virus (HCV) ausgelöste oder durch Alkohol ausgelöste Leberfibrose ist.

4. PSMP-Antagonist zur Verwendung nach Anspruch 2, wobei die Leberfibrose mit einer Erkrankung assoziiert ist, ausgewählt aus der Gruppe bestehend aus nichtalkoholischer Fettlebererkrankung (NAFLE), alkoholischer Lebererkrankung (ALE), primär sklerosierender Cholangitis (PSC) und primär biliärer Cholangitis (PBC), wobei optional die nichtalkoholische Fettlebererkrankung nichtalkoholische Steatohepatitis (NASH) ist.

5. PSMP-Antagonist zur Verwendung nach Anspruch 1, wobei der PSMP-Antagonist zur Verwendung bei der Behandlung von Lungenfibrose dient, wobei optional die Lungenfibrose mit einer durch Arzneimittel ausgelösten Lungenschädigung assoziiert ist.

6. PSMP-Antagonist nach Anspruch 1, wobei der PSMP-Antagonist zur Verwendung bei der Behandlung von Nierenfibrose dient,
wobei optional die Nierenfibrose mit einer Erkrankung oder Störung assoziiert ist, ausgewählt aus der Gruppe bestehend aus Lupus-Nephritis, IgA-Nephropathie und membranöser Glomerulonephritis.

7. Antagonist des PC3-sekretierten Mikroproteins (PSMP) zur Verwendung bei der Behandlung einer Erkrankung, ausgewählt aus der Gruppe bestehend aus nichtalkoholischer Fettlebererkrankung (NAFLE), alkoholischer Lebererkrankung (ALE), primär sklerosierender Cholangitis (PSC) und primär biliärer Cholangitis (PBC), wobei der PSMP-Antagonist ein Antikörper ist, der spezifisch an PSMP bindet.

8. PSMP-Antagonist zur Verwendung nach Anspruch 7, wobei die nichtalkoholische Fettlebererkrankung nichtalkoholische Steatohepatitis (NASH) ist.

9. Antagonist des PC3-sekretierten Mikroproteins (PSMP) zur Verwendung bei der Behandlung von akutem Nierenversagen (ANV) oder chronischer Nierenerkrankung (CNK), wobei der PSMP-Antagonist ein Antikörper ist, der spezifisch an PSMP bindet,
wobei optional das ANV durch Rhabdomyolyse ausgelöst ist,
wobei optional die CNK von einer Erkrankung oder Störung ausgelöst ist, ausgewählt aus der Gruppe bestehend aus Lupus-Nephritis, IgA-Nephropathie und membranöser Glomerulonephritis.

10. Antagonist des PC3-sekretierten Mikroproteins (PSMP) zur Verwendung bei der Behandlung einer Erkrankung oder Störung, ausgewählt aus der Gruppe bestehend aus Graft-versus-Host-Krankheit (GvHK), systemischem Lupus erythematodes (SLE) und Lupus-Nephritis, wobei der PSMP-Antagonist ein Antikörper ist, der spezifisch an PSMP bindet.

11. PSMP-Antagonist zur Verwendung nach einem der Ansprüche 1, 7, 9 und 10, wobei der Antikörper mit einem zweiten Antikörper, umfassend eine variable Domäne der schweren Kette (VH) mit der in SEQ ID NO:4 gezeigten Aminosäuresequenz und eine variable Domäne der leichten Kette (VL) mit der in SEQ ID NO:5 gezeigten Aminosäuresequenz, um die Bindung an PSMP konkurriert.

12. PSMP-Antagonist zur Verwendung nach Anspruch 11, wobei der Antikörper eine VH-Domäne, umfassend die komplementaritätsbestimmenden Regionen (CDRs) CDR-H1_{Ab}, CDR-H2_{Ab} und CDR-H3_{Ab} umfasst, wobei der Satz von VH-CDRs drei oder weniger Aminosäuresubstitutionen in Bezug auf einen Referenzsatz der CDRs CDR-H1_{Ref}, CDR-H2_{Ref} und CDR-H3_{Ref} hat, wobei
CDR-H1_{Ref} eine CDR-H1 von SEQ ID NO:4 ist;
CDR-H2_{Ref} eine CDR-H2 von SEQ ID NO:4 ist; und
CDR-H3_{Ref} eine CDR-H3 von SEQ ID NO:4 ist;
wobei optional jede VH-CDR gemäß der Chothia-Definition von CDR definiert ist, wobei
CDR-H1_{Ref} die Aminosäuresequenz hat, die in den Resten 31-35 von SEQ ID NO:4 gezeigt ist;
CDR-H2_{Ref} die Aminosäuresequenz hat, die in den Resten 50-69 von SEQ ID NO:4 gezeigt ist; und
CDR-H3_{Ref} die Aminosäuresequenz hat, die in den Resten 99-108 von SEQ ID NO:4 gezeigt ist.

13. PSMP-Antagonist zur Verwendung nach Anspruch 12, wobei der Antikörper eine VL-Domäne, umfassend die komplementaritätsbestimmenden Regionen (CDRs) CDR-L1_{Ab}, CDR-L2_{Ab} und CDR-L3_{Ab} umfasst, wobei der Satz von VL-CDRs drei oder weniger Aminosäuresubstitutionen in Bezug auf einen Referenzsatz der CDRs CDR-L1_{Ref}, CDR-L2_{Ref} und CDR-L3_{Ref} hat, wobei
CDR-L1_{Ref} eine CDR-L1 von SEQ ID NO:5 ist;
CDR-L2_{Ref} eine CDR-L2 von SEQ ID NO:5 ist; und
CDR-L3_{Ref} eine CDR-L3 von SEQ ID NO:5 ist;
wobei optional jede VL-CDR gemäß der Chothia-Definition von CDR definiert ist, wobei
CDR-L1_{Ref} die Aminosäuresequenz hat, die in den Resten 24-34 von SEQ ID NO:5 gezeigt ist;
CDR-L2_{Ref} die Aminosäuresequenz hat, die in den Resten 50-56 von SEQ ID NO:5 gezeigt ist; und
CDR-L3_{Ref} die Aminosäuresequenz hat, die in den Resten 89-97 von SEQ ID NO:5 gezeigt ist.

14. PSMP-Antagonist zur Verwendung nach Anspruch 11, wobei der Antikörper ein humanisierter Antikörper, ein chimärer Antikörper oder ein menschlicher Antikörper ist.

15. PSMP-Antagonist nach einem der Ansprüche 11 bis 13, wobei der Antikörper ein einkettiger Antikörper ist.

## Revendications

1. Antagoniste de la microprotéine sécrétée par PC3 (PSMP) destiné à être utilisé dans le traitement de la fibrose hépatique, pulmonaire ou rénale, dans lequel l'antagoniste de la PSMP est un anticorps qui se lie spécifiquement à la PSMP.

2. Antagoniste de la PSMP pour utilisation selon la revendication 1, dans lequel l'antagoniste de la PSMP est destiné à être utilisé dans le traitement de la fibrose hépatique, éventuellement dans lequel la fibrose hépatique a évolué vers une cirrhose hépatique.

3. Antagoniste de la PSMP pour utilisation selon la revendication 2, dans lequel la fibrose hépatique est une fibrose hépatique induite par le virus de l'hépatite B (VHB), par le virus de l'hépatite C (VHC), ou une fibrose hépatique induite par l'alcool.

4. Antagoniste de la PSMP pour utilisation selon la revendication 2, dans lequel la fibrose hépatique est associée à une maladie choisie dans le groupe composé de la stéatose hépatique non alcoolique (SHNA), de la maladie alcoolique du foie (MAF), de la cholangite sclérosante primitive (CSP) et de la cholangite biliaire primitive (CBP), éventuellement dans laquelle la stéatose hépatique non alcoolique est la stéatohépatite non alcoolique (SHNA).

5. Antagoniste de la PSMP pour utilisation selon la revendication 1, dans lequel l'antagoniste de la PSMP est destiné à être utilisé dans le traitement de la fibrose pulmonaire, éventuellement dans lequel la fibrose pulmonaire est associée à une lésion pulmonaire induite par un médicament.

6. Antagoniste de la PSMP selon la revendication 1, dans lequel l'antagoniste de la PSMP est destiné à être utilisé dans le traitement de la fibrose rénale,
éventuellement dans lequel la fibrose rénale est associée à une maladie ou un trouble choisi dans le groupe constitué de la néphrite lupique, de la néphropathie à IgA et de la glomérulonéphrite membraneuse.

7. Antagoniste de la microprotéine sécrétée par PC3 (PSMP) destiné à être utilisé dans le traitement d'une maladie choisie dans le groupe constitué de la stéatose hépatique non alcoolique (SHNA), de la maladie alcoolique du foie (MAF), de la cholangite sclérosante primitive (CSP) et de la cholangite biliaire primitive (CBP), dans lequel l'antagoniste de la PSMP est un anticorps qui se lie spécifiquement à la PSMP.

8. Antagoniste de la PSMP pour utilisation selon la revendication 7, dans lequel la stéatose hépatique non alcoolique est la stéatohépatite non alcoolique (SHNA).

9. Antagoniste de la microprotéine sécrétée par PC3 (PSMP) destiné à être utilisé dans le traitement de la lésion rénale aiguë (LRA) ou de l'insuffisance rénale chronique (IRC), dans lequel l'antagoniste de la PSMP est un anticorps qui se lie spécifiquement à la PSMP,
éventuellement dans lequel la LRA est induite par la rhabdomyolyse,
éventuellement dans lequel l'IRC est causée par une maladie ou un trouble choisi dans le groupe constitué de la néphrite lupique, de la néphropathie à IgA et de la glomérulonéphrite membraneuse.

10. Antagoniste de la microprotéine sécrétée par PC3 (PSMP) destiné à être utilisé dans le traitement d'une maladie ou d'un trouble choisi dans le groupe constitué de la maladie du greffon contre l'hôte (MGCH), du lupus érythémateux disséminé (LED) et de la néphrite lupique, dans lequel l'antagoniste de la PSMP est un anticorps qui se lie spécifiquement à la PSMP.

11. Antagoniste de la PSMP pour utilisation selon l'une quelconque des revendications 1, 7, 9 et 10, dans lequel l'anticorps entre en compétition pour la liaison à la PSMP avec un second anticorps comprenant un domaine variable de chaîne lourde (VH) ayant la séquence d'acides aminés représentée dans la SEQ ID NO:4 et un domaine variable de chaîne légère (VL) ayant la séquence d'acides aminés représentée dans SEQ ID NO:5.

12. Antagoniste de la PSMP pour utilisation selon la revendication 11, dans lequel l'anticorps comprend un domaine VH comprenant des régions déterminant la complémentarité (CDR) CDR-H1_{Ab}, CDR-H2_{Ab} et CDR-H3_{Ab}, ledit ensemble de CDR VH ayant trois substitutions d'acides aminés ou moins par rapport à un ensemble de CDR de référence CDR-H1_{Réf}, CDR-H2_{Réf} et CDR-H3_{Réf} dans lequel
CDR-H1_{Réf} est un CDR-H1 ayant la SEQ ID NO:4 ;
CDR-H2_{Réf} est un CDR-H2 ayant la ID NO:4 ; et
CDR-H3_{Réf} est un CDR-H3 ayant la SEQ ID NO:4 ;
éventuellement dans lequel chaque CDR VH est défini selon la définition Chothia du CDR, selon laquelle
CDR-H1_{Réf} a la séquence d'acides aminés représentée dans les résidus 31 à 35 de la SEQ ID NO:4 ;
CDR-H2_{Réf} a la séquence d'acides aminés représentée dans les résidus 50 à 69 de la SEQ ID NO:4 ; et
CDR-H3_{Réf} a la séquence d'acides aminés représentée dans les résidus 99 à 108 de la SEQ ID NO:4.

13. Antagoniste de la PSMP pour utilisation selon la revendication 12, dans lequel l'anticorps comprend un domaine VL comprenant des régions déterminant la complémentarité (CDR) CDR-L1_{Ab}, CDR-L2_{Ab} et CDR-L3_{Ab}, ledit ensemble de CDR VL ayant trois substitutions d'acides aminés ou moins par rapport à un ensemble de CDR de référence CDR-L1_{Réf}, CDR-L2_{Réf} et CDR-L3_{Réf} dans lequel
CDR-L1_{Réf} est un CDR-L1 ayant la SEQ ID NO:5 ;
CDR-L2_{Réf} est un CDR-L2 ayant la SEQ ID NO: 5 ; et
CDR-L3_{Réf} est un CDR-L3 ayant la SEQ ID NO: 5 ;
éventuellement dans lequel chaque CDR VL est défini selon la définition Chothia du CDR, selon laquelle
CDR-L1_{Réf} a la séquence d'acides aminés représentée dans les résidus 24 à 34 de la SEQ ID NO:5 ;
CDR-L2_{Réf} a la séquence d'acides aminés représentée dans les résidus 50 à 56 de la SEQ ID NO: 5 ; et
CDR-L3_{Réf} a la séquence d'acides aminés représentée dans les résidus 89 à 97 de la SEQ ID NO: 5.

14. Antagoniste de la PSMP pour utilisation selon la revendication 11, dans lequel l'anticorps est un anticorps humanisé, un anticorps chimérique ou un anticorps humain.

15. Antagoniste de la PSMP selon l'une quelconque des revendications 11 à 13, dans lequel l'anticorps est un anticorps à chaîne unique.
